# EUROPEAN PATENT SPECIFICATION

(11) **EP 4 460 334 B1**
(45) Date of publication and mention of the grant of the patent: **14.10.2026**
(21) Application number: 23706945.5
(22) Date of filing: 06.01.2023
(51) Int. Cl.: A61K 48/00, A61K 31/00, C12N 15/67, C12N 15/85

(54) **OPTIMIZED POLYNUCLEOTIDES FOR PROTEIN EXPRESSION**
OPTIMIERTE POLYNUKLEOTIDE ZUR PROTEINEXPRESSION
POLYNUCLÉOTIDES OPTIMISÉS POUR L'EXPRESSION DE PROTÉINES

(30) Priority: 07.01.2022 US 202263297630 P
(43) Date of publication of application: 13.11.2024
(73) Proprietor: Precision BioSciences, Inc., Durham, NC 27701 (US)
(72) Inventor: HARRIS, Jason Richard, Durham, NC 27712 (US); HEKELE, Armin, Cary, NC 27519 (US)
(74) Representative: Grund, Martin
(86) International application number: PCT/US2023/060258
(87) International publication number: WO 2023/133525

(56) References cited:
- WO-A1-2017/001554
- WO-A1-2020/132659
- WO-A1-2022/150616
- WO-A2-2013/143700
- WO-A2-2020/198641
- AU-A1- 2018 339 089
- KWON HYOKYOUNG ET AL: "Emergence of synthetic mRNA:In vitrosynthesis of mRNA and its applications in regenerative medicine", BIOMATERIALS, ELSEVIER, AMSTERDAM, NL, vol. 156, 23 November 2017 (2017-11-23), pages 172 - 193, XP085314415, ISSN: 0142-9612, DOI: 10.1016/J.BIOMATERIALS.2017.11.034
- TUSUP MARINA ET AL: "AN EIF4G-RECRUITING APTAMER INCREASES THE FUNCTIONALITY OF IN VITRO TRANSCRIBED MRNA", EPH - INTERNATIONAL JOURNAL OF MEDICAL AND HEALTH SCIENCE, vol. 4, no. 2, 5 June 2018 (2018-06-05), pages 29 - 34, XP093041229, DOI: 10.53555/eijmhs.v4i2.36

## Description

### FIELD OF THE INVENTION

The invention relates to the field of molecular biology and recombinant nucleic acid technology. In particular, the invention relates to optimized polynucleotides useful for protein expression *in vitro* and *in vivo* including, for example, engineered nucleases.

### BACKGROUND OF THE INVENTION

Messenger RNA (mRNA)-based chromosomal editing techniques may hold the key for the treatment of many genetic diseases. However, mRNA-based editing platforms contain multiple opportunities for improvement including the short half-life of exogenous mRNA and therefore a shorter "time on target" for the encoded protein to edit the chromosome effectively. Within mRNA molecules, information in the 5' and 3' untranslated region (5' or 3' UTR) can regulate their targeting, translational efficiency, and stability (Mayr, Cold Spring Harb Perspect Biol.;11(10):a034728, 2019; van der Velden et al., Int J Biochem Cell Biol. 1, 87-106. 1999; Araujo et al., Comp Funct Genomics;2012:475731, 2012). Given the wide range of regulatory effects UTRs have on mRNA, the modulation of UTRs can potentially enhance both mRNA stability and translation efficiency in a system.

UTRs play critical roles in the post-transcriptional regulation of gene expression. This regulation is mediated by several factors. Nucleotide motifs situated in both the 5' and 3' UTRs can form secondary structure and/or interact directly with motif specific RNA-binding proteins. In addition, UTRs may contain repetitive elements that regulate expression at the RNA level. For example, CUG-binding proteins may bind to CUG repeats in the 5' UTR of specific mRNAs affecting their translation efficiency (Timchenko, Am J Hum Genet. 64:360-364, 1999). Interactions between these UTR sequence elements and non-coding RNAs have also been shown to play key regulatory roles (Sweeney et al., Proc Natl Acad Sci USA, 93:8518-8523, 1996). Therefore, post-translational control is a combination of primary and/or secondary structure interactions with the surrounding cellular environment. Taken together the UTR sequence and cellular environment are key to RNA regulation.

AU 2018/339089 A1, WO 2017(001554 A1, WO 2020/198641 A2, and WO 2013/143700 A2 disclose mRNAs with different 5' and 3' UTR sequences aimed at increasing expression of the mRNA.

mRNA turnover (i.e., mRNA half-life) is another regulating step in protein expression. An mRNA with a short half-life will not have the opportunity to generate as much protein as a mRNA with a long half-life (regardless of 5' UTR efficiency). mRNA degradation is mostly regulated by motifs located in the 3' UTR. An example of such a motif is the AU-rich element (ARE). AREs promote mRNA decay in response to specific intra- and extra-cellular signals. AREs are grouped into classes based on sequence motifs: class I and II are characterized by the presence of multiple copies of an AUUUA motif (Peng et al., Mol Cell Biol. 16:1490-1499, 1996). This class of ARE control the cytoplasmic deadenylation of mRNAs by generating RNA with short poly(A) tails of about 30-60 nucleotides. RNA with such short tails are then rapidly degraded. These motifs and others like it are generally found in mRNAs encoding for "fast response" genes/proteins.

### SUMMARY OF THE INVENTION

The invention is defined by the appended claims.

In one aspect, the disclosure provides a polynucleotide comprising a nucleic acid sequence encoding an engineered meganuclease, wherein the nucleic acid sequence comprises: (a) a 5' untranslated region (UTR) comprising a nucleic acid sequence set forth in SEQ ID NO: 1; (b) a coding sequence encoding said engineered meganuclease, wherein said engineered meganuclease comprises a first nuclear localization sequence (NLS) positioned at the N-terminus and a second NLS positioned at the C-terminus, wherein said first NLS and said second NLS each comprise an amino acid sequence set forth in SEQ ID NO: 15; (c) a 3' UTR comprising a nucleic acid sequence set forth in SEQ ID NO: 10; (d) a poly A sequence; and (e) a promoter operably linked to said nucleic acid sequence encoding said engineered meganuclease.

In some embodiments, the 5' UTR does not comprise an upstream uATG sequence or upstream open reading frame sequence.

In some embodiments, the 5' UTR further comprises a eukaryotic initiation factor (eIF) recruitment sequence. In some embodiments, the eIF recruitment sequence comprises an eIF4A recruitment sequence. In some embodiments, the eIF recruitment sequence comprises an eIF4G recruitment sequence. In some embodiments, the eIF4G recruitment sequence comprises an APT17 sequence. In some embodiments, the APT17 sequence comprises a nucleic acid sequence having at least 80%, at least at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more, sequence identity to a sequence set forth in SEQ ID NO: 14. In some embodiments, the APT17 sequence comprises a nucleic acid sequence set forth in SEQ ID NO: 14.

In some embodiments, the 5' UTR does not form a stable secondary sequence structure that contains a heterologous protein start codon. In some embodiments, the 5' UTR does not form a stable secondary sequence structure that contains a heterologous protein start codon with a change in free energy (ΔG) below about -10 kcal/mol to about -80 kcal/mol. In some embodiments, the 5' UTR does not form a stable secondary sequence structure that contains a heterologous protein start codon with a change in free energy (ΔG) below about - 30 kcal/mol to about -50 kcal/mol. In some embodiments, the 5' UTR does not form a stable secondary sequence structure that contains a heterologous protein start codon with a change in free energy (ΔG) below about -30 kcal/mol. In some embodiments, the 5' UTR does not form a stable secondary sequence structure that contains a heterologous protein start codon with a change in free energy (ΔG) below about -50 kcal/mol.

In some embodiments, the 5' UTR further comprises a UTR Kozak sequence. In some embodiments, the UTR Kozak sequence comprises a nucleic acid sequence set forth in any one of SEQ ID NOs: 50-149. In some embodiments, the UTR Kozak sequence comprises a nucleic acid sequence set forth in SEQ ID NO: 114.

In some embodiments, the 5' UTR is from about 30 nucleotides to about 250 nucleotides in length.

In some embodiments, the 5' UTR further comprises an internal ribosomal entry site (IRES).

In some embodiments, the 3' UTR has less than about 5 AU rich elements (AREs). In some embodiments, the 3' UTR has less than about 3 AREs. In some embodiments, the 3' UTR does not comprise any AREs. In some embodiments, the ARE is a class I ARE. In some embodiments, the ARE is a class II ARE. In some embodiments, the ARE is a class III ARE.

In some embodiments, the 3' UTR is from about 30 nucleotides to about 700 nucleotides in length. In some embodiments, the 3' UTR is from about 100 nucleotides to about 500 nucleotides in length. In some embodiments, the 3' UTR is from about 50 nucleotides to about 250 nucleotides in length.

In some embodiments, the polynucleotide further comprises modification to a coding sequence of the engineered meganuclease to reduce ribosomal stacking or stalling during protein translation of the coding sequence, wherein the modification comprises changing one or more three base codons in the coding sequence that promote ribosomal stalling to a three base codon that reduces ribosomal stalling, thereby reducing ribosomal stalling or stacking during protein translation of the engineered meganuclease. In some embodiments, the modification does not alter the amino acid sequence of the engineered meganuclease. In some embodiments, the modification comprises modifying the codons encoding amino acid positions 3, 4, 5, 6, 7, 8, 9, or 10 of the coding sequence. In some embodiments, the modification comprises modifying the codons encoding amino acid positions 3, 4, and 5 of the coding sequence.

In some embodiments, the polynucleotide further comprises a modification to a coding sequence of the engineered meganuclease to reduce thymidine or uridine content of the coding sequence, wherein the modification does not alter the amino acid sequence of the engineered meganuclease. In some embodiments, the modification comprises changing a first three base codon containing a thymidine or uridine that encodes an amino acid to an alternative three base codon that has less thymidine or uridine than the first three base codon. In some embodiments, the modification comprises changing a first three base codon containing a thymidine or uridine that encodes an amino acid to an alternative three base codon that has no thymidine or uridine content. In some embodiments, the coding sequence has between 10% and 90% reduced thymidine or uridine content compared to a coding sequence that has not been modified to reduce thymidine or uridine content. In some embodiments, the coding sequence has between 30% and 70% reduced thymidine or uridine content compared to a coding sequence that has not been modified to reduce thymidine or uridine content. In some embodiments, the coding sequence has about 40% reduced thymidine or uridine content compared to a coding sequence that has not been modified to reduce thymidine or uridine content.

In some embodiments, the polynucleotide further comprises a modification to a coding sequence of the engineered meganuclease to increase the guanosine or cytosine content of the coding sequence, wherein the modification does not alter the amino acid sequence of the engineered meganuclease. In some embodiments, the modification comprises changing a first three base codon uridine that encodes an amino acid to an alternative three base codon that has increased guanosine or cytosine content. In some embodiments, the coding sequence has between 10% and 50% increased guanosine or cytosine content compared to a coding sequence that has not been modified to increase the guanosine or cytosine content.

In some embodiments, the engineered meganuclease comprises a first subunit and a second subunit, wherein the first subunit binds to a first recognition half-site of the recognition sequence and comprises a first hypervariable (HVR1) region, the second subunit binds to a second recognition half-site of the recognition sequence and comprises a second hypervariable (HVR2) region, and the first subunit and the second subunit each comprise an amino acid sequence having at least 80%, 85%, 90%, 91%, 92%, 93%, 94%, 95%, 96%, 97%, 98%, 99% or more sequence identity to a sequence set forth in SEQ ID NO: 169. In some embodiments, the first subunit and the second subunit each comprise an amino acid sequence having at least 80%, 85%, 90%, 91%, 92%, 93%, 94%, 95%, 96%, 97%, 98%, 99%, or more, sequence identity to residues 7-153 of SEQ ID NO: 169. In some embodiments, the engineered meganuclease comprises an amino acid sequence having at least 80%, 85%, 90%, 91%, 92%, 93%, 94%, 95%, 96%, 97%, 98%, 99% or more sequence identity to a sequence set forth in SEQ ID NO: 170. In some such embodiments, codons encoding amino acids that are conserved between the first subunit and the second subunit are wobbled; i.e., are not identical to one another but still encode the same amino acid.

In some embodiments, the 5' UTR comprises a nucleic acid sequence set forth in SEQ ID NO: 1; wherein the 5' UTR comprises: a UTR Kozak sequence comprising a nucleic acid sequence set forth in any one of SEQ ID NOs: 50-149; wherein the 5' UTR does not comprise an upstream uATG sequence or upstream open reading frame sequence; wherein the engineered meganuclease comprising a first NLS at the N-terminus and a second NLS at the C-terminus; wherein the first NLS and the second NLS are identical and comprise an amino acid sequence set forth in SEQ ID NO: 15; wherein the coding sequence of the heterologous protein has been modified to have reduced thymidine or uridine content; wherein the 3' UTR comprises a nucleic acid sequence set forth in SEQ ID NO: 10; and wherein the 3' UTR does not comprise any AREs.

In some embodiments, the polynucleotide is an mRNA described herein. In some embodiments, the mRNA comprises a 5' cap. In some embodiments, the 5' cap comprises a 5' methyl guanosine cap. In some embodiments, a uridine present in the mRNA is pseudouridine or 2-thiouridine. In some embodiments, a uridine present in the mRNA is methylated. In some embodiments, a uridine present in the mRNA is N1-methylpseudouridine, 5-methyluridine, or 2'-O-methyluridine.

In another aspect, the disclosure provides a recombinant DNA construct that comprises a polynucleotide that is described herein. In some embodiments, the recombinant DNA construct encodes a recombinant virus comprising the polynucleotide. In some embodiments, the recombinant virus is a recombinant adenovirus, a recombinant lentivirus, a recombinant retrovirus, or a recombinant adeno-associated virus (AAV). In some embodiments, the recombinant virus is a recombinant AAV.

In another aspect, the disclosure provides a recombinant virus that comprises a polynucleotide that is described herein. In some embodiments, the recombinant virus is a recombinant adenovirus, a recombinant lentivirus, a recombinant retrovirus, or a recombinant adeno-associated virus (AAV). In some embodiments, the recombinant virus is a recombinant AAV.

In another aspect, the disclosure provides a lipid nanoparticle composition comprising lipid nanoparticles comprising a polynucleotide that is described herein. In some embodiments, the polynucleotide comprised by the lipid nanoparticle composition is an mRNA described herein.

In another aspect, the disclosure provides a pharmaceutical composition comprising a pharmaceutically acceptable carrier and a polynucleotide that is described herein.

In another aspect, the disclosure provides a pharmaceutical composition comprising a pharmaceutically acceptable carrier and a recombinant DNA construct that is described herein.

In another aspect, the disclosure provides a pharmaceutical composition comprising a pharmaceutically acceptable carrier and a recombinant virus that is described herein.

In another aspect, the disclosure provides a pharmaceutical composition comprising a pharmaceutically acceptable carrier and a lipid nanoparticle composition that is described herein.

### BRIEF DESCRIPTION OF THE SEQUENCES

SEQ ID NO: 1 sets forth a DNA nucleic acid sequence of a 5'ALB UTR.

SEQ ID NO: 2 sets forth a DNA nucleic acid sequence of a 5' FGA UTR.

SEQ ID NO: 3 sets forth a DNA nucleic acid sequence of a 5' FTH1 UTR.

SEQ ID NO: 4 sets forth a DNA nucleic acid sequence of a 5' GAPDH UTR .

SEQ ID NO: 5 sets forth a DNA nucleic acid sequence of a 5'HBA2 UTR.

SEQ ID NO: 6 sets forth a DNA nucleic acid sequence of a 5' SNRPB Variant 1 UTR.

SEQ ID NO: 7 sets forth a DNA nucleic acid sequence of a 5' XBG UTR.

SEQ ID NO: 8 sets forth a DNA nucleic acid sequence of a 3' HBA2 UTR.

SEQ ID NO: 9 sets forth a DNA nucleic acid sequence of a 3'HBB UTR.

SEQ ID NO: 10 sets forth a DNA nucleic acid sequence of a 3' SNRPB Variant 1 UTR.

SEQ ID NO: 11 sets forth a DNA nucleic acid sequence of a 3' SNRPB Variant 2 UTR.

SEQ ID NO: 12 sets forth a DNA nucleic acid sequence of a 3' XBG UTR.

SEQ ID NO: 13 sets forth a DNA nucleic acid sequence of a 3' WPRE UTR.

SEQ ID NO: 14 sets forth a DNA nucleic acid sequence of an APT17 recruiter sequence.

SEQ ID NO: 15 sets forth the amino acid sequence of an SV40 nuclear localization sequence.

SEQ ID NO: 16 sets forth the amino acid sequence of a NLS5 nuclear localization sequence.

SEQ ID NO: 17 sets forth the amino acid sequence of a CMYC nuclear localization sequence.

SEQ ID NO: 18 sets forth the amino acid sequence of an SV40H2 nuclear localization sequence.

SEQ ID NO: 19 sets forth a DNA nucleic acid sequence of an SV40 nuclear localization sequence.

SEQ ID NO: 20 sets forth a DNA nucleic acid sequence of an NLS5 nuclear localization sequence.

SEQ ID NO: 21 sets forth a DNA nucleic acid sequence that comprises from 5' to 3' a T7AG promoter, an APT17 ribosomal recruiter sequence, a 5' HBA2 UTR, a coding sequence for an N terminal SV40 nuclear localization sequence, an HAO 1-2L.30S19 engineered meganuclease coding sequence, and a 3' WPRE UTR.

SEQ ID NO: 22 sets forth a DNA nucleic acid sequence that comprises from 5' to 3' a T7AG promoter, an APT17 ribosomal recruiter sequence, a coding sequence for an N terminal SV40 nuclear localization sequence, an HAO 1-2L.30S19 engineered meganuclease coding sequence, and a 3' WPRE UTR.

SEQ ID NO: 23 sets forth a DNA nucleic acid sequence that comprises from 5' to 3' a T7AG promoter, a 5' XBG UTR, a coding sequence for an NLS5 nuclear localization sequence, an HAO 1-2L.30S19 engineered meganuclease coding sequence, and a 3' XBG UTR.

SEQ ID NO: 24 sets forth a DNA nucleic acid sequence that comprises from 5' to 3' a T7AG promoter, a 5' XBG UTR, a coding sequence for an N terminal SV40 nuclear localization sequence, an HAO 1-2L.30S19 engineered meganuclease coding sequence, and a 3' XBG UTR.

SEQ ID NO: 25 sets forth a DNA nucleic acid sequence that comprises from 5' to 3' a T7AG promoter, a 5' SNRPB V1 UTR, a coding sequence for an N terminal SV40 nuclear localization sequence, an HAO 1-2L.30S19 engineered meganuclease coding sequence, and a 3' SNRPB V1 UTR.

SEQ ID NO: 26 sets forth a DNA nucleic acid sequence that comprises from 5' to 3' a T7AG promoter, a 5' SNRPB V1 UTR, a coding sequence for an N terminal SV40 nuclear localization sequence, an HAO 1-2L.30S19 engineered meganuclease coding sequence, and a 3' SNRPB V2 UTR.

SEQ ID NO: 27 sets forth a DNA nucleic acid sequence that comprises from 5' to 3' a T7AG promoter, a 5' HBA2 UTR, a coding sequence for an N terminal SV40 nuclear localization sequence, an HAO 1-2L.30S19 engineered meganuclease coding sequence, and a 3' WPRE UTR.

SEQ ID NO: 28 sets forth a DNA nucleic acid sequence that comprises from 5' to 3' a T7AG promoter, a 5' HBA2 UTR, a coding sequence for N terminal SV40 nuclear localization sequence, an HAO 1-2L.30S19 engineered meganuclease coding sequence, and a 3' HBA2 UTR.

SEQ ID NO: 29 sets forth a DNA nucleic acid sequence that comprises from 5' to 3' a T7AG promoter, a 5' HBA2 UTR, a coding sequence for an N terminal SV40 nuclear localization sequence, an HAO 1-2L.30S19 engineered meganuclease coding sequence, and a 3'HBB UTR.

SEQ ID NO: 30 sets forth a DNA nucleic acid sequence that comprises from 5' to 3' a T7AG promoter, a 5' ALB UTR, a coding sequence for an N terminal SV40 nuclear localization sequence, an HAO 1-2L.30S19 engineered meganuclease coding sequence, and a 3' XBG UTR.

SEQ ID NO: 31 sets forth a DNA nucleic acid sequence that comprises from 5' to 3' a T7AG promoter, a 5' FGA UTR, a coding sequence for an N terminal SV40 nuclear localization sequence, an HAO 1-2L.30S19 engineered meganuclease coding sequence, and a 3' XBG UTR.

SEQ ID NO: 32 sets forth a DNA nucleic acid sequence that comprises from 5' to 3' a T7AG promoter, a 5' FTH1 UTR, a coding sequence for an N terminal SV40 nuclear localization sequence, an HAO 1-2L.30S19 engineered meganuclease coding sequence, and a 3' XBG UTR.

SEQ ID NO: 33 sets forth a DNA nucleic acid sequence that comprises from 5' to 3' a T7AG promoter, a 5' GAPDH UTR, a coding sequence for an N terminal SV40 nuclear localization sequence, an HAO 1-2L.30S19 engineered meganuclease coding sequence, and a 3' XBG UTR.

SEQ ID NO: 34 sets forth a DNA nucleic acid sequence that comprises from 5' to 3' a T7AG promoter, a 5' HBA2 UTR, a coding sequence for an N terminal SV40 nuclear localization sequence, an HAO 1-2L.30S19 engineered meganuclease coding sequence, and a 3' XBG UTR.

SEQ ID NO: 35 sets forth a DNA nucleic acid sequence that comprises from 5' to 3' a T7AG promoter, a 5' SNRPB V1 UTR, a coding sequence for an N terminal SV40 nuclear localization sequence, an HAO 1-2L.30S19 engineered meganuclease coding sequence, and a 3' XBG UTR.

SEQ ID NO: 36 sets forth a DNA nucleic acid sequence that comprises from 5' to 3' a T7AG promoter, a 5' XBG UTR, a coding sequence for an N terminal SV40 nuclear localization sequence, an HAO 1-2L.30S19 engineered meganuclease coding sequence, and a 3' HBA2 UTR.

SEQ ID NO: 37 sets forth a DNA nucleic acid sequence that comprises from 5' to 3' a T7AG promoter, a 5' XBG UTR, a coding sequence for an N terminal SV40 nuclear localization sequence, an HAO 1-2L.30S19 engineered meganuclease coding sequence, and a 3'HBB UTR.

SEQ ID NO: 38 sets forth a DNA nucleic acid sequence that comprises from 5' to 3' a T7AG promoter, a 5' XBG UTR, a coding sequence for an N terminal SV40 nuclear localization sequence, an HAO 1-2L.30S19 engineered meganuclease coding sequence, and a 3' SNRPB V1 UTR.

SEQ ID NO: 39 sets forth a DNA nucleic acid sequence that comprises from 5' to 3' a T7AG promoter, a 5' ALB UTR, a coding sequence for an N terminal SV40 nuclear localization sequence, an HAO 1-2L.30S19 engineered meganuclease coding sequence, and a 3' SNRPB V1 UTR.

SEQ ID NO: 40 sets forth a DNA nucleic acid sequence that comprises from 5' to 3' a T7AG promoter, a 5' FGA UTR, a coding sequence for an N terminal SV40 nuclear localization sequence, an HAO 1-2L.30S19 engineered meganuclease coding sequence, and a 3' SNRPB V1 UTR.

SEQ ID NO: 41 sets forth a DNA nucleic acid sequence that comprises from 5' to 3' a T7AG promoter, a 5' FTH1 UTR, a coding sequence for an N terminal SV40 nuclear localization sequence, an HAO 1-2L.30S19 engineered meganuclease coding sequence, and a 3' SNRPB V1 UTR.

SEQ ID NO: 42 sets forth a DNA nucleic acid sequence that comprises from 5' to 3' a T7AG promoter, a 5' GAPDH UTR, a coding sequence for an N terminal SV40 nuclear localization sequence, an HAO 1-2L.30S19 engineered meganuclease coding sequence, and a 3' SNRPB V1 UTR.

SEQ ID NO: 43 sets forth a DNA nucleic acid sequence that comprises from 5' to 3' a T7AG promoter, a 5' HBA2 UTR, a coding sequence for an N terminal SV40 nuclear localization sequence, an HAO 1-2L.30S19 engineered meganuclease coding sequence, and a 3' SNRPB V1 UTR.

SEQ ID NO: 44 sets forth a DNA nucleic acid sequence that comprises from 5' to 3' a T7AG promoter, a 5' SNRPB V1 UTR, a coding sequence for an N terminal SV40 nuclear localization sequence, an HAO 1-2L.30S19 engineered meganuclease coding sequence, and a 3' HBA2 UTR.

SEQ ID NO: 45 sets forth a DNA nucleic acid sequence that comprises from 5' to 3' a T7AG promoter, a 5' SNRPB V1 UTR, a coding sequence for an N terminal SV40 nuclear localization sequence, an HAO 1-2L.30S19 engineered meganuclease coding sequence, and a 3'HBB UTR.

SEQ ID NO: 46 sets forth a DNA nucleic acid sequence that comprises from 5' to 3' a T7AG promoter, a 5' HBA2 UTR, a coding sequence for an N terminal SV40 nuclear localization sequence, an HAO 25-26L.1128 engineered meganuclease coding sequence, and a 3' WPRE UTR.

SEQ ID NO: 47 sets forth a DNA nucleic acid sequence that comprises from 5' to 3' a T7AG promoter, a 5' HBA2 UTR, a coding sequence for an N terminal SV40 nuclear localization sequence, an HAO 25-26L.1434 engineered meganuclease coding sequence, and a 3' WPRE UTR.

SEQ ID NO: 48 sets forth a DNA nucleic acid sequence that comprises from 5' to 3' a T7AG promoter, a 5' ALB UTR, a coding sequence for an N terminal SV40 nuclear localization sequence, an HAO 25-26L.1128 engineered meganuclease coding sequence, a C terminal SV40 nuclear localization sequence, and a 3' SNRPB V1 UTR.

SEQ ID NO: 49 sets forth a DNA nucleic acid sequence that comprises from 5' to 3' a T7AG promoter, a 5' ALB UTR, a coding sequence for an N terminal SV40 nuclear localization sequence, an HAO 25-26L.1434 engineered meganuclease coding sequence, a C terminal SV40 nuclear localization sequence, and a 3' SNRPB V1 UTR.

SEQ ID NO: 50 sets forth a DNA nucleic acid sequence of a UTR Kozak sequence.

SEQ ID NO: 51 sets forth a DNA nucleic acid sequence of a UTR Kozak sequence.

SEQ ID NO: 52 sets forth a DNA nucleic acid sequence of a UTR Kozak sequence.

SEQ ID NO: 53 sets forth a DNA nucleic acid sequence of a UTR Kozak sequence.

SEQ ID NO: 54 sets forth a DNA nucleic acid sequence of a UTR Kozak sequence.

SEQ ID NO: 55 sets forth a DNA nucleic acid sequence of a UTR Kozak sequence.

SEQ ID NO: 56 sets forth a DNA nucleic acid sequence of a UTR Kozak sequence.

SEQ ID NO: 57 sets forth a DNA nucleic acid sequence of a UTR Kozak sequence.

SEQ ID NO: 58 sets forth a DNA nucleic acid sequence of a UTR Kozak sequence.

SEQ ID NO: 59 sets forth a DNA nucleic acid sequence of a UTR Kozak sequence.

SEQ ID NO: 60 sets forth a DNA nucleic acid sequence of a UTR Kozak sequence.

SEQ ID NO: 61 sets forth a DNA nucleic acid sequence of a UTR Kozak sequence.

SEQ ID NO: 62 sets forth a DNA nucleic acid sequence of a UTR Kozak sequence.

SEQ ID NO: 63 sets forth a DNA nucleic acid sequence of a UTR Kozak sequence.

SEQ ID NO: 64 sets forth a DNA nucleic acid sequence of a UTR Kozak sequence.

SEQ ID NO: 65 sets forth a DNA nucleic acid sequence of a UTR Kozak sequence.

SEQ ID NO: 66 sets forth a DNA nucleic acid sequence of a UTR Kozak sequence.

SEQ ID NO: 67 sets forth a DNA nucleic acid sequence of a UTR Kozak sequence.

SEQ ID NO: 68 sets forth a DNA nucleic acid sequence of a UTR Kozak sequence.

SEQ ID NO: 69 sets forth a DNA nucleic acid sequence of a UTR Kozak sequence.

SEQ ID NO: 70 sets forth a DNA nucleic acid sequence of a UTR Kozak sequence.

SEQ ID NO: 71 sets forth a DNA nucleic acid sequence of a UTR Kozak sequence.

SEQ ID NO: 72 sets forth a DNA nucleic acid sequence of a UTR Kozak sequence.

SEQ ID NO: 73 sets forth a DNA nucleic acid sequence of a UTR Kozak sequence.

SEQ ID NO: 74 sets forth a DNA nucleic acid sequence of a UTR Kozak sequence.

SEQ ID NO: 75 sets forth a DNA nucleic acid sequence of a UTR Kozak sequence.

SEQ ID NO: 76 sets forth a DNA nucleic acid sequence of a UTR Kozak sequence.

SEQ ID NO: 77 sets forth a DNA nucleic acid sequence of a UTR Kozak sequence.

SEQ ID NO: 78 sets forth a DNA nucleic acid sequence of a UTR Kozak sequence.

SEQ ID NO: 79 sets forth a DNA nucleic acid sequence of a UTR Kozak sequence.

SEQ ID NO: 80 sets forth a DNA nucleic acid sequence of a UTR Kozak sequence.

SEQ ID NO: 81 sets forth a DNA nucleic acid sequence of a UTR Kozak sequence.

SEQ ID NO: 82 sets forth a DNA nucleic acid sequence of a UTR Kozak sequence.

SEQ ID NO: 83 sets forth a DNA nucleic acid sequence of a UTR Kozak sequence.

SEQ ID NO: 84 sets forth a DNA nucleic acid sequence of a UTR Kozak sequence.

SEQ ID NO: 85 sets forth a DNA nucleic acid sequence of a UTR Kozak sequence.

SEQ ID NO: 86 sets forth a DNA nucleic acid sequence of a UTR Kozak sequence.

SEQ ID NO: 87 sets forth a DNA nucleic acid sequence of a UTR Kozak sequence.

SEQ ID NO: 88 sets forth a DNA nucleic acid sequence of a UTR Kozak sequence.

SEQ ID NO: 89 sets forth a DNA nucleic acid sequence of a UTR Kozak sequence.

SEQ ID NO: 90 sets forth a DNA nucleic acid sequence of a UTR Kozak sequence.

SEQ ID NO: 91 sets forth a DNA nucleic acid sequence of a UTR Kozak sequence.

SEQ ID NO: 92 sets forth a DNA nucleic acid sequence of a UTR Kozak sequence.

SEQ ID NO: 93 sets forth a DNA nucleic acid sequence of a UTR Kozak sequence.

SEQ ID NO: 94 sets forth a DNA nucleic acid sequence of a UTR Kozak sequence.

SEQ ID NO: 95 sets forth a DNA nucleic acid sequence of a UTR Kozak sequence.

SEQ ID NO: 96 sets forth a DNA nucleic acid sequence of a UTR Kozak sequence.

SEQ ID NO: 97 sets forth a DNA nucleic acid sequence of a UTR Kozak sequence.

SEQ ID NO: 98 sets forth a DNA nucleic acid sequence of a UTR Kozak sequence.

SEQ ID NO: 99 sets forth a DNA nucleic acid sequence of a UTR Kozak sequence.

SEQ ID NO: 100 sets forth a DNA nucleic acid sequence of a UTR Kozak sequence.

SEQ ID NO: 101 sets forth a DNA nucleic acid sequence of a UTR Kozak sequence.

SEQ ID NO: 102 sets forth a DNA nucleic acid sequence of a UTR Kozak sequence.

SEQ ID NO: 103 sets forth a DNA nucleic acid sequence of a UTR Kozak sequence.

SEQ ID NO: 104 sets forth a DNA nucleic acid sequence of a UTR Kozak sequence.

SEQ ID NO: 105 sets forth a DNA nucleic acid sequence of a UTR Kozak sequence.

SEQ ID NO: 106 sets forth a DNA nucleic acid sequence of a UTR Kozak sequence.

SEQ ID NO: 107 sets forth a DNA nucleic acid sequence of a UTR Kozak sequence.

SEQ ID NO: 108 sets forth a DNA nucleic acid sequence of a UTR Kozak sequence.

SEQ ID NO: 109 sets forth a DNA nucleic acid sequence of a UTR Kozak sequence.

SEQ ID NO: 110 sets forth a DNA nucleic acid sequence of a UTR Kozak sequence.

SEQ ID NO: 111 sets forth a DNA nucleic acid sequence of a UTR Kozak sequence.

SEQ ID NO: 112 sets forth a DNA nucleic acid sequence of a UTR Kozak sequence.

SEQ ID NO: 113 sets forth a DNA nucleic acid sequence of a UTR Kozak sequence.

SEQ ID NO: 114 sets forth a DNA nucleic acid sequence of a UTR Kozak sequence.

SEQ ID NO: 115 sets forth a DNA nucleic acid sequence of a UTR Kozak sequence.

SEQ ID NO: 116 sets forth a DNA nucleic acid sequence of a UTR Kozak sequence.

SEQ ID NO: 117 sets forth a DNA nucleic acid sequence of a UTR Kozak sequence.

SEQ ID NO: 118 sets forth a DNA nucleic acid sequence of a UTR Kozak sequence.

SEQ ID NO: 119 sets forth a DNA nucleic acid sequence of a UTR Kozak sequence.

SEQ ID NO: 120 sets forth a DNA nucleic acid sequence of a UTR Kozak sequence.

SEQ ID NO: 121 sets forth a DNA nucleic acid sequence of a UTR Kozak sequence.

SEQ ID NO: 122 sets forth a DNA nucleic acid sequence of a UTR Kozak sequence.

SEQ ID NO: 123 sets forth a DNA nucleic acid sequence of a UTR Kozak sequence.

SEQ ID NO: 124 sets forth a DNA nucleic acid sequence of a UTR Kozak sequence.

SEQ ID NO: 125 sets forth a DNA nucleic acid sequence of a UTR Kozak sequence.

SEQ ID NO: 126 sets forth a DNA nucleic acid sequence of a UTR Kozak sequence.

SEQ ID NO: 127 sets forth a DNA nucleic acid sequence of a UTR Kozak sequence.

SEQ ID NO: 128 sets forth a DNA nucleic acid sequence of a UTR Kozak sequence.

SEQ ID NO: 129 sets forth a DNA nucleic acid sequence of a UTR Kozak sequence.

SEQ ID NO: 130 sets forth a DNA nucleic acid sequence of a UTR Kozak sequence.

SEQ ID NO: 131 sets forth a DNA nucleic acid sequence of a UTR Kozak sequence.

SEQ ID NO: 132 sets forth a DNA nucleic acid sequence of a UTR Kozak sequence.

SEQ ID NO: 133 sets forth a DNA nucleic acid sequence of a UTR Kozak sequence.

SEQ ID NO: 134 sets forth a DNA nucleic acid sequence of a UTR Kozak sequence.

SEQ ID NO: 135 sets forth a DNA nucleic acid sequence of a UTR Kozak sequence.

SEQ ID NO: 136 sets forth a DNA nucleic acid sequence of a UTR Kozak sequence.

SEQ ID NO: 137 sets forth a DNA nucleic acid sequence of a UTR Kozak sequence.

SEQ ID NO: 138 sets forth a DNA nucleic acid sequence of a UTR Kozak sequence.

SEQ ID NO: 139 sets forth a DNA nucleic acid sequence of a UTR Kozak sequence.

SEQ ID NO: 140 sets forth a DNA nucleic acid sequence of a UTR Kozak sequence.

SEQ ID NO: 141 sets forth a DNA nucleic acid sequence of a UTR Kozak sequence.

SEQ ID NO: 142 sets forth a DNA nucleic acid sequence of a UTR Kozak sequence.

SEQ ID NO: 143 sets forth a DNA nucleic acid sequence of a UTR Kozak sequence.

SEQ ID NO: 144 sets forth a DNA nucleic acid sequence of a UTR Kozak sequence.

SEQ ID NO: 145 sets forth a DNA nucleic acid sequence of a UTR Kozak sequence.

SEQ ID NO: 146 sets forth a DNA nucleic acid sequence of a UTR Kozak sequence.

SEQ ID NO: 147 sets forth a DNA nucleic acid sequence of a UTR Kozak sequence.

SEQ ID NO: 148 sets forth a DNA nucleic acid sequence of a UTR Kozak sequence.

SEQ ID NO: 149 sets forth a DNA nucleic acid sequence of a UTR Kozak sequence.

SEQ ID NO: 150 sets forth the nucleic acid sequence of a ddPCR probe.

SEQ ID NO: 151 sets forth the nucleic acid sequence of a forward primer sequence.

SEQ ID NO: 152 sets forth the nucleic acid sequence of a reverse primer sequence.

SEQ ID NO: 153 sets forth the nucleic acid sequence of a ddPCR probe.

SEQ ID NO: 154 sets forth the nucleic acid sequence of a forward primer sequence.

SEQ ID NO: 155 sets forth the nucleic acid sequence of a reverse primer sequence.

SEQ ID NO: 156 sets forth the nucleic acid sequence of a ddPCR probe.

SEQ ID NO: 157 sets forth the nucleic acid sequence of a forward primer sequence.

SEQ ID NO: 158 sets forth the nucleic acid sequence of a reverse primer sequence.

SEQ ID NO: 159 sets forth the nucleic acid sequence of a ddPCR probe.

SEQ ID NO: 160 sets forth the nucleic acid sequence of a ddPCR probe.

SEQ ID NO: 161 sets forth the nucleic acid sequence of a ddPCR probe.

SEQ ID NO: 162 sets forth the nucleic acid sequence of a forward primer sequence.

SEQ ID NO: 163 sets forth the nucleic acid sequence of a reverse primer sequence.

SEQ ID NO: 164 sets forth the nucleic acid sequence of a ddPCR probe.

SEQ ID NO: 165 sets forth the nucleic acid sequence of a forward primer sequence.

SEQ ID NO: 166 sets forth the nucleic acid sequence of a reverse primer sequence.

SEQ ID NO: 167 sets forth the amino acid sequence of an SV40 nuclear localization sequence.

SEQ ID NO: 168 sets forth the DNA nucleic acid sequence encoding an SV40 nuclear localization sequence.

SEQ ID NO: 169 sets forth the amino acid sequence of the wild-type I-CreI meganuclease.

SEQ ID NO: 170 sets forth the amino acid sequence of an engineered meganuclease comprising two subunits having wild-type I-CreI residues.

SEQ ID NO: 171 sets forth the DNA sequence of a standard control mRNA that comprises from 5' to 3' a T7AG promoter, a 5' HBA2 UTR , an N terminal SV40 nuclear localization sequence, a TRC 1-2L.2307 engineered meganuclease, and a 3' WPRE UTR.

SEQ ID NO: 172 sets forth the DNA sequence of an mRNA that comprises from 5' to 3' a T7AG promoter, a 5' ALB UTR, an N terminal SV40 nuclear localization sequence, a TRC 1-2L.2307 engineered meganuclease, a C terminal SV40 nuclear localization sequence, and a 3' SNRPB V1 UTR.

SEQ ID NO: 173 sets forth the DNA sequence of an mRNA that comprises from 5' to 3' a T7AG promoter, a 5' XBG UTR, an N terminal SV40 nuclear localization sequence, an HAO 1-2L.30S19 engineered meganuclease, a C terminal SV40 nuclear localization sequence, and a 3' XBG UTR. The sequence also includes an Ssp1 linearization sequence.

SEQ ID NO: 174 sets forth the DNA sequence of a standard control mRNA that comprises from 5' to 3' a T7AG promoter, a 5' HBA2 UTR, an N terminal SV40 nuclear localization sequence, an HAO 1-2L.30S19 engineered meganuclease, and a 3' WPRE UTR. The sequence also includes an BspQ1 linearization sequence.

SEQ ID NO: 175 sets forth the DNA sequence of an mRNA that comprises from 5' to 3' a T7AG promoter, a 5' XBG UTR, an N terminal SV40 nuclear localization sequence, an HAO 1-2L.30S19 engineered meganuclease, a C terminal SV40 nuclear localization sequence, and a 3' XBG UTR. The sequence also includes an BspQ1 linearization sequence.

SEQ ID NO: 176 sets forth the DNA sequence of an mRNA that comprises from 5' to 3' a T7AG promoter, a 5' XBG UTR, an N terminal cMyc nuclear localization sequence, an HAO 1-2L.30S19 engineered meganuclease, a C terminal cMyc nuclear localization sequence, and a 3' XBG UTR. The sequence also includes an BspQ1 linearization sequence.

SEQ ID NO: 177 sets forth the DNA sequence of an mRNA that comprises from 5' to 3' a T7AG promoter, a 5' ALB UTR, an N terminal SV40 nuclear localization sequence, an HAO 1-2L.30S19 engineered meganuclease, a C terminal SV40 nuclear localization sequence, and a 3' SNRPB V1 UTR. The sequence also includes an BspQ1 linearization sequence.

SEQ ID NO: 178 sets forth the DNA sequence of an mRNA that comprises from 5' to 3' a T7AG promoter, anAPT17 ribosomal recruiter sequence, a 5' ALB UTR, an N terminal SV40 nuclear localization sequence, an HAO 1-2L.30S19 engineered meganuclease, a C terminal SV40 nuclear localization sequence, and a 3' SNRPB V1 UTR. The sequence also includes an BspQ1 linearization sequence.

SEQ ID NO: 179 sets forth the DNA sequence of an mRNA that comprises from 5' to 3' a T7AG promoter, a 5' XBG UTR, an N terminal cMyc nuclear localization sequence, an HAO 1-2L.30S19 engineered meganuclease, a C terminal cMyc nuclear localization sequence, and a 3' XBG UTR.

SEQ ID NO: 180 sets forth the DNA sequence of an mRNA that comprises from 5' to 3' a T7AG promoter, a 5' XBG UTR, an N terminal cMyc nuclear localization sequence, an HBV 11-12L.1090 engineered meganuclease, a C terminal cMyc nuclear localization sequence, and a 3' XBG UTR.

SEQ ID NO: 181 sets forth the DNA sequence of a standard control mRNA that comprises from 5' to 3' a T7AG promoter, a 5' HBA2 UTR, an N terminal SV40 nuclear localization sequence, an HAO 1-2L.30S19 engineered meganuclease, and a 3' WPRE UTR.

SEQ ID NO: 182 sets forth the DNA sequence of a standard control mRNA that comprises from 5' to 3' a T7AG promoter, a 5' HBA2 UTR, an N terminal SV40 nuclear localization sequence, an HBV 11-12L.1090 engineered meganuclease, and a 3' WPRE UTR.

SEQ ID NO: 182 sets forth the DNA sequence of an mRNA that comprises from 5' to 3' a T7AG promoter, a 5' HBA2 UTR, an N terminal SV40 nuclear localization sequence, an HBV 11-12L.1090 engineered meganuclease, and a 3' WPRE UTR.

SEQ ID NO: 183 sets forth the DNA sequence of an mRNA that comprises from 5' to 3' a T7AG promoter, a 5' HBA2 UTR, an N terminal SV40 nuclear localization sequence, an HAO 25-26L.1128 engineered meganuclease, and a 3' WPRE UTR.

SEQ ID NO: 184 sets forth the DNA sequence of an mRNA that comprises from 5' to 3' a T7AG promoter, a 5' ALB UTR, an N terminal SV40 nuclear localization sequence, an HAO 25-26L.1128 engineered meganuclease, a C terminal SV40 nuclear localization sequence, and a 3' SNRPB V1 UTR.

SEQ ID NO: 185 sets forth the DNA sequence of an mRNA that comprises from 5' to 3' a T7AG promoter, a 5' HBA2 UTR, an N terminal SV40 nuclear localization sequence, an HAO 25-26L.1434 engineered meganuclease , and a 3' WPRE UTR.

SEQ ID NO: 186 sets forth the DNA sequence of an mRNA that comprises from 5' to 3' a T7AG promoter, a 5' ALB UTR, an N terminal SV40 nuclear localization sequence, an HAO 25-26L.1434 engineered meganuclease , a C terminal SV40 nuclear localization sequence, and a 3' SNRPB V1 UTR.

SEQ ID NO: 187 sets forth the DNA sequence of an mRNA that comprises from 5' to 3' a T7AG promoter, a 5' HBA2 UTR , an N terminal SV40 nuclear localization sequence, an HAO 25-26x.227 engineered meganuclease , and a 3' WPRE UTR.

SEQ ID NO: 188 sets forth the DNA sequence of an mRNA that comprises from 5' to 3' a T7AG promoter, a 5' HBA2 UTR, an N terminal SV40 nuclear localization sequence, a TTR 15-16x.81 engineered meganuclease, and a 3' WPRE UTR.

SEQ ID NO: 189 sets forth the DNA sequence of an mRNA that comprises from 5' to 3' a T7AG promoter, a 5' XBG UTR, an N terminal cMyc nuclear localization sequence, a TTR 15-16x.81 engineered meganuclease, a C terminal cMyc nuclear localization sequence, and a 3' XBG UTR.

### BRIEF DESCRIPTION OF THE DRAWINGS

FIG. 1 provides a bar graph showing the percentage of indel generation in HEK 293 cells at 2.5 hours, 5 hours, and 24 hours for cells electroporated with 2 ng of the indicated mRNA detailed in Table 1 of Example 1 encoding the HAO1-2L.30S19 engineered meganuclease.
FIG. 2 provides a bar graph showing the percentage of indel generation in BNL C.2 cells electroporated with either 20 ng or 200 ng of the indicated mRNA detailed in Table 2 of Example 2 encoding the F8R17-18L.1.35 engineered meganuclease.

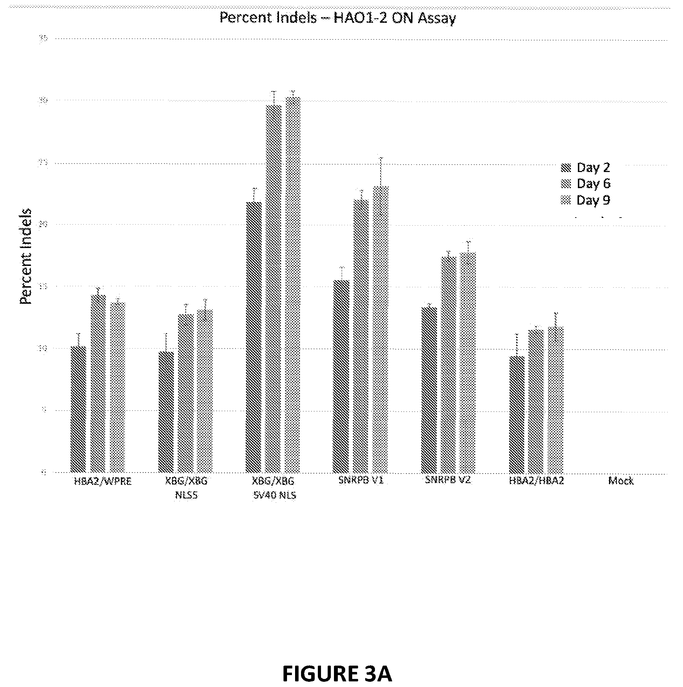

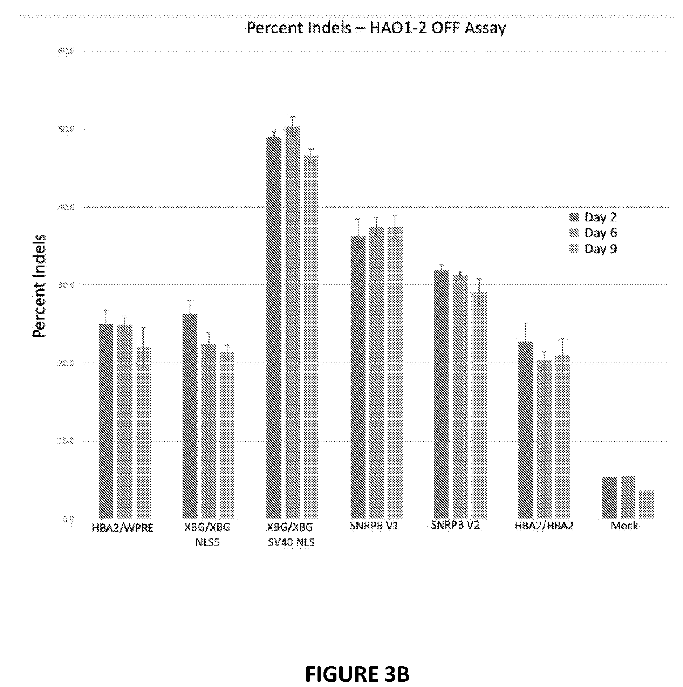

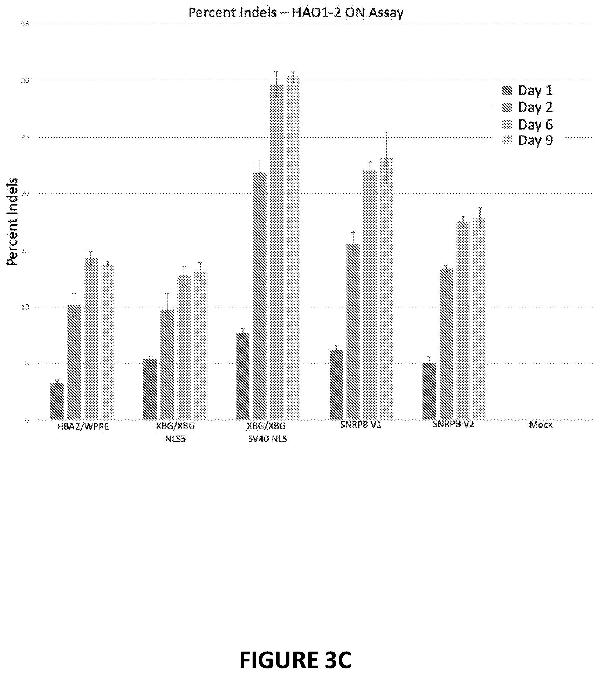

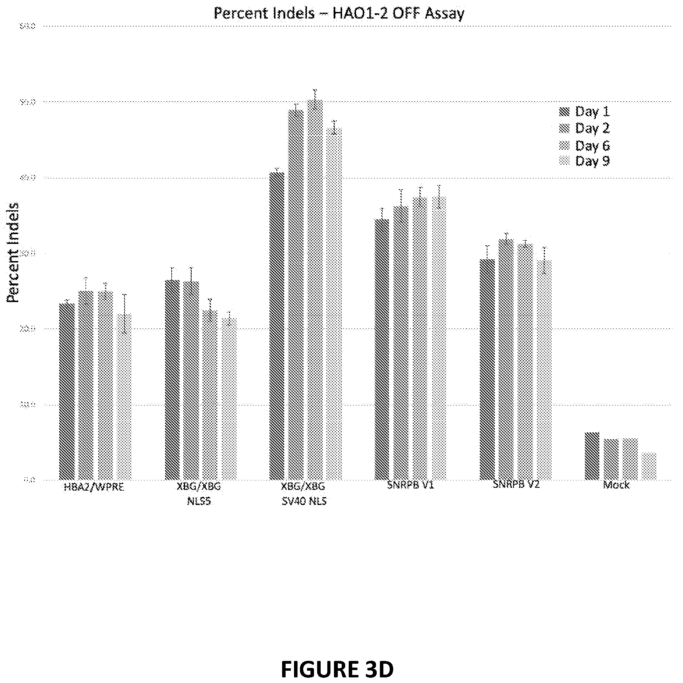

FIG. 3A-3D provides a bar graph showing the percentage of indel generation in HEP3B cells at 2 days, 6 days, and 9 days or at 1 day, 2 days, 6 days, and 9 days post electroporation with 2 ng of the indicated mRNA detailed in Table 3 of Example 3 encoding the HAO1-2L.30S19 engineered meganuclease. FIG. 3A provides the results for the "ON" ddPCR assay, which uses a ddPCR primer and probe set at the engineered meganuclease recognition sequence at 2 days, 6 days, and 9 days post electroporation. FIG. 3B shows the results for the "OFF" ddPCR assay, which utilizes a primer and probe set away from the recognition sequence at 2 days, 6 days, and 9 days post electroporation. FIG. 3C provides the results for the "ON" ddPCR assay and FIG. 3D provides the results for the "OFF" ddPCR assay at 1 day, 2 days, 6 days, and 9 days post electroporation.
FIG. 4A-4D provides a bar graph showing the percentage of indel generation in HEP3B cells at 2 days, 6 days, and 9 days post electroporation with 2 ng of the indicated mRNA detailed in Table 4 of Example 4 encoding the HAO1-2L.30S19 engineered meganuclease. FIG. 4A provides the results for the "OFF" ddPCR assay and FIG. 4B provides the results for the "ON" ddPCR assay at 2 days, 6 days, and 9 days. FIG. 4C and FIG. 4D provide the data shown in FIG. 4A-4B re-arranged by 5' UTR and 3' UTR combination.
FIG. 5A-5B provides a line graph showing the percentage of indel generation in HEP3B cells electroporated with either 0.25 ng, 0.5ng, 1ng, or 2 ng of the indicated mRNA detailed in Table 7 of Example 5 encoding the HAO1-2L.30S19 engineered meganuclease. FIG. 5A provides the results for the "OFF" ddPCR assay and FIG. 5B provides the results for the "ON" ddPCR assay at 2 days, 6 days, and 9 days.
FIG. 6 provides a line graph showing the percentage of indel generation in HepG2 cells electroporated with either 0.1ng, 0.5ng, 2ng, 10ng, 50ng, and 100ng of the indicated mRNA detailed in Table 10 of Example 6 encoding either the HAO25-26L.1434 or HAO25-26L.1128 engineered meganuclease.

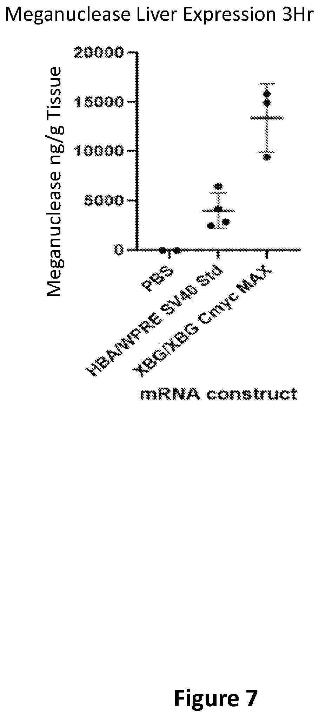

FIG. 7 provides a graph showing the protein level of an engineered meganuclease in mice that were administered to LNP formulation comprising the indicated mRNA encoding the engineered meganuclease.
FIG. 8 provides a graph showing the dose response curve of the TRC 1-2L.2307 meganuclease for knocking out cell surface CD3 assessed by flow cytometry. The meganuclease was encoded by the optimized Max construct according to the disclosure herein or by a standard control construct. The EC90 and EC50 values are provided for each construct.
FIG. 9 provides a bar graph providing the percentage of indels in Hep3B cells following treatment with the indicated HAO 1-2 L.30S19 meganuclease encoded by the indicated constructs.

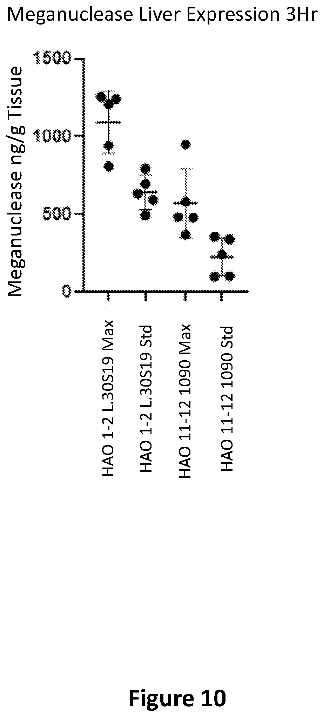

FIG. 10 provides a graph showing the protein level of an engineered meganuclease in mice that were administered to LNP formulation comprising the indicated mRNA encoding the engineered meganucleases.

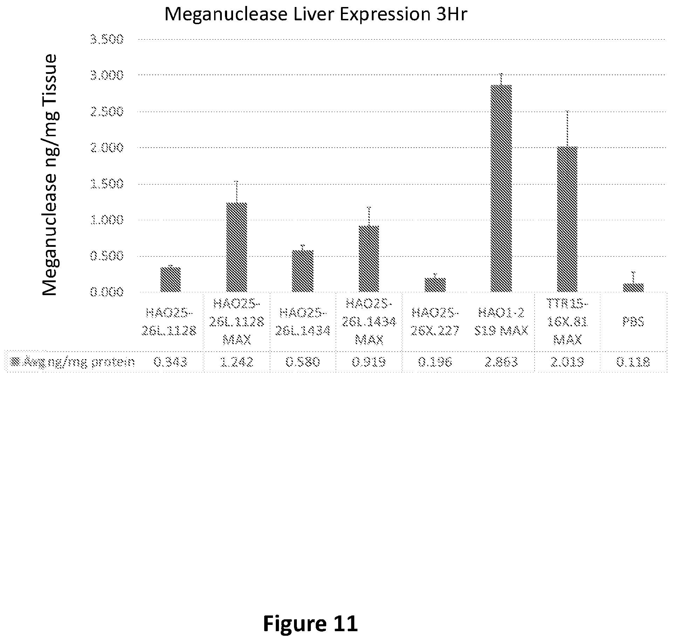

FIG. 11 provides a graph showing the protein level of an engineered meganuclease in mice that were administered to LNP formulation comprising the indicated mRNA encoding the engineered meganucleases.

### DETAILED DESCRIPTION

The invention is defined by the appended claims.

The patent and scientific literature referred to herein establishes knowledge that is available to those of skill in the art.

The present invention can be embodied in different forms and should not be construed as limited to the embodiments set forth herein. Rather, these embodiments are provided so that this disclosure will be thorough and complete, and will fully convey the scope of the invention to those skilled in the art. For example, features illustrated with respect to one embodiment can be incorporated into other embodiments, and features illustrated with respect to a particular embodiment can be deleted from that embodiment. In addition, numerous variations and additions to the embodiments suggested herein will be apparent to those skilled in the art in light of the instant disclosure, which do not depart from the instant invention.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

### I. Principle of the invention

mRNA based chromosomal editing techniques may hold the key for the treatment of genetic diseases. However, an mRNA editing platform contains multiple opportunities for improvement including extending the half-life of exogenous mRNA and therefore a longer "time on target" for the encoded protein to edit the chromosome effectively. Within mRNA molecules, information in the 5' and 3' untranslated region (5' or 3' UTR) can regulate their targeting, translational efficiency, and stability.

Here, a polynucleotide encoding an exogenous mRNA with modulated half-life is provided. The half-life may be increased or decreased to achieve optimal expression levels of the exogenous mRNA and downstream protein. As described herein, the polynucleotide comprises a 5' untranslated region (UTR); a coding sequence encoding a heterologous protein; a 3' UTR; and a poly A sequence as defined in claim 1. The 5' UTR and 3' UTR can be optimized such that the half-life of the exogenous mRNA is increased, as is the level of the encoded heterologous protein in a eukaryotic cell. In addition, certain combinations of 5' UTR and 3' UTRs can reduce the persistence of an exogenous mRNA molecule. As described and demonstrated experimentally herein, certain combinations of UTRs provide for higher levels of expression than others. Therefore, the combination of a 5' UTR and 3' UTR allows for tunability of mRNA persistence and consequently downstream heterologous protein expression. The heterologous protein is an engineered meganuclease. In some embodiments, the genomic editing efficiency of the engineered nuclease is advantageously increased compared to a control mRNA construct. In other embodiments, the genomic editing efficiency is advantageously decreased compared to a control mRNA construct. Also provided herein are pharmaceutical compositions comprising the polynucleotide, a method for expressing a heterologous protein in a eukaryotic cell using the polynucleotide, and the pharmaceutical composition for use in treating a disease in a subject.

### II. Definitions

As used herein, "a," "an," or "the" can mean one or more than one. For example, "a" cell can mean a single cell or a multiplicity of cells.

As used herein, unless specifically indicated otherwise, the word "or" is used in the inclusive sense of "and/or" and not the exclusive sense of "either/or."

As used herein, all polynucleotide sequences written using the nucleic acid standard notation of the International Union of Pure and Applied Chemistry (IUPAC, Biochemistry (1970) Vol. 9:4022-4027); adenine (A), thymine (T), guanine (G), and cytosine (C) are equivalent to the corresponding RNA polynucleotide sequences. Therefore, "T" (Thymine) in all sequences is equivalent to "U" (uracil). For example, the sequence AATAAA in a DNA coding strand would also indicate the corresponding mRNA sequence AAUAAA.

As used herein, the use of the term "polynucleotide", "DNA", or "nucleic acid" is not intended to limit the present invention to polynucleotides comprising DNA. Those of ordinary skill in the art will recognize that polynucleotides can comprise ribonucleotides and combinations of ribonucleotides and deoxyribonucleotides. Such deoxyribonucleotides and ribonucleotides include both naturally occurring molecules and synthetic analogues. The polynucleotides of the invention also encompass all forms of sequences including, but not limited to, single-stranded forms, double-stranded forms, hairpins, stem-and-loop structures, and the like.

As used herein, the term "5' untranslated region" or "5' UTR" stands for the region of a messenger RNA (mRNA) that is directly upstream from the initiation codon. This region is important for the regulation of translation of a transcript by differing mechanisms in viruses, prokaryotes and eukaryotes. While called untranslated, the 5' UTR or a portion of it is sometimes translated into a protein product. This product can then regulate the translation of the main coding sequence of the mRNA. In many organisms, however, the 5' UTR is completely untranslated, instead forming complex secondary structure that can regulate translation.

The average length of 5' UTRs is about 30 to about 220 nucleotides across species. In vertebrates, 5' UTRs tend to be longer in transcripts encoding transcription factors, protooncogenes, growth factors, and their receptors, and proteins that are poorly translated under normal conditions. High GC content is also a conserved feature of the 5' UTR, with values surpassing 60% in the case of warm-blooded vertebrates. In the context of hairpin structures, GC content can affect protein translation efficiency independent of hairpin thermal stability and hairpin position. UTRs of eukaryotic mRNAs also display a variety of repeats that include short and long interspersed elements (SINEs and LINEs, resp.), simple sequence repeats (SSRs), minisatellites, and macrosatellites. Translation initiation in eukaryotes requires the recruitment of ribosomal subunits at either the 5' m7G cap structure. Genes presenting differences in the 5' UTR of their transcripts are relatively common. 10-18% of genes express alternative 5' UTR by using multiple promoters while alternative splicing within UTRs is estimated to affect 13% of genes in the mammalian transcriptome. These variations in 5' UTR can function as important switches to regulate gene expression. 5' UTR can form a secondary structure, i.e., a hairpin loop, which impacts the regulation of translation.

In some embodiments, the 5' UTR does not form stable secondary sequence structure that contains a heterologous protein start codon. In some embodiments, the 5' UTR does not form stable secondary sequence structure that contains a heterologous protein start codon with a change in free energy (ΔG) below about -10 kcal/mol to about -80 kcal/mol. In specific embodiments, the change in free energy is below about -5 kcal/mol, -10kcal/mol, -20 kcal/mol, -30 kcal/mol, -40 kcal/mol, -50 kcal/mol, -60 kcal/mol, -70 kcal/mol, -80 kcal/mol, -90 kcal/mol, or below about -100 kcal/mol. In some embodiments, the 5' UTR comprises internal ribosomal entry site (IRES).

According to the invention, the 5' UTR is the 5' UTR of the ALB gene (SEQ ID NO: 1). In various embodiments, the 5' UTR comprises a UTR Kozak sequence. In some embodiments, the UTR Kozak sequence is any one of SEQ ID NOs: 50-149. In a specific embodiment, the UTR Kozak sequence comprises SEQ ID NO: 114. In some embodiments, the 5' UTR comprises a eukaryotic initiation factor (eIF) recruitment sequence.

As used herein, the term "3' untranslated region" or "3' UTR" is the section of messenger RNA (mRNA) that immediately follows the translation termination codon. On average the length for the 3' UTR in humans is approximately 800 nucleotides. The length of the 3' UTR is significant since longer 3' UTRs are associated with lower levels of gene expression. One possible explanation for this phenomenon is that longer regions have a higher probability of possessing more miRNA binding sites that have the ability to inhibit translation.

The 3' UTR often contains regulatory regions that post-transcriptionally influence gene expression. Regulatory regions within the 3' UTR can influence polyadenylation, translation efficiency, localization, and stability of the mRNA. The 3' UTR can contain both binding sites for regulatory proteins as well as microRNAs (miRNAs). By binding to specific sites within the 3' UTR, miRNAs can decrease gene expression of various mRNAs by either inhibiting translation or directly causing degradation of the transcript. The 3' UTR can also have silencer regions which bind to repressor proteins and will inhibit the expression of the mRNA. Many 3' UTRs also contain AU-rich elements (AREs). Proteins bind AREs to affect the stability or decay rate of transcripts in a localized manner or affect translation initiation. Furthermore, the 3' UTR can contain the sequence AAUAAA that directs addition of several hundred adenine residues called the poly(A) tail to the end of the mRNA transcript. Poly(A) binding protein (PABP) binds to this tail, contributing to regulation of mRNA translation, stability, and export. For example, poly(A) tail bound PABP interacts with proteins associated with the 5' end of the transcript, causing a circularization of the mRNA that promotes translation. The 3' UTR can also contain sequences that attract proteins to associate the mRNA with the cytoskeleton, transport it to or from the cell nucleus, or perform other types of localization. In addition to sequences within the 3' UTR, the physical characteristics of the region, including its length and secondary structure, contribute to translation regulation. These diverse mechanisms of gene regulation ensure that the correct genes are expressed in the correct cells at the appropriate times.

According to the invention, the 3' UTR is the 3' UTR of the SNRPB variant 1 (SEQ ID NO: 10).

As used herein, the term "Kozak sequence" is a nucleic acid motif that functions as the protein translation initiation site in most eukaryotic mRNA transcripts. The vertebrate Kozak sequences have a consensus sequence of "gcc A/G ccATGG" (SEQ ID NO: 190), wherein the upper case positions are more conserved than the lower case positions; wherein the ATG is the start codon. Therefore, Kozak sequence spans across 5' UTR and the coding sequence, wherein the portion within 5' UTR is UTR Kozak sequence. For example, a UTR Kozak sequence is the portion of the Kozak sequence from the first to the sixth base pair. In various embodiments, the first nucleotide of the Kozak sequence is A or G. In various embodiments, the second nucleotide of the Kozak sequence is C or T. In various embodiments, the third nucleotide of the Kozak sequence is A or C. In various embodiments, the fourth nucleotide of the Kozak sequence is A or G. In various embodiments, the fifth nucleotide of the Kozak sequence is A or C. In various embodiments, the sixth nucleotide of the Kozak sequence is A, C, or G. In specific embodiments, the Kozak sequence includes the sequence GCCACC that is part of a 5' UTR. In various embodiments, the seventh to tenth nucleotides of the Kozak sequence are ATGG. In specific embodiments, the Kozak sequence can include a portion of a NLS of the polynucleotide. For example, the Kozak sequence can include the sequence ATGGC that is part of the SV40 NLS. In various embodiments, a UTR Kozak sequence comprises any one of SEQ ID NOs: 50-149.

As used herein, the term "GC content" refers to the percentage of nitrogenous bases in a DNA or RNA molecule that are either guanine (G) or cytosine (C). This measure indicates the proportion of G and C bases out of an implied four total bases, also including adenine and thymine in DNA and adenine and uracil in RNA. DNA with low GC-content is less stable than DNA with high GC-content; however, the hydrogen bonds themselves do not have a particularly significant impact on molecular stability, which is instead caused mainly by molecular interactions of base stacking.

As used herein, the term "adenine or thymine content" or "AT content" refers to the percentage of nitrogenous bases in a DNA that are either adenine (A) or thymine (T), or an RNA molecule that are either adenine (A) or uracil (U). This measure indicates the proportion of A and T bases out of an implied four total bases in DNA, or the proportion of A and U bases out of an implied four total bases in RNA.

As used herein, the term "5' cap" is a specially altered nucleotide on the 5' end of some primary transcripts such as precursor messenger RNA. This process, known as mRNA capping, is highly regulated and vital in the creation of stable and mature messenger RNA able to undergo translation during protein synthesis. Mitochondrial mRNA and chloroplastic mRNA are not capped. In eukaryotes, the 5' cap found on the 5' end of an mRNA molecule, consists of a guanine nucleotide connected to mRNA via an unusual 5' to 5' triphosphate linkage. This guanosine is methylated on the 7 position directly after capping in vivo by a methyltransferase. It is referred to as a 7-methylguanylate cap, abbreviated m7G. In multicellular eukaryotes and some viruses, further modifications exist, including the methylation of the 2' hydroxy-groups of the first 2 ribose sugars of the 5' end of the mRNA. cap-1 has a methylated 2'-hydroxy group on the first ribose sugar, while cap-2 has methylated 2'-hydroxy groups on the first two ribose sugars, shown on the right. The 5' cap is chemically similar to the 3' end of an RNA molecule (the 5' carbon of the cap ribose is bonded, and the 3' unbonded). This provides significant resistance to 5' exonucleases.

As used herein, the term "indel" is a molecular biology term for an insertion or deletion of bases in the genome of an organism. In coding regions of the genome, unless the length of an indel is a multiple of three, it will produce a frameshift mutation. Indels can be contrasted with a point mutation. An indel inserts and deletes nucleotides from a sequence, while a point mutation is a form of substitution that replaces one of the nucleotides without changing the overall number in the DNA. Indels can also be contrasted with Tandem Base Mutations (TBM), which may result from fundamentally different mechanisms. Indels, being either insertions, or deletions, can be used as genetic markers in natural populations, especially in phylogenetic studies (Vali et al., BMC Genet., 2008; 9:8; Erixon et al., PLoS One, 2008; 3(1): e1386). Indel percentage can be measured using various method, for example, using ddPCR. Indel percentage can be used to evaluate the genome editing efficiency of an engineered nuclease. For example, indel percentage can be used to evaluate the genome editing efficiency of any engineered nuclease used in the instant invention, including but not limited to engineered meganuclease, zinc finger nuclease, TALEN, compact TALEN, CRISPR system nuclease, and megaTAL

As used herein, the term "heterologous" or "exogenous" in reference to a nucleotide sequence or amino acid sequence are intended to mean a sequence that is purely synthetic, that originates from a foreign species, or, if from the same species, is substantially modified from its native form in composition and/or genomic locus by deliberate human intervention.

As used herein, the term "endogenous" in reference to a nucleotide sequence or protein is intended to mean a sequence or protein that is naturally comprised within or expressed by a cell.

As used herein, the term "modification" with respect to polynucleotide refers to any insertion, deletion, or substitution of one or more than one base pairs in the polynucleotide. In some embodiments, the modification is applied to a coding sequence of a heterologous protein without changing the amino acid sequence of the heterologous protein. In some embodiments, the heterologous protein is an engineered nuclease. In some embodiments, the modification of a coding sequence of a heterologous protein comprises changing a first three base codon containing a thymidine or uridine to a second three base codon containing less thymidine or uridine without changing the amino acid sequence of the heterologous protein. In some embodiments, the modification of a coding sequence of a heterologous protein comprises changing a first three base codon containing a thymidine or uridine to a second three base codon containing no thymidine or uridine without changing the amino acid sequence of the heterologous protein. In some embodiments, the modification reduces the thymidine or uridine content of the coding sequence. In some embodiments, the modification increases the guanine or cytosine content of the coding sequence. In some embodiments, the coding sequence has between 10% and 90%, or between 20% and 80%, or between 30% and 70%, or between 40% and 60%, or between 45% and 55% reduced thymidine or uridine content compared to a coding sequence that has not been modified to reduce thymidine or uridine content. In some embodiments, the coding sequence has 40% reduced thymidine or uridine content compared to a coding sequence that has not been modified to reduce thymidine or uridine content. In various embodiments, the modification does not alter the protein level of the heterologous protein. In some embodiments, the modification results in enhanced expression of the heterologous protein. In some embodiments, the modification can enhance the in expression of the heterologous protein by at least 5%, 10%, 15%, 20%, 25%, 50%, 75%, 100%, 200%, 500%, 1000%, or more, when compared to that without the modification.

As used herein, the term "AU-rich element", "adenylate-uridylate-rich element" or "ARE" refers to a nucleic acid sequence found in the 3' untranslated region (UTR) of many mRNAs that code for proto-oncogenes, nuclear transcription factors, and cytokines. AREs are one of the most common determinants of RNA stability in mammalian cells. AREs are defined as a region with frequent adenine and uridine bases in an mRNA. AREs usually target the mRNA for rapid degradation. AREs have been divided into three classes with different sequences. The best characterized AREs have a core sequence of AUUUA within U-rich sequences (for example WWWU(AUUUA)UUUW where W is A or U). This lies within a 50-150 base sequence, repeats of the core AUUUA element are often required for function. Class I ARE AREs, like the c-fos gene, have dispersed AUUUA motifs within or near U-rich regions. Class II AREs, like the GM-CSF gene, have overlapping AUUUA motifs within or near U-rich regions. Class III elements, like the c-jun gene, are a much less well-defined class-they have a U-rich region but no AUUUA repeats.

As used therein, the term "open reading frame" refers to is a portion of a DNA molecule that, when translated into amino acids, contains no stop codons. The genetic code reads DNA sequences in groups of three base pairs, which means that a double-stranded DNA molecule can read in any of six possible reading frames--three in the forward direction and three in the reverse. A long open reading frame is likely part of a gene.

As used herein, the term "eukaryotic initiation factor (eIF) recruitment sequence" or "eIF recruitment sequence" refers to a sequence within the 5' UTR to which eIF binds. In some embodiments, the eIF recruitment sequence comprises an eIF4G recruitment sequence. In some embodiments, the eIF4G recruitment sequence comprises APT17. In some embodiments, the APT17 sequence comprises at least 80%, at least 85, at least 90%, at least 91%, at least 92%, at least 93%, at least 94%, at least 95%, at least 96%, at least 97%, at least 98%, at least 99%, or more sequence identity to SEQ ID NO: 14.

As used herein, the term "nuclear localization sequence" or "NLS" refers to generally short peptides that act as a signal fragment that mediates the transport of proteins from the cytoplasm into the nucleus. Classical NLS encompasses two categories: monopartite (MP) and bipartite NLS. Monopartite NLSs have a single cluster composed of 4-8 basic amino acids, which generally contains 4 or more positively charged residues, that is, arginine (R) or lysine (K). The characteristic motif of MP NLS is usually defined as K (K/R) X (K/R), where X can be any residue. For example, the NLS of SV40 large T-antigen is ^{126p}KKKRKV¹³² (SEQ ID NO: 15), with five consecutive positively charged amino acids (KKKRK) (SEQ ID NO: 191). Bipartite NLSs are characterized by two clusters of 2-3 positively charged amino acids that are separated by a 9-12 amino acid linker region, which contains several proline (P) residues. The consensus sequence can be expressed as R/K(X)₁₀₋₁₂KRXK. Notably, in bipartite NLSs, the upstream and downstream clusters of amino acids are interdependent and indispensable, and jointly determine the localization of the protein in the cell. Non-classical nuclear localization sequences are neither similar to canonical signals nor rich in arginine or lysine residues. Among non-classical nuclear localization sequences, the "proline-tyrosine" category was studied in the most detail. PY-NLS is characterized by 20-30 amino acids that assume a disordered structure, consisting of N-terminal hydrophobic or basic motifs and C-terminal R/K/H(X)₂₋₅PY motifs (where X₂₋₅ is any sequence of 2-5 residues). Two subclasses, hPY-NLS and bPY-NLS, were defined according to their N-terminal motifs. The hPY-NLS contains φG/A/Sφφ motifs (where φ is a hydrophobic residue), whereas bPY-NLS is enriched in basic residues. Collectively, the PY-NLS consensus corresponds to [basic/hydrophobic]-Xn- [R/H/K]-(X)2-5-PY, where X can be any residue. Human heterogeneous nuclear ribonucleoprotein A1 (hnRNP A1) is known as hPY-NLS due to its sequence 263FGNYNNQSSNFGPMKGGNFGGRSSGPY289 (SEQ ID NO: 192), which includes a hydrophobic region (²⁷³FGPM²⁷⁶) (SEQ ID NO: 193) required for its nuclear localization.

According to the invention, an NLS comprises the amino acid sequence of SEQ ID NO: 15.

As used herein, the term "wild-type" refers to the most common naturally occurring allele (i.e., polynucleotide sequence) in the allele population of the same type of gene, wherein a polypeptide encoded by the wild-type allele has its original functions. The term "wild-type" also refers to a polypeptide encoded by a wild-type allele. Wild-type alleles (i.e., polynucleotides) and polypeptides are distinguishable from mutant or variant alleles and polypeptides, which comprise one or more mutations and/or substitutions relative to the wild-type sequence(s). Whereas a wild-type allele or polypeptide can confer a normal phenotype in an organism, a mutant or variant allele or polypeptide can, in some instances, confer an altered phenotype. Wild-type nucleases are distinguishable from recombinant or non-naturally-occurring nucleases. The term "wild-type" can also refer to a cell, an organism, and/or a subject which possesses a wild-type allele of a particular gene, or a cell, an organism, and/or a subject used for comparative purposes.

As used herein, the term with respect to both amino acid sequences and nucleic acid sequences, the terms "percent identity," "sequence identity," "percentage similarity," "sequence similarity" and the like refer to a measure of the degree of similarity of two sequences based upon an alignment of the sequences that maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov/), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:33 89-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3.

As used herein, the term "recombinant DNA construct," "recombinant construct," "expression cassette," "expression construct," "chimeric construct," "construct," and "recombinant DNA fragment" are used interchangeably herein and are single or double-stranded polynucleotides. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector. In some embodiments, a recombinant DNA construct is a plasmid.

As used herein, the terms "treatment", "treating", or "treating a subject" refers to the administration of a pharmaceutical composition disclosed herein, comprising a therapeutically effective amount of the polynucleotide described herein, wherein the heterologous protein is a therapeutic protein. Methods of treatment are not part of the invention. For example, the subject can have a disease such as genetic disease, and treatment can represent genetic therapy for the treatment of the disease. Desirable effects of treatment include, but are not limited to, correcting disease-associated mutations in the subject, preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, the treatment comprises administering to a subject in need thereof a nanoparticle comprising the pharmaceutical composition described herein. In various embodiments, the heterologous protein is an engineered nuclease. In various embodiments, the engineered nuclease has increased protein level in a eukaryotic cell. In various embodiments, the engineered nuclease results indel in the eukaryotic cell.

As used herein, the term "a control polynucleotide" refers to a polynucleotide encoding the heterologous protein as described herein, but does not comprise a 5' UTR, or a 3' UTR, or both, or does not comprise the 5' UTR, or the 3' UTR, or both as described herein. In some embodiments, a control polynucleotide is an mRNA. In some embodiments, a control polynucleotide is a recombinant DNA construct. In some embodiments, a control polynucleotide is introduced into a eukaryotic cell by a lipid nanoparticle. In some embodiments, a control polynucleotide is introduced into a eukaryotic cell by a recombinant virus.

In various embodiments, a control polynucleotide does not comprise the 5' UTR of the ALB gene, or FGA gene, or FTH1 gene, or GAPDH gene, or HBA2 gene, or SNRPB V1 gene, or SNRPB 1 gene, or XBG gene. In various embodiments, a control polynucleotide does not comprise a 5' UTR comprising at least 80%, at least 85%, at least 86%, at least 87%, at least 88%, at least 89%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to any one of SEQ ID NOs: 1-7. In various embodiments, a control polynucleotide does not comprise a 5' UTR that is any one of SEQ ID NOs: 1-7. In various embodiments, a control polynucleotide does not comprise a UTR Kozak sequence. In some embodiments, a control polynucleotide does not comprise a UTR Kozak sequence that is any one of SEQ ID NOs: 50-149. In various embodiments, a control polynucleotide does not comprise the 3' UTR of the HBA2 gene, or the 3' UTR of the SNRPB V1 gene, or the 3' UTR of the SNRPB V2 gene, or the 3' UTR of the WPRE gene, or the 3' UTR of the XBG gene. In various embodiments, a control polynucleotide does not comprise a 3' UTR having at least 80%, at least 85%, at least 86%, at least 87%, at least 88%, at least 89%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to any one of SEQ ID NOs: 8-13. In various embodiments, a control polynucleotide does not comprise a 3' UTR that is any one of SEQ ID NOs: 8-13.

In some embodiments, a control polynucleotide does not comprise an NLS. In some embodiments, a control polynucleotide does not comprise an NLS comprising an amino acid sequence having at least 80%, at least 85%, at least 86%, at least 87%, at least 88%, at least 89%, at least 86%, at least 87%, at least 88%, at least 89%, at least 90%, at least 91%, at least 92%, at least 93%, at least 94%, at least 95%, at least 96%, at least 97%, at least 98%, at least 99%, or more sequence identity to any one of SEQ ID NOs: 15, 16, 17 or 18. In some embodiments, an NLS comprises an amino acid sequence of any one of SEQ ID NOs: 15-18. In some embodiments, a control polynucleotide does not comprise an NLS comprising an amino acid sequence of any one of SEQ ID NOs: 15-18.

In various embodiments, a control polynucleotide does not comprise pseudouridine or 2-thiouridine. In various embodiments, a control polynucleotide is not methylated. In various embodiments, a control polynucleotide does not comprise N1-methylpseudouridine, 5-methyluridine, or 2'-O-methyluridine.

In some embodiments, a control polynucleotide comprises the 5' UTR of the HBA2 gene (i.e., SEQ ID NO: 5) and the 3'UTR of the WPRE gene (i.e., SEQ ID NO: 13). In some embodiments, a control polynucleotide comprises an SV40 NLS (i.e., SEQ ID NO: 15). In some embodiments, a control polynucleotide comprises an N terminal SV40 NLS (i.e., SEQ ID NO: 15). In some embodiments, a control polynucleotide comprises a C-terminal SV40 NLS (i.e., SEQ ID NO: 15). In some embodiments, a control polynucleotide comprises an N terminal SV40 NLS (i.e., SEQ ID NO: 15), a the 5' UTR of the HBA2 gene (i.e., SEQ ID NO: 5), and the 3'UTR of the WPRE gene (i.e., SEQ ID NO: 13).

As used herein, the term "a control cell" refers to a cell comprising a control polynucleotide. A control cell can provide a reference point for measuring fold change of the heterologous protein level, or of the mRNA persistence. In some embodiments, the protein level of the heterologous protein is increased by about 2 to 10 fold in the eukaryotic cell compared to the control eukaryotic cell. In various embodiments, the mRNA persistence is increased by about 2 to 10 fold in the eukaryotic cell compared to the control eukaryotic cell. In some embodiments, the control cell is a mammalian cell. In some embodiments, the control cell is a human cell. In some embodiments, the control cell is part of a tissue. In some embodiments, the control cell is in a mammal. In some embodiments, the control cell is in a human.

As used herein, the term "effective amount" or "therapeutically effective amount" of a pharmaceutical composition is that amount sufficient to effect beneficial or desired results, for example, upon single or multiple dose administration to a subject cell, in curing, alleviating, relieving or improving one or more symptoms of a disorder, clinical results, and, as such, an "effective amount" depends upon the context in which it is being applied. For example, in the context of administering an agent that treats genetic disease, an effective amount of a pharmaceutical composition is, for example, an amount sufficient to achieve treatment, as defined herein, of the genetic disease, as compared to the response obtained without administration of the pharmaceutical composition.

As used herein, the term "vector" or "recombinant DNA vector" may be a construct that includes a replication system and sequences that are capable of transcription and translation of a polypeptide-encoding sequence in a given host cell. If a vector is used, then the choice of vector is dependent upon the method that will be used to transform host cells as is well known to those skilled in the art. Vectors can include, without limitation, plasmid vectors and recombinant AAV vectors, or any other vector known in the art suitable for delivering a gene to a target cell. The skilled artisan is well aware of the genetic elements that must be present on the vector in order to successfully transform, select and propagate host cells comprising any of the isolated nucleotides or nucleic acid sequences of the invention. In some embodiments, a "vector" also refers to a virus (i.e., a viral vector). Viruses can include, without limitation retroviruses, lentiviruses, adenoviruses, and adeno-associated viruses (AAVs). In some embodiments, a vector may refer to a plasmid.

### III. Engineered Nuclease

As described herein, the heterologous protein is an engineered meganuclease.

Engineered meganucleases that bind double-stranded DNA at a recognition sequence that is greater than 12 base pairs can be used for the presently disclosed methods. A meganuclease can be an endonuclease that is derived from I-CreI and can refer to an engineered variant of I-CreI that has been modified relative to natural I-CreI with respect to, for example, DNA-binding specificity, DNA cleavage activity, DNA-binding affinity, or dimerization properties. Methods for producing such modified variants of I-CreI are known in the art (e.g. WO 2007/047859). A meganuclease as used herein binds to double-stranded DNA as a heterodimer. A meganuclease may also be a "single-chain meganuclease" in which a pair of DNA-binding domains is joined into a single polypeptide using a peptide linker.

In particular embodiments, the nucleases used to practice the invention are single-chain meganucleases. A single-chain meganuclease comprises an N-terminal subunit and a C-terminal subunit joined by a linker peptide. Each of the two domains recognizes half of the recognition sequence (i.e., a recognition half-site) and the site of DNA cleavage is at the middle of the recognition sequence near the interface of the two subunits. DNA strand breaks are offset by four base pairs such that DNA cleavage by a meganuclease generates a pair of four base pair, 3' single-strand overhangs. For example, nuclease-mediated insertion using engineered single-chain meganucleases has been disclosed in International Publication Nos. WO 2017/062439 and WO 2017/062451.

### IV. mRNA-Based Chromosomal Editing Platform

Provided herein is a polynucleotide comprising a nucleic acid sequence encoding a heterologous protein, wherein the nucleic acid sequence comprises a 5' UTR, a coding sequence encoding the heterologous protein, a 3' UTR, and a polyA sequence. In various embodiments, the polynucleotide does not comprise an upstream uATG sequence or upstream open reading frame sequence.

The 5' UTR is the 5' UTR of the ALB gene (SEQ ID NO: 1). In various embodiments, the 5' UTR comprises a UTR Kozak sequence. In some embodiments, the UTR Kozak sequence comprises at least 60%, at least 70%, at least 80%, at least 90%, or at least 95% sequence identity to any one of SEQ ID NOs: 50-149. In some specific embodiments, the UTR Kozak sequence comprises any one of SEQ ID NOs: 50-149. In a specific embodiment, the UTR Kozak sequence comprises SEQ ID NO: 114.

In particular embodiments, the 3' UTR is the 3' UTR of the SNRPB variant 1 (SEQ ID NO: 10).

In various embodiments, the 5' UTR further comprises a eukaryotic initiation factor (eIF) recruitment sequence. In some embodiments, the eIF recruitment sequence comprises an eIF4G recruitment sequence. In some embodiments, the eIF4G recruitment sequence comprises APT17. In some embodiments, the APT17 comprises at least at least 60%, at least 70%, at least 80%, at least 90%, or at least 95% sequence identity to SEQ ID NO: 14. In some specific embodiments, the APT17 comprises the sequence of SEQ ID NO: 14.

In various embodiments, the 5' UTR does not form a stable secondary sequence structure that contains a heterologous protein start codon. For example, in some embodiments, the 5'UTR does not form a stable secondary sequence structure that contains a heterologous protein start codon with a change in free energy (ΔG) below about -10 kcal/mol to about -80 kcal/mol.

In various embodiments, the 5' UTR is from about 30 nucleotides to about 250 nucleotides in length. In some embodiments, the 5' UTR is 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 39 nucleotides, 40 nucleotides, 41 nucleotides, 42 nucleotides, 43 nucleotides, 44 nucleotides, 45 nucleotides, 46 nucleotides, 47 nucleotides, 48 nucleotides, 49 nucleotides, 50 nucleotides, 51 nucleotides, 52 nucleotides, 53 nucleotides, 54 nucleotides, 55 nucleotides, 56 nucleotides, 57 nucleotides, 58 nucleotides, 59 nucleotides, 60 nucleotides, 61 nucleotides, 62 nucleotides, 63 nucleotides, 64 nucleotides, 65 nucleotides, 66 nucleotides, 67 nucleotides, 68 nucleotides, 69 nucleotides, 70 nucleotides, 71 nucleotides, 72 nucleotides, 73 nucleotides, 74 nucleotides, 75 nucleotides, 76 nucleotides, 77 nucleotides, 78 nucleotides, 79 nucleotides, 80 nucleotides, 81 nucleotides, 82 nucleotides, 83 nucleotides, 84 nucleotides, 85 nucleotides, 86 nucleotides, 87 nucleotides, 88 nucleotides, 89 nucleotides, 90 nucleotides, 91 nucleotides, 92 nucleotides, 93 nucleotides, 94 nucleotides, 95 nucleotides, 96 nucleotides, 97 nucleotides, 98 nucleotides, 99 nucleotides, 100 nucleotides, 101 nucleotides, 102 nucleotides, 103 nucleotides, 104 nucleotides, 105 nucleotides, 106 nucleotides, 107 nucleotides, 108 nucleotides, 109 nucleotides, 110 nucleotides, 111 nucleotides, 112 nucleotides, 113 nucleotides, 114 nucleotides, 115 nucleotides, 116 nucleotides, 117 nucleotides, 118 nucleotides, 119 nucleotides, 120 nucleotides, 121 nucleotides, 122 nucleotides, 123 nucleotides, 124 nucleotides, 125 nucleotides, 126 nucleotides, 127 nucleotides, 128 nucleotides, 129 nucleotides, 130 nucleotides, 131 nucleotides, 132 nucleotides, 133 nucleotides, 134 nucleotides, 135 nucleotides, 136 nucleotides, 137 nucleotides, 138 nucleotides, 139 nucleotides, 140 nucleotides, 141 nucleotides, 142 nucleotides, 143 nucleotides, 144 nucleotides, 145 nucleotides, 146 nucleotides, 147 nucleotides, 148 nucleotides, 149 nucleotides, 150 nucleotides, 151 nucleotides, 152 nucleotides, 153 nucleotides, 154 nucleotides, 155 nucleotides, 156 nucleotides, 157 nucleotides, 158 nucleotides, 159 nucleotides, 160 nucleotides, 161 nucleotides, 162 nucleotides, 163 nucleotides, 164 nucleotides, 165 nucleotides, 166 nucleotides, 167 nucleotides, 168 nucleotides, 169 nucleotides, 170 nucleotides, 171 nucleotides, 172 nucleotides, 173 nucleotides, 174 nucleotides, 175 nucleotides, 176 nucleotides, 177 nucleotides, 178 nucleotides, 179 nucleotides, 180 nucleotides, 181 nucleotides, 182 nucleotides, 183 nucleotides, 184 nucleotides, 185 nucleotides, 186 nucleotides, 187 nucleotides, 188 nucleotides, 189 nucleotides, 190 nucleotides, 191 nucleotides, 192 nucleotides, 193 nucleotides, 194 nucleotides, 195 nucleotides, 196 nucleotides, 197 nucleotides, 198 nucleotides, 199 nucleotides, 200 nucleotides, 201 nucleotides, 202 nucleotides, 203 nucleotides, 204 nucleotides, 205 nucleotides, 206 nucleotides, 207 nucleotides, 208 nucleotides, 209 nucleotides, 210 nucleotides, 211 nucleotides, 212 nucleotides, 213 nucleotides, 214 nucleotides, 215 nucleotides, 216 nucleotides, 217 nucleotides, 218 nucleotides, 219 nucleotides, 220 nucleotides, 221 nucleotides, 222 nucleotides, 223 nucleotides, 224 nucleotides, 225 nucleotides, 226 nucleotides, 227 nucleotides, 228 nucleotides, 229 nucleotides, 230 nucleotides, 231 nucleotides, 232 nucleotides, 233 nucleotides, 234 nucleotides, 235 nucleotides, 236 nucleotides, 237 nucleotides, 238 nucleotides, 239 nucleotides, 240 nucleotides, 241 nucleotides, 242 nucleotides, 243 nucleotides, 244 nucleotides, 245 nucleotides, 246 nucleotides, 247 nucleotides, 248 nucleotides, 249 nucleotides, 250 nucleotides, 251 nucleotides, 252 nucleotides, 252 nucleotides, 253 nucleotides, 254 nucleotides, or 255 nucleotides in length.

In certain embodiments, the 5' UTR further comprises an internal ribosomal entry site (IRES). Internal ribosome entry site (IRES) elements are cis-acting RNA regions that promote internal initiation of protein synthesis using cap-independent mechanisms. Distinct types of IRES elements present in the genome of various RNA viruses can perform the same function despite lacking conservation of sequence and secondary RNA structure. Likewise, IRES elements can differ in host factor requirement to recruit the ribosomal subunits.

In some embodiments, the 3' UTR has less than about 3 AU-rich elements (AREs). In certain embodiments, the 3' UTR has 2 AREs. In some other embodiments, the 3' UTR has 1 ARE. In yet other embodiments, the UTR has no ARE. In some embodiments, the AU-rich element is a class I ARE. In other embodiments, the AU-rich element is a class II ARE. In yet other embodiments, the AU-rich element is a class III ARE. Class I ARE elements, like the c-fos gene, have dispersed AUUUA motifs within or near U-rich regions. Class II elements, like the GM-CSF gene, have overlapping AUUUA motifs within or near U-rich regions. Class III elements, like the c-jun gene, are a much less well-defined class-they have a U-rich region but no AUUUA repeats.

The mRNA polynucleotide can comprise a poly A tail or poly A sequence for nuclear export, translation and stability of mRNA. Polyadenylation is the addition of a poly(A) tail to an RNA transcript, typically a messenger RNA (mRNA). The poly(A) tail consists of a stretch of RNA that has only adenine bases.

In some embodiments, the polynucleotide comprises a modification to a coding sequence of the heterologous protein to reduce ribosomal stacking or stalling during protein translation of the coding sequence, wherein the modification comprises changing one or more three base codons in the coding sequence that promote ribosomal stalling to a three base codon that reduces ribosomal stalling, thereby reducing ribosomal stalling or stacking during protein translation of the heterologous protein. Ribosomal stalling or stacking can be reduced by at least 5%, 10%, 15%, 20%, 25%, 50%, 75%, 90%, or 100%, as measured by standard methods in the art. In some embodiments, the modification does not alter the amino acid sequence of the heterologous protein. In particular embodiments, the modification comprises modifying the codons encoding amino acid positions 3, 4, 5, 6, 7, 8, 9, or 10 of the coding sequence in order to reduce ribosomal stalling or stacking. In some embodiments, the modification comprises modifying the codons encoding amino acid positions 3, 4, and 5 of the coding sequence in order to reduce ribosomal stalling or stacking.

In various embodiments, the polynucleotide further comprises a modification to the coding sequence of the heterologous protein to reduce thymidine or uridine content of the coding sequence, wherein the modification does not alter the amino acid sequence of the heterologous protein.

In some embodiments, the modification comprises changing a first codon containing a thymidine or uridine that encodes an amino acid to an alternative codon that has less thymidine or uridine bases than the first codon, wherein the modification does not alter the amino acid sequence of the heterologous protein. In some embodiments, the modification comprises changing a first three base codon containing a thymidine or uridine that encodes an amino acid to an alternative three base codon that has no thymidine or uridine content, wherein the modification does not alter the amino acid sequence of the heterologous protein. In various embodiments, the modification results in between 10% and 90% reduced thymidine or uridine content in the coding sequence, wherein the modification does not alter the amino acid sequence of the heterologous protein. In some embodiments, the modification results in between 20% and 80% reduced thymidine or uridine content in the coding sequence, wherein the modification does not alter the amino acid sequence of the heterologous protein. In some embodiments, the modification results in between 30% and 70% reduced thymidine or uridine content in the coding sequence, wherein the modification does not alter the amino acid sequence of the heterologous protein. In some embodiments, the modification results in between 40% and 60% reduced thymidine or uridine content in the coding sequence, wherein the modification does not alter the amino acid sequence of the heterologous protein. In some embodiments, the modification results in about 50% reduced thymidine or uridine content in the coding sequence, wherein the modification does not alter the amino acid sequence of the heterologous protein. In a specific embodiment, the modification results in about 40% reduced thymidine or uridine content in the coding sequence, wherein the modification does not alter the amino acid sequence of the heterologous protein. In some embodiments, the first three base codon is modified to remove 1, 2, or 3 thymidine and/or uridine bases without changing the amino acid that is encoded by the codon.

In various embodiments, the polynucleotide further comprises a modification to the coding sequence of the heterologous protein to increase the GC content without altering the amino acid sequence of the heterologous protein. In some embodiments, the modification comprises changing a first three base codon containing a guanine or cytosine that encodes an amino acid to an alternative three base codon that has more guanine or cytosine than the first three base codon, wherein the modification does not alter the amino acid sequence of the heterologous protein. In some embodiments, the modification results in at least 30% CG content in the coding sequence without altering the amino acid sequence of the heterologous protein. In some embodiments, the modification results in at least 35% CG content in the coding sequence without altering the amino acid sequence of the heterologous protein. In some embodiments, the modification results in at least 40% CG content in the coding sequence without altering the amino acid sequence of the heterologous protein. In some embodiments, the modification results in at least 45% CG content in the coding sequence without altering the amino acid sequence of the heterologous protein. In some embodiments, the modification results in at least 50% CG content in the coding sequence without altering the amino acid sequence of the heterologous protein. In some embodiments, the modification results in at least 55% CG content in the coding sequence without altering the amino acid sequence of the heterologous protein. In some embodiments, the modification results in at least 60% CG content in the coding sequence without altering the amino acid sequence of the heterologous protein. In some embodiments, the modification results in at least 65% CG content in the coding sequence without altering the amino acid sequence of the heterologous protein. In some embodiments, the modification results in at least 70% CG content in the coding sequence without altering the amino acid sequence of the heterologous protein. In some embodiments, the modification results in at least 75% CG content in the coding sequence without altering the amino acid sequence of the heterologous protein. In some embodiments, the modification results in at least 80% CG content in the coding sequence without altering the amino acid sequence of the heterologous protein. In various embodiments, the modification is a codon-optimization process which can be realized, for example, through an algorithm or a software.

The heterologous protein comprises an NLS at the N-terminus and an identical NLS at the C-terminus of the heterologous protein. The NLS comprises an amino acid sequence of SEQ ID NO: 15.

In some specific embodiments, the polynucleotide comprises a 5' UTR which comprises SEQ ID NO: 1 and a UTR Kozak sequence according to any one of SEQ ID NOs: 50-149; wherein the 5'UTR does not comprise an upstream uATG sequence or upstream open reading frame sequence; wherein the heterologous protein is an engineered meganuclease comprising a first NLS at the N-terminus and a second NLS at the C-terminus of the engineered nuclease; wherein the first NLS and the second NLS are identical and comprise SEQ ID NO: 15; wherein the coding sequence of the heterologous protein has been modified to have reduced thymidine or uridine content; wherein the 3' UTR comprises SEQ ID NO: 10; and wherein the 3' UTR does not comprise any AREs.

In various embodiments, the polynucleotide is an mRNA. In some embodiments, the mRNA comprises a 5' cap. In some embodiments, the 5' cap comprises a 5' methyl guanosine cap. In some embodiments, the uridine present in the mRNA is pseudouridine or 2-thiouridine. In other embodiments, a uridine presented in the mRNA is methylated. In some embodiments, the uridine presented in the mRNA is N1-methylpseudouridine, 5-methyluridine, or 2'-O-methyluridine.

Further provided herein is a recombinant DNA construct comprising the polynucleotide. In some embodiments, the recombinant construct encodes a recombinant virus comprising the polynucleotide. Such viruses are known in the art and include recombinant retroviruses, recombinant lentiviruses, recombinant adenoviruses, and recombinant adeno-associated viruses (AAVs) (reviewed in Vannucci, et al. (New Microbiol. 2013, 36:1-22). AAVs useful in the invention can have any serotype that allows for transduction of the virus into a target cell type and expression of the heterologous protein in the target cell. In particular embodiments, AAVs have a serotype of AAV2 or AAV6. AAVs can be single-stranded AAVs or alternatively, can be self-complementary such that they do not require second-strand DNA synthesis in the host cell (McCarty, et al., Gene Ther., 2001, 8:1248-54).

Polynucleotides comprising a nucleic acid sequence encoding the heterologous protein can be delivered in DNA form (e.g. plasmid) and/or via a virus (e.g. AAV). In some embodiments, the nucleic acid sequence encoding the protein can be operably linked to a promoter. In various embodiments, the polynucleotide comprises a promoter operably linked to the nucleic acid sequence encoding the heterologous protein. "Operably linked", as used herein, is intended to mean a functional linkage between two or more elements. For example, an operable linkage between a polynucleotide of interest and a regulatory sequence (i.e., a promoter) is a functional link that allows for expression of the polynucleotide of interest. Operably linked elements may be contiguous or non-contiguous. When used to refer to the joining of two polypeptide coding regions, by operably linked is intended that the coding regions are in the same reading frame. The cassette may additionally contain at least one additional gene to be co-transformed into the organism. Alternatively, the additional gene(s) can be provided on multiple expression cassettes. Such an expression cassette is provided with a plurality of restriction sites and/or recombination sites for insertion of the polynucleotides to be under the transcriptional regulation of the regulatory regions. The expression cassette may additionally contain selectable marker genes.

A number of promoters can be used in the practice of the invention. The promoters can be selected based on the desired outcome. The encoding sequence can be combined with constitutive, tissue-specific, inducible, or other promoters for expression in the host cell. For example, a constitutive promoter can be selected from the list of, without limitation, T7AG, SV40, CMV, UBC, EF1A, PGK, ACTB, EF1α, PGK, UbC and CAGG promoters (Norman et al., PLoS ONE, 2010, 5(8): e12413; Qin et al., PLoS ONE, 2010, 5(5): e10611). In some embodiments, it can be a viral promoter such as endogenous promoters from the virus (e.g. the LTR of a lentiviral vector). In a preferred embodiment, the heterologous polypeptide coding sequence is operably linked to a promoter that drives gene expression preferentially in the target cell. In some examples, heterologous polypeptide coding sequence is operably linked to a synthetic promoter, such as a JeT promoter (US6555674).

In some embodiments, the polynucleotide is delivered through a vector, for example, a plasmid. Various plasmids can be used in the instant invention. For example, the plasmid can be one that has a nucleic acid sequence with at least 80%, at least at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or more to any one of SEQ ID NOs 21-49. In some specific embodiments, the plasmid vector can be any one of SEQ ID NOs 21-49.

Further provided herein is lipid particle comprising the polynucleotide. In some embodiments, the lipid particle is a lipid nanoparticle. In some embodiments, lipid nanoparticle comprises a polynucleotide that is an mRNA. In some embodiments, the polynucleotide encodes an engineered nuclease. As used herein, the term "lipid nanoparticle" refers to a lipid composition having a typically spherical structure with an average diameter between 10 and 1000 nanometers. In some formulations, lipid nanoparticles can comprise at least one cationic lipid, at least one non-cationic lipid, and at least one conjugated lipid. Lipid nanoparticles known in the art that are suitable for encapsulating nucleic acids, such as mRNA, are contemplated for use in the invention.

Also provided herein is a eukaryotic cell comprising the polynucleotide. The protein level of the encoded heterologous protein in the eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least700%, at least 800%, at least 900%, at least 1000%, or more compared with a control cell. In the event the polynucleotide is an mRNA, the half-life of the polynucleotide in a eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least700%, at least 800%, at least 900%, at least 1000%, or more compared with a control cell. In the event the polynucleotide is an DNA, the half-life of the mRNA produced from the polynucleotide in a eukaryotic cell comprising the polynucleotide is increased by 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least700%, at least 800%, at least 900%, at least 1000%, or more compared with a control cell.

The polynucleotide encodes an engineered meganuclease. The protein level of the encoded engineered meganuclease in the eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell. In the event the polynucleotide is an mRNA, the half-life of the polynucleotide in a eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell. In the event the polynucleotide is an DNA, the half-life of the mRNA produced from the polynucleotide in a eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell.

The eukaryotic cell comprising the polynucleotide has increased genomic editing efficiency by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell. In various embodiments, the genomic editing efficiency is measured by indel percentage.

### V. Methods of Expressing A Heterologous Protein

Methods of expressing a heterologous protein in a eukaryotic cell are provided herein comprising introducing the polynucleotide into the eukaryotic cell such that the heterologous protein is expressed in the cell. The heterologous protein is an engineered meganuclease. In various embodiments, the polynucleotide is a recombinant DNA construct as disclosed elsewhere herein. The polynucleotide can be introduced into a eukaryotic cell by a lipid nanoparticle, a recombinant virus, or any other means for introducing a polynucleotide into a cell. In some embodiments, the polynucleotide is introduced into a eukaryotic cell by a recombinant virus that is any one of a recombinant adenovirus, a recombinant lentivirus, a recombinant retrovirus, or a recombinant adeno-associated virus. In some embodiments, the heterologous protein is expressed in a eukaryotic cell, wherein the genomic editing efficiency is increased in the cell when compared with a control cell.

In some embodiments, the protein level of the heterologous protein in the eukaryotic cell is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell. In the event the polynucleotide is an mRNA, or at least about 1 fold, 2 fold, 3 fold, 4 fold, 5 fold, 6 fold, 7 fold, 8 fold, 9 fold, 10 fold, 12 fold, 13 fold, 14 fold, 15 fold or more when compared to a control cell. Likewise, the half-life of the mRNA polynucleotide in the eukaryotic cell can be increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell, or at least about 1 fold, 2 fold, 3 fold, 4 fold, 5 fold, 6 fold, 7 fold, 8 fold, 9 fold, 10 fold, 12 fold, 13 fold, 14 fold, 15 fold or more when compared to a control cell. In the event the polynucleotide is an DNA, the half-life of the mRNA produced from the DNA polynucleotide in a eukaryotic cell comprising the polynucleotide is increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell, or at least about 1 fold, 2 fold, 3 fold, 4 fold, 5 fold, 6 fold, 7 fold, 8 fold, 9 fold, 10 fold, 12 fold, 13 fold, 14 fold, 15 fold or more when compared to a control cell.

In specific embodiments, the mRNA persistence is increased by about 2 to 10 fold in the eukaryotic cell compared to the control eukaryotic cell. For example, mRNA persistence can be increased by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, at least 900%, at least 1000%, or more compared with a control cell, or at least about 1 fold, 2 fold, 3 fold, 4 fold, 5 fold, 6 fold, 7 fold, 8 fold, 9 fold, 10 fold, 12 fold, 13 fold, 14 fold, 15 fold or more when compared to a control cell. mRNA polynucleotides disclosed herein can persist in a eukaryotic cell for about 1 hour to about 96 hours. In some embodiments, the mRNA persists in the cell for about 8 hours to about 48 hours. In particular embodiments, the mRNA persists in the cell for about 1 hr, 2 hrs, 3 hrs, 4 hrs, 5 hrs, 6 hrs, 7 hrs, 8 hrs, 9 hrs, 10 hrs, 15 hrs, 20 hrs, 24 hrs, 25 hrs, 30 hrs, 35 hrs, 36 hrs, 40 hrs, 45 hrs, 48 hrs, 50 hrs, 55 hrs, 60 hrs, 65 hrs, 70 hrs, 72 hrs, 75 hrs, 80 hrs, 85 hrs, 90 hrs, 95 hrs, 100 hrs, 105 hrs, 110 hrs or more. In some embodiments, the mRNA persists in the cell for at least 8 hours. In some embodiments, the mRNA persists in the cell for at least 24 hours.

Also provided herein, but not part of the invention, is a method for treating a disease in a subject in need thereof, comprising administering a therapeutically effective amount of the polynucleotide encoding a heterologous protein disclosed herein. In some embodiments, the disease is a genetic disease. The engineered meganuclease can induce indel mutations in the subject such that the genetic mutation associated with the genetic disease is corrected and/or so that symptoms resulting from the genetic disease are reduced or ameliorated.

In some embodiments, the method for treating a disease comprises local administration of the pharmaceutical composition described herein to a subject in need thereof. In some other embodiments, the method for treating a disease comprises intravenous injection or infusion of the pharmaceutical composition described herein to a subject in need thereof. In some embodiments, the administration of the pharmaceutical composition is completed instantaneously. In some embodiments, the local administration of the pharmaceutical composition is completed instantaneously. In some embodiments, the local administration of the pharmaceutical composition is completed during a process of about 1 minute, about 2 minutes, about 3 minutes, about 4 minutes, about 5 minutes, about 10 minutes, about 20 minutes, about 30 minutes, about 40 minutes, about 50 minutes, or about 60 minutes. In some embodiments, the intravenous injection of the pharmaceutical composition is completed instantaneously. In some embodiments, the intravenous infusion of the pharmaceutical composition is completed during a process of about 1 minute, about 2 minutes, about 3 minutes, about 4 minutes, about 5 minutes, about 10 minutes, about 20 minutes, about 30 minutes, about 40 minutes, about 50 minutes, or about 60 minutes.

### VI. Pharmaceutical compositions

Also provided herein is a pharmaceutical composition comprising the polynucleotide. Such pharmaceutical compositions can be prepared in accordance with known techniques. In some embodiments, the pharmaceutical composition comprises the polynucleotide encoding the heterologous protein and a pharmaceutically acceptable carrier. In some embodiments, the pharmaceutical composition comprises a recombinant DNA construct comprising the polynucleotide encoding the heterologous protein, and a pharmaceutically acceptable carrier. In particular embodiments, the pharmaceutical composition comprises a recombinant virus comprising the polynucleotide encoding the heterologous protein, and a pharmaceutically acceptable carrier. The carrier must, of course, be acceptable in the sense of being compatible with any other ingredients in the formulation and must not be deleterious to the subject. In some embodiments, pharmaceutical compositions used in the methods and compositions disclosed herein can further comprise one or more additional agents useful in the treatment of a disease in the subject.

In some embodiments, the pharmaceutical composition comprises a recombinant virus comprising the polynucleotide encoding the heterologous protein described herein, and a pharmaceutically acceptable carrier. In some embodiments, the pharmaceutical composition includes an AAV with a concentration of between 1.0×10¹¹ and 1.0×10¹³ vector genome per milliliter. In some embodiments, the pharmaceutical composition includes a recombinant adeno-associated virus with a concentration of between 1.0×10¹¹ and 1.0×10¹³ vector genome per milliliter. In some embodiments, the pharmaceutical composition includes a recombinant retrovirus with a concentration between 1.0×10¹¹ and 1.0×10¹³ vector genome per milliliter. In some embodiments, the pharmaceutical composition includes a recombinant lentivirus with a concentration between 1.0×10¹¹ and 1.0×10¹³ vector genome per milliliter. In some embodiments, the pharmaceutical composition includes a recombinant adenovirus with a concentration between 1.0×10¹¹ and 1.0×10¹³ vector genome per milliliter.

In some embodiments, the pharmaceutical composition comprises the heterologous protein polynucleotide that is an mRNA, and a pharmaceutically acceptable carrier. In some embodiments, the composition comprising an mRNA encoding the heterologous protein comprises mRNA at a concentration of at least 0.1 mg/ml. In some embodiments, the composition comprising an mRNA encoding the heterologous protein comprises mRNA at a concentration of at least 0.2 mg/ml. In some embodiments, the composition comprising an mRNA encoding the heterologous protein comprises mRNA at a concentration of at least 0.3 mg/ml. In some embodiments, the composition comprising an mRNA encoding the heterologous protein comprises mRNA at a concentration of at least 0.4 mg/ml. In some embodiments the composition comprising an mRNA encoding the heterologous protein comprises mRNA at a concentration of at least 0.5 mg/ml. In some embodiments, the composition comprising an mRNA encoding the heterologous protein comprises mRNA at a concentration of at least 0.6 mg/ml. In some embodiments, the composition comprising an mRNA encoding the heterologous protein comprises mRNA at a concentration of at least 0.7 mg/ml. In some embodiments, the composition comprising an mRNA encoding the heterologous protein comprises mRNA at a concentration of at least 0.8 mg/ml. In some embodiments, the composition comprising an mRNA encoding the heterologous protein comprises mRNA at a concentration of at least 0.9 mg/ml. In some embodiments, the composition comprising an mRNA encoding the heterologous protein comprises mRNA at a concentration of at least 1.0 mg/ml. In some embodiments, the composition comprising an mRNA encoding the heterologous protein comprises mRNA at a concentration of at least 2.0 mg/ml. In some embodiments, the composition comprising an mRNA encoding the heterologous protein comprises mRNA at a concentration of at least 3.0 mg/ml. In some embodiments, the composition comprising an mRNA encoding the heterologous protein comprises mRNA at a concentration of at least 4.0 mg/ml. In some embodiments, the composition comprising an mRNA encoding the heterologous protein comprises mRNA at a concentration of at least 5.0 mg/ml. In some embodiments, the composition comprising an mRNA encoding the heterologous protein comprises mRNA at a concentration of at least 6.0 mg/ml. In some embodiments, the composition comprising an mRNA encoding the heterologous protein comprises mRNA at a concentration of at least 7.0 mg/ml. In some embodiments, the composition comprising an mRNA encoding the heterologous protein comprises mRNA at a concentration of at least 8.0 mg/ml. In some embodiments, the composition comprising an mRNA encoding the heterologous protein comprises mRNA at a concentration of at least 9.0 mg/ml. In some embodiments, the composition comprising an mRNA encoding the heterologous protein comprises mRNA at a concentration of at least 10.0 mg/ml. the composition comprising an mRNA encoding the heterologous protein comprises mRNA at a concentration ranging from 0.1 mg/ml to 10.0 mg/ml.

In some embodiments, the pharmaceutical composition comprises a recombinant DNA vector comprising the polynucleotide encoding the heterologous protein, and a pharmaceutically acceptable carrier. In some embodiments, the composition comprises about at least 0.1 mg/ml of the recombinant DNA vector with the polynucleotide encoding the heterologous protein. In some embodiments, the composition comprises about at least 0.2 mg/ml of the recombinant DNA vector with the polynucleotide encoding the heterologous protein. In some embodiments, the composition comprising about at least 0.3 mg/ml of the recombinant DNA vector which comprises the polynucleotide encoding the heterologous protein. In some embodiments, the composition comprising about at least 0.4 mg/ml of the recombinant DNA vector which comprises the polynucleotide encoding the heterologous protein. In some embodiments, the composition comprising about at least 0.5 mg/ml of the recombinant DNA vector which comprises the polynucleotide encoding the heterologous protein. In some embodiments, the composition comprising about at least 0.6 mg/ml of the recombinant DNA vector which comprises the polynucleotide encoding the heterologous protein. In some embodiments, the composition comprising about at least 0.7 mg/ml of the recombinant DNA vector which comprises the polynucleotide encoding the heterologous protein. In some embodiments, the composition comprising about at least 0.8 mg/ml of the recombinant DNA vector which comprises the polynucleotide encoding the heterologous protein. In some embodiments, the composition comprising about at least 0.9 mg/ml of the recombinant DNA vector which comprises the polynucleotide encoding the heterologous protein. In some embodiments, the composition comprising about at least 1.0 mg/ml of the recombinant DNA vector which comprises the polynucleotide encoding the heterologous protein. In some embodiments, the composition comprising about at least 2.0 mg/ml of the recombinant DNA vector which comprises the polynucleotide encoding the heterologous protein. In some embodiments, the composition comprising about at least 3.0 mg/ml of the recombinant DNA vector which comprises the polynucleotide encoding the heterologous protein. In some embodiments, the composition comprising about at least 4.0 mg/ml of the recombinant DNA vector which comprises the polynucleotide encoding the heterologous protein. In some embodiments, the composition comprising about at least 5.0 mg/ml of the recombinant DNA vector which comprises the polynucleotide encoding the heterologous protein. In some embodiments, the composition comprising about at least 6.0 mg/ml of the recombinant DNA vector which comprises the polynucleotide encoding the heterologous protein. In some embodiments, the composition comprising about at least 7.0 mg/ml of the recombinant DNA vector which comprises the polynucleotide encoding the heterologous protein. In some embodiments, the composition comprising about at least 8.0 mg/ml of the recombinant DNA vector which comprises the polynucleotide encoding the heterologous protein. In some embodiments, the composition comprising about at least 9.0 mg/ml of the recombinant DNA vector which comprises the polynucleotide encoding the heterologous protein. In some embodiments, the composition comprising about at least 10.0 mg/ml of the recombinant DNA vector which comprises the polynucleotide encoding the heterologous protein.

When the terms an "effective amount" or "therapeutic amount" are used herein, the precise amount to be administered can be determined by a physician with consideration of individual differences in age, weight, disease state, tumor size (if present), extent of infection or metastasis, and condition of the patient (subject). In certain embodiments, a subject may be administered the pharmaceutical composition comprising the recombinant virus of the present disclosure at a dose of about 1×10¹¹ to about 1×10¹³ vector genomes at a volume of 1 ml. In certain embodiments, a subject may be administered the pharmaceutical composition comprising the recombinant virus of the present disclosure at a dose of about 1×10¹¹ to about 1×10¹³ vector genomes at a volume of 2 ml. In certain embodiments, a subject may be administered the pharmaceutical composition comprising the recombinant virus of the present disclosure at a dose of about 1×10¹¹ to about 1×10¹³ vector genomes at a volume of 3 ml. In certain embodiments, a subject may be administered the pharmaceutical composition comprising the recombinant virus of the present disclosure at a dose of about 1×10¹¹ to about 1×10¹³ vector genomes at a volume of 4 ml. In certain embodiments, a subject may be administered the pharmaceutical composition comprising the recombinant virus of the present disclosure at a dose of about 1×10¹¹ to about 1×10¹³ vector genomes at a volume of 5 ml. The optimal dosage and treatment regime for a particular patient can readily be determined by one skilled in the art of medicine by monitoring the patient for signs of disease and adjusting the treatment accordingly.

In certain embodiments, the pharmaceutical composition comprising the mRNA is administered to a subject at a dose comprising about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, or about 100 mg the mRNA. The optimal dosage and treatment regime for a particular patient can readily be determined by one skilled in the art of medicine by monitoring the patient for signs of disease and adjusting the treatment accordingly.

In certain embodiments, the pharmaceutical composition comprising the recombinant DNA vector is administered to a subject at a dose comprising about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, or about 100 mg the DNA vector. The optimal dosage and treatment regime for a particular patient can readily be determined by one skilled in the art of medicine by monitoring the patient for signs of disease and adjusting the treatment accordingly.

In certain embodiments, the pharmaceutical composition comprising the polynucleotide of the present disclosure may be administered via a single dose intravenous delivery. In certain embodiments, the single dose intravenous delivery may be a one-time treatment. The single dose intravenous delivery can produce durable relief for subjects with genetic disease and/or related symptoms. The relief may last for minutes such as, but not limited to, 1, 2, 3, 4, 5, 6, 7, 8, 9, 10, 11, 12, 13, 14, 15, 16, 17, 18, 19, 20, 21, 22, 23, 24, 25, 26, 27.28, 29.30, 31, 32, 33, 34, 35, 36, 37, 38, 39, 40, 41, 42, 43, 44, 45, 46, 47, 48, 49, 50, 51, 52, 53, 54, 55, 56, 57, 58, 59 minutes or more than 59 minutes: hours such as, but not limited to, 1, 2, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more than 48 hours; days such as, but not limited to, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or more than 31 days; weeks such as, but not limited to, 1, 2, 3, 4, 5, 6, 7, 8, 9, 10, 11, 12, 13, 14, 15, 16, or more than 16 weeks; months such as, but not limited to, 1, 2, 3, 4, 5, 6, 7, 8, 9, 10, 11, 12, 13, 14, 15, 16, 17, 18, 19, 20, 21, 22, 23, 24, or more than 24 months; years such as, but not limited to, 1, 2, 3, 4, 5, 6, 7, 8, 9, 10, 11, 12, 13, 14, 15, or more than 15 years. In other embodiments, the pharmaceutical composition comprising the polynucleotide of the present disclosure may be administered via multiple doses of intravenous delivery.

### EXAMPLES

This invention, which is defined by the appended claims, is further illustrated by the following examples, which should not be construed as limiting. Those skilled in the art will recognize, or be able to ascertain, using no more than routine experimentation, numerous equivalents to the specific substances and procedures described herein.

### EXAMPLE 1

### Effect of ribosomal recruitment sequence APT 17 on the editing of an engineered meganuclease recognition sequence HAO 1-2 in human cell lines

### 1. Methods and Materials

These studies were conducted using in vitro cell-based systems to evaluate whether the improved mRNA designs increased the in vitro editing efficiencies of an engineered meganuclease designed to target a recognition sequence within the human HAO gene using an indel detection assay. The engineered meganuclease used in this experiment was HAO 1-2L.30S19 that has previously been described in PCT International Publication No. WO 2020/132659.

In these experiments, several different mRNA designs were tested to evaluate the effect of a ribosomal recruitment sequence on in vitro editing efficiency. The mRNA according to Table 1 was electroporated into human cells (HEK293 at 2 ng) using the Lonza Amaxa 4D system. All coding sequences for the meganucleases were further modified using alternative codon sequences to reduce uridine content, while leaving the amino acid sequence identical. Each mRNA contained N1-methylpseudouridine and a 7-methylguanosine cap. The recruiting sequence only mRNA had the recruiter sequence linked to a Kozak sequence (GGCCCCATGGC, SEQ ID NO: 145).

**Table 1. Example mRNA of Example 1**

| Name | Promoter | Recruitment Sequence | 5' UTR | N-term NLS | Meganuclease | 3' UTR |
|---|---|---|---|---|---|---|
| RS HBA2 (SEQ ID NO: 21) | T7AG | APT17 | HBA2 | SV40 | HAO1-2L.30S19 | WPRE |
| RS Only (SEQ ID NO: 22) | T7AG | APT17 | - | SV40 | HAO1-2L.30S19 | WPRE |
| Control (SEQ ID NO: 27) | T7AG | - | HBA2 | SV40 | HAO1-2L.30S19 | WPRE |

Cells were collected at 2.5, 5, and 24 hours post electroporation for gDNA preparation and evaluated for transfection efficiency using a Beckman Coulter CytoFlex S cytometer. Transfection efficiency exceeded 90%. gDNA was prepared using the Macherey Nagel NucleoSpin Blood QuickPure kit.

Digital droplet PCR was utilized to determine the frequency of target insertions and deletions (indel%) using primers P1, F1, and R1 at the HAO 1-2 recognition sequence, as well as primers P2, F2, R2 to generate a reference amplicon. Amplifications were multiplexed in a 20uL reaction containing 1x ddPCR Supermix for Probes (no dUTP, BioRad), 250nM of each probe, 900nM of each primer, 5U of HindIII-HF, and about 50ng cellular gDNA. Droplets were generated using a QX100 droplet generator (BioRad). Cycling conditions for HAO 1-2 were as follows: 1 cycle of 95°C (2°C/s ramp) for 10 minutes, 44 cycles of 95°C (1°C/s ramp) for 30 seconds, 62°C (1°C/s ramp) for 30 seconds, 72C (0.2°C/s ramp) for 2 minutes, 1 cycle of 98°C for 10 minutes, 4°C hold.

Droplets were analyzed using a QX200 droplet reader (BioRad) and QuantaSoft analysis software (BioRad) was used to acquire and analyze data. Indel frequencies were calculated by dividing the number of positive copies for the binding site probe by the number of positive copies for the reference probe and comparing loss of FAM+ copies in nuclease-treated cells to mock-transfected cells.

### Primer Sets

P1: 42 HAO1 2 BHQ 1 BS PROBE: TTCCTCACCAATGTCTTGT FAM (SEQ ID NO: 150)
F1: 28-HAO21-22 F2: CCACATAAGATTTGGCAAGCC (SEQ ID NO: 151)
R1: 27-HAO21-22 R2: GGAAAAGAACGACACCCTTTG (SEQ ID NO: 152)
P2: 33 HAO23/24 P1 REF: CCCGGCTAATTTGTATCA VIC (SEQ ID NO: 153)
F2: 29-HAO23-24 f1: GCTCACTTGATGTAAGCAACAG (SEQ ID NO: 154)
R2: 32-HAO23-24 R2: ACACACCACCAACGTAAAAC (SEQ ID NO: 155)

### 2. Results

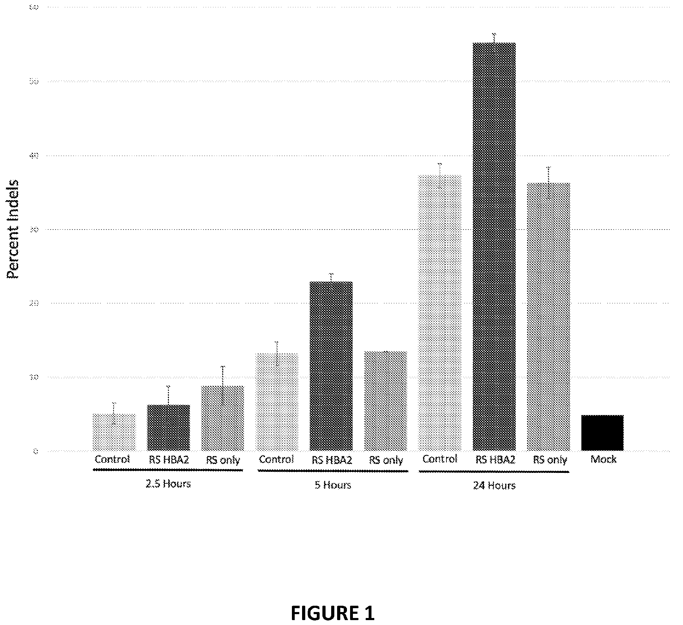

In these studies, indels (insertions and deletions) were measured by ddPCR across multiple timepoints. In HEK293 cells, the low 2 ng mRNA dose of the control mRNA showed indels ranging from 5% at 2.5 hours to 13% at 5 hours to 37% at 24 hours. Indels for the RS HBA2 mRNA ranged from 6%, 22% and 55% across time points, with indels from HAO1-RS only mRNA at 5%, 13%, and 36% at the same time points (FIG. 1).

### 3. Conclusions

These studies demonstrate the ability of the improved mRNA encoding engineered meganucleases to generate indels at the HAO 1-2 recognition sequence in human cell lines in vitro. MRNA encoding meganucleases containing variations of the recruiting sequence were compared directly to a meganuclease that targets the HAO 1-2 site without the recruiting sequence, and in the case of the recruiting sequence linked to a UTR, the RS HBA2 mRNA encoding the same HAO 1-2 nuclease had a higher editing efficiency at 5 and 24 hours than did the control or RS only linked mRNAs in the human cell line, indicating that a ribosomal recruiting sequence addition to the mRNA may improve protein expression and concomitant gene editing efficiency.

### EXAMPLE 2

### The effect of different UTRs on the editing of an engineered meganuclease recognition sequence F8R 17-18 in human cell lines

### 1. Methods and Materials

These studies were conducted using in vitro cell-based systems to evaluate whether the improved mRNA designs increased the in vitro editing efficiencies of an engineered meganuclease designed to target a recognition sequence within the human F8R gene by digital PCR using an indel detection assay. The engineered meganuclease used in this experiment was the F8R 17-18L.1.35 meganuclease that has previously been described in PCT International Publication No. WO 2019/089913.

In this experiment, mRNAs encoding the F8R 17-18L1.35 meganuclease according to Table 2 were electroporated into BNL C.2 cells (200ng or 20 ng) using the Lonza Amaxa 4D system.

**Table 2**

| Name | Promoter | 5' UTR | N-term NLS | Meganuclease | 3' UTR |
|---|---|---|---|---|---|
| HBA2/WPRE Control (SEQ ID NO: 27 ) | T7AG | HBA2 | SV40 | F8R 17-18L.1.35 | WPRE |
| HSD 17B4/HBA2 | T7AG | HSD 17B4 | SV40 | F8R 17-18L.1.35 | HBA2 |
| MOD/HBA2 | T7AG | MOD | SV40 | F8R 17-18L.1.35 | HBA2 |

Cells were collected 24 hours post electroporation for gDNA preparation and evaluated for transfection efficiency using a Beckman Coulter CytoFlex S cytometer. Transfection efficiency exceeded 90%. gDNA was prepared using the Macherey Nagel NucleoSpin Blood QuickPure kit.

Digital droplet PCR was utilized to determine the frequency of target insertions and deletions (indel%) using primers P1, F1, and R1 at the F8R17-18 recognition sequence, as well as primer P2 to generate a reference. Amplifications were multiplexed in a 20uL reaction containing 1x ddPCR Supermix for Probes (no dUTP, BioRad), 250nM of each probe, 900nM of each primer, 5U of HindIII-HF, and about 50ng cellular gDNA. Droplets were generated using a QX100 droplet generator (BioRad). Cycling conditions for F8R17-18 were as follows: 1 cycle of 95°C (2°C/s ramp) for 10 minutes, 44 cycles of 94°C (2°C/s ramp) for 30 seconds, 56°C (2°C/s ramp) for 30 seconds, 72C (2°C/s ramp) for 2 minutes, 1 cycle of 98°C for 10 minutes, 4°C hold.

Droplets were analyzed using a QX200 droplet reader (BioRad) and QuantaSoft analysis software (BioRad) was used to acquire and analyze data. Indel frequencies were calculated by dividing the number of positive copies for the binding site probe by the number of positive copies for the reference probe and comparing loss of HEX+ copies in nuclease-treated cells to mock-transfected cells.

### Primer Sets

P1: 720 F8R17-18 BS PROBE: CCTCCCAGGAGTACTTCTCCAGG HEX (SEQ ID NO: 156)
F1: 721 F8R17-18 FWD1 F1: GATGCCTTCAGTGTCCTT (SEQ ID NO: 157)
R1: 724 F8R17-18REV2 R1: CTTTGCTGACGTCCTAGT (SEQ ID NO: 158)
P2: 771 F8R17-18REF2 PROBE: TACACGGGACACCTCACACCTG FAM (SEQ ID NO: 159)

### 2. Results

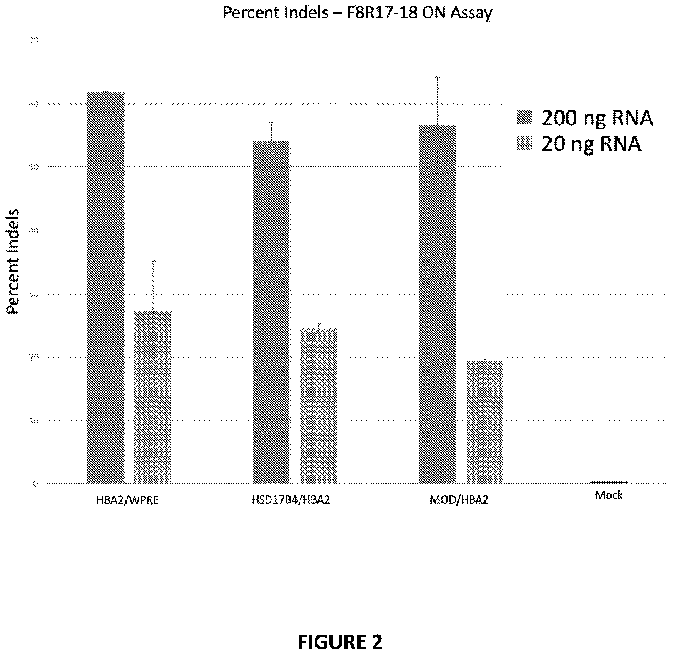

In these studies, indels (insertions and deletions) were measured by ddPCR at 24 hours. In BNL C.2 cells at 200 or 20 ng of mRNA, the high mRNA dose of F8R17-18L1.35 HBA2 showed indels >60% at 24 hours. Indels for F8R17-18L1.35 HSD17B4 at 24 hours were >50%, and indels from F8R17-18L1.35 MOD >55%. The low mRNA dose of F8R17-18L1.35 HBA2 showed indels >25% at 24 hours. Indels for F8R17-18L1.35 HSD17B4 at 24 hours were 25%, and indels from F8R17-18L1.35 MOD <20% (FIG. 2).

### 3. Conclusions

These studies demonstrate the ability of the F8R17-18 meganucleases to generate indels at the F8R17-18 recognition sequence in vitro. mRNA encoding meganucleases containing variations of the 5' UTR sequence either HSD 17B4 or MOD were compared directly to a control mRNA containing the 5' HBA2 UTR and 3' WPRE UTR, and in all cases at the high or low mRNA doses, the control mRNA had a higher or similar editing efficiency to the other combinations of UTRs. These results indicated that these combinations of test UTRs were not superior to the 5'HBA2 UTR and 3' WPRE UTR combination.

### EXAMPLE 3

### The effect of different UTRs on the editing of an engineered meganuclease recognition sequence HAO 1-2 in human cell lines

### 1. Methods and Materials

These studies were conducted using in vitro cell-based systems to evaluate whether the improved mRNA designs increased the in vitro editing efficiencies of an engineered meganuclease designed to target a recognition sequence within the human HAO gene by digital PCR using an indel detection assay. The engineered meganuclease used in this experiment was HAO 1-2L.30S19 that has previously been described in PCT International Publication No. WO 2020/132659.

In these experiments, mRNAs encoding the HAO1-2L.30 S19 meganuclease according to Table 3 testing different 5' and 3' UTR combinations were electroporated into human cells (HEP3B, 2ng) using the Lonza Amaxa 4D system. All coding sequences for the meganucleases were further modified using alternative codon sequences to reduce uridine content, while leaving the amino acid sequence identical. Each mRNA contained N1-methylpseudouridine and a 7-methylguanosine cap.

**Table 3. Example 3 mRNA**

| Name | Promoter | 5' UTR | N-term NLS | Meganuclease | 3' UTR |
|---|---|---|---|---|---|
| HBA2/WPRE Control (SEQ ID NO: 27) | T7AG | HBA2 | SV40 | HAO1-2L.30S19 | WPRE |
| XBG/XBG NLS5 (SEQ ID NO: 23) | T7AG | XBG | NLS5 | HAO1-2L.30S19 | XBG |
| XBG/XBG SV40 (SEQ ID NO: 24) | T7AG | XBG | SV40 | HAO1-2L.30S19 | XBG |
| SNRPBV1 (SEQ ID NO: 25) | T7AG | SNRPBV1 | SV40 | HAO1-2L.30S19 | SNRPBV1 |
| SNRPBV2 (SEQ ID NO: 26) | T7AG | SNRPBV1 | SV40 | HAO1-2L.30S19 | SNRPBV2 |
| HBA2/HBA2 (SEQ ID NO: 28) | T7AG | HBA2 | SV40 | HAO1-2L.30S19 | HBA2 |

Cells were collected at either one day and two days or only two days post electroporation for gDNA preparation and evaluated for transfection efficiency using a Beckman Coulter CytoFlex S cytometer. Transfection efficiency exceeded 90%. Two additional time points were collected at between 6 and 9-days post electroporation for gDNA extractions. gDNA was prepared using the Macherey Nagel NucleoSpin Blood QuickPure kit.

Digital droplet PCR was utilized to determine the frequency of target insertions and deletions (indel%) using primers P1, F1, and R1 at the HAO 1-2 recognition sequence, as well as primers P2, F2, R2 to generate a reference amplicon external of the HAO 1-2 recognition sequence (OFF amplicon ddPCR). In addition, a separate digital droplet PCR was utilized to determine the frequency of target insertions and deletions (indel%) using primers P1, F1, R1, and P3 at the HAO 1-2 recognition sequence. In this ddPCR primer P3 is used as an internal amplicon reference (ON amplicon ddPCR). Amplifications were multiplexed in a 20uL reaction containing 1x ddPCR Supermix for Probes (no dUTP, BioRad), 250nM of each probe, 900nM of each primer, 5U of HindIII-HF, and about 50ng cellular gDNA. Droplets were generated using a QX100 droplet generator (BioRad). Cycling conditions for HAO 1-2 (OFF) were as follows: 1 cycle of 95°C (2°C/s ramp) for 10 minutes, 44 cycles of 95°C (1°C/s ramp) for 30 seconds, 62°C (1°C/s ramp) for 30 seconds, 72C (0.2°C/s ramp) for 2 minutes, 1 cycle of 98°C for 10 minutes, 4°C hold. Cycling conditions for HAO 1-2 (ON) were as follows: 1 cycle of 95°C (2°C/s ramp) for 10 minutes, 44 cycles of 95°C (1°C/s ramp) for 30 seconds, 61°C (1°C/s ramp) for 30 seconds, 72C (0.2°C/s ramp) for 2 minutes, 1 cycle of 98°C for 10 minutes, 4°C hold.

Droplets were analyzed using a QX200 droplet reader (BioRad) and QuantaSoft analysis software (BioRad) was used to acquire and analyze data. Indel frequencies were calculated by dividing the number of positive copies for the binding site probe by the number of positive copies for the reference probe and comparing loss of FAM+ copies in nuclease-treated cells to mock-transfected cells.

### Primer Sets

P1: 42 HAO1 2 BHQ 1 BS PROBE: TTCCTCACCAATGTCTTGT FAM (SEQ ID NO: 150)
F1: 28-HAO21-22 F2: CCACATAAGATTTGGCAAGCC (SEQ ID NO: 151)
R1: 27-HAO21-22 R2: GGAAAAGAACGACACCCTTTG (SEQ ID NO: 152)
P2: 33 HAO23/24 P1 REF: CCCGGCTAATTTGTATCA VIC (SEQ ID NO: 153)
F2: 29-HAO23-24 f1: GCTCACTTGATGTAAGCAACAG (SEQ ID NO: 154 )
R2: 32-HAO23-24 R2: ACACACCACCAACGTAAAAC (SEQ ID NO: 155)
P3: 44 HAO12 Ref PROBE1: TGTGGTCACCCTCTGCACAGTGT HEX (SEQ ID NO: 160)

### 2. Results

In FIG. 3A using the "ON" ddPCR indel assay, the HBA2/WPRE control mRNA provided between about 10% to 15% indels from day 2 to day 9. The XBG/XBG NLS5 mRNA and HBA2/HBA2 mRNA performed similarly. In contrast the XBG/XBG SV40 (SEQ ID NO: 24) mRNA generated indels ranging from greater than 20% to about 30% from day 2 to day 9. The SNRPB V1 mRNA generated indels ranging from about 15% to about 23% from day 2 to day 9 and the SNRPBV2 mRNA generated indels from about 13% to about 18%. Similar results using the same tested mRNAs were obtained in FIG. 3B, 3C, and 3D.

### 3. Conclusions

These studies demonstrate the ability of the HAO 1-2 meganucleases to generate indels at the HAO 1-2 recognition sequence in a human cell line in vitro. MRNA encoding meganucleases containing variations of the 5' and 3' UTR sequences had increased indels compared to the control HBA2/WPRE mRNA. Using the NLS5 N terminal NLS reduced the percentage of detected indels significantly at all time points indicating that the SV40 NLS may be superior when used with an engineered nuclease that needs to migrate to the nuclease in order to perform its function of cleaving DNA. In addition, the addition of the SNRPBV2 3' UTR decreased indel% slightly. This finding may be due to the presence of an AU rich element found in the 3' UTR.

### EXAMPLE 4

### The effect of different UTRs on the editing of an engineered meganuclease recognition sequence HAO 1-2 in human cell lines

### 1. Methods and Materials

These studies were conducted using in vitro cell-based systems to evaluate whether the improved mRNA designs increased the in vitro editing efficiencies of an engineered meganuclease designed to target a recognition sequence within the human HAO gene by digital PCR using an indel detection assay. The engineered meganuclease used in this experiment was HAO 1-2L.30S19 that has previously been described in PCT International Publication No. WO 2020/132659

In these experiments, mRNAs encoding the HAO1-2L.30 S19 meganuclease with additional variable 5' and 3' UTRs according to Table 4 were electroporated into human cells (HEP3B, 2ng) using the Lonza Amaxa 4D system. All coding sequences for the meganucleases were further modified using alternative codon sequences to reduce uridine content, while leaving the amino acid sequence identical. Each mRNA contained N1-methylpseudouridine and a 7-methylguanosine cap.

**Table 4. Example 4 mRNA**

| Name | Promoter | 5' UTR | N-term NLS | Meganuclease | 3' UTR |
|---|---|---|---|---|---|
| HBA2.WPRE (SEQ ID NO: 27) | T7AG | HBA2 | SV40 | HAO1-2L.30S19 | WPRE |
| HBA2.HBA2 (SEQ ID NO: 28) | T7AG | HBA2 | SV40 | HAO1-2L.30S19 | HBA2 |
| XBG.XBG (SEQ ID NO: 24) | T7AG | XBG | SV40 | HAO1-2L.30S19 | XBG |
| SNRPB.SNRPB1 (SEQ ID NO: 25) | T7AG | SNRPBV1 | SV40 | HAO1-2L.30S19 | SNRPBV1 |
| ALB.XBG (SEQ ID NO: 30) | T7AG | ALB | SV40 | HAO1-2L.30S19 | XBG |
| FGA.XBG (SEQ ID NO: 31) | T7AG | FGA | SV40 | HAO1-2L.30S19 | XBG |
| FTH1.XBG (SEQ ID NO: 32) | T7AG | FTH1 | SV40 | HAO1-2L.30S19 | XBG |
| GAPDH.XBG (SEQ ID NO: 33) | T7AG | GAPDH | SV40 | HAO1-2L.30S19 | XBG |
| HBA2.XBG (SEQ ID NO: 34) | T7AG | HBA2 | SV40 | HAO1-2L.30S19 | XBG |
| SNRPB.XBG (SEQ ID NO: 35) | T7AG | SNRPBV1 | SV40 | HAO1-2L.30S19 | XBG |
| XBG.HBA2 (SEQ ID NO: 36) | T7AG | XBG | SV40 | HAO1-2L.30S19 | HBA2 |
| XBG.HBB (SEQ ID NO: 37) | T7AG | XBG | SV40 | HAO1-2L.30S19 | HBB |
| XBG.SNRPB1 (SEQ ID NO: 38) | T7AG | XBG | SV40 | HAO1-2L.30S19 | SNRPBV1 |
| ALB.SNRPB1 (SEQ ID NO: 39) | T7AG | ALB | SV40 | HAO1-2L.30S19 | SNRPBV1 |
| FGA.SNRPB1 (SEQ ID NO: 40) | T7AG | FGA | SV40 | HAO1-2L.30S19 | SNRPBV1 |
| FTH1.SNRPB1 (SEQ ID NO: 41) | T7AG | FTH1 | SV40 | HAO1-2L.30S19 | SNRPBV1 |
| GAPDH.SNRPB1 (SEQ ID NO: 42) | T7AG | GAPDH | SV40 | HAO1-2L.30S19 | SNRPBV1 |
| HBA2.SNRPB1 (SEQ ID NO: 43) | T7AG | HBA2 | SV40 | HAO1-2L.30S19 | SNRPBV1 |
| SNRPB.HBA2 (SEQ ID NO: 44) | T7AG | SNRPBV1 | SV40 | HAO1-2L.30S19 | HBA2 |
| SNRPB.HBB (SEQ ID NO: 45) | T7AG | SNRPBV1 | SV40 | HAO1-2L.30S19 | HBB |

Cells were collected at two days post electroporation for gDNA preparation and evaluated for transfection efficiency using a Beckman Coulter CytoFlex S cytometer. Transfection efficiency exceeded 90%. Two additional time points were collected at between 6 and 9-days post electroporation for gDNA extractions. gDNA was prepared using the Macherey Nagel NucleoSpin Blood QuickPure kit.

Digital droplet PCR to determine the frequency of target insertions and deletions (indel%) for both the "ON" and "OFF" assay was conducted as described in Example 3.

### 2. Results

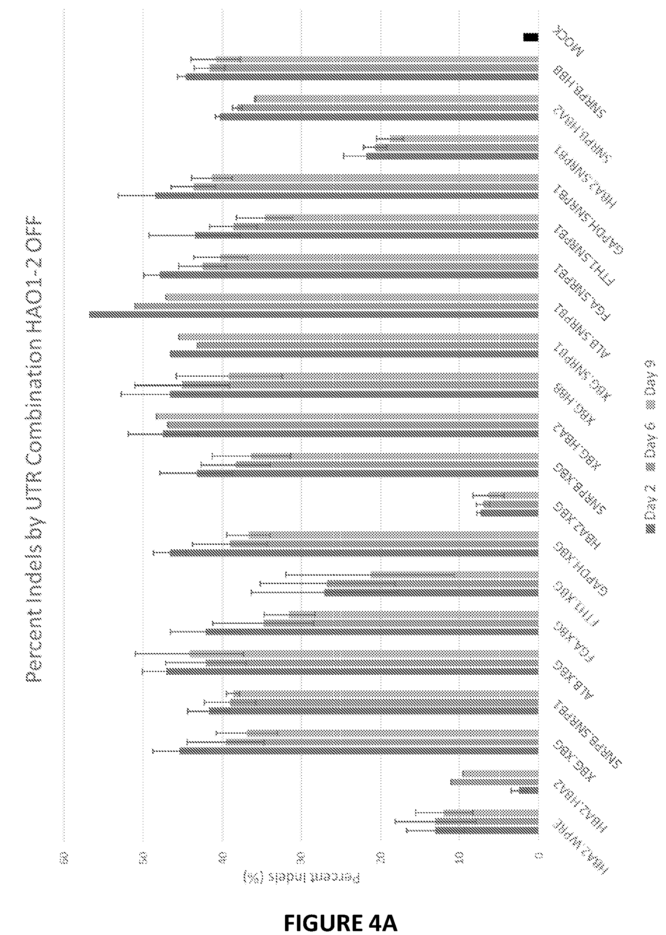

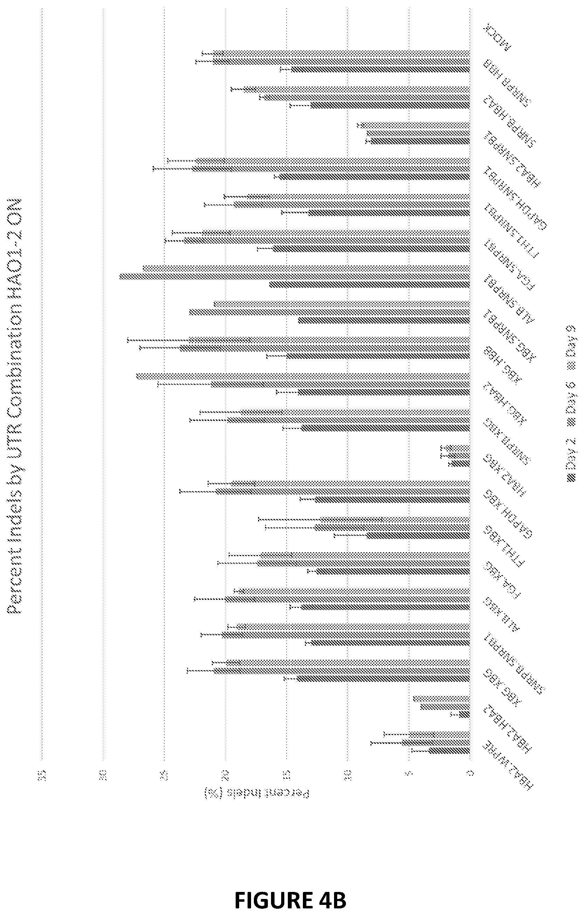

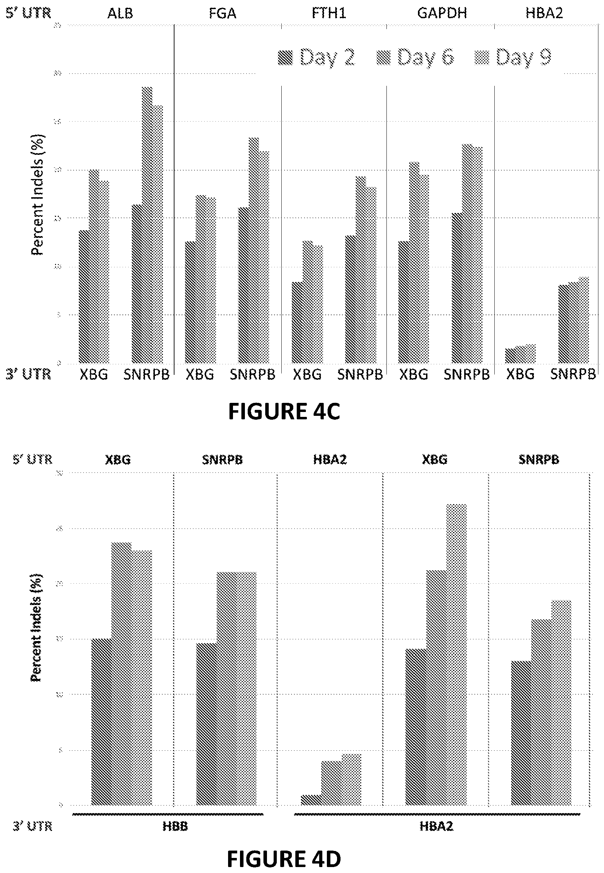

In these studies, indels (insertions and deletions) were measured by ddPCR across multiple timepoints in HEP3B cells at a 2ng mRNA using two biological replicates. Experimental data for HAO1-2 "OFF" amplicon assay is provided in Table 5 and in Figure 4A. Experimental data for HAO1-2 "ON" amplicon assay is provided in Table 6 and in Figure 4B. The data for this experiment was re-arranged to visualize any potential trend in 5' or 3' UTR selection as shown in Figures 4C and 4D.

**Table 5. Indel% by UTR combination for the HAO1-2 OFF ddPCR assay**

| **UTR Combination** | **Day2 Indel %** | **Day6 Indel %** | **Day9 Indel %** |
|---|---|---|---|
| HBA2.WPRE | 13.1 | 13.1 | 12.0 |
| HBA2.HBA2 | 2.5 | 11.1 | 9.6 |
| XBG.XBG | 45.5 | 39.6 | 36.9 |
| SNRPB.SNRPB1 | 41.8 | 39.1 | 38.7 |
| ALB.XBG | 47.1 | 42.1 | 44.2 |
| FGA.XBG | 42.2 | 34.8 | 31.5 |
| FTH1.XBG | 27.1 | 26.7 | 21.3 |
| GAPDH.XBG | 46.7 | 39.1 | 36.7 |
| HBA2.XBG | 7.4 | 7.1 | 6.4 |
| SNRPB.XBG | 43.3 | 38.3 | 36.4 |
| XBG.HBA2 | 47.5 | 46.9 | 48.3 |
| XBG.HBB | 46.7 | 45.1 | 39.2 |
| XBG.SNRPB1 | 46.6 | 43.2 | 45.5 |
| ALB.SNRPB1 | 56.8 | 51.1 | 47.2 |
| FGA.SNRPB1 | 47.9 | 42.5 | 40.3 |
| FTH1.SNRPB1 | 43.5 | 38.6 | 34.7 |
| GAPDH.SNRPB1 | 48.5 | 43.7 | 41.4 |
| HBA2.SNRPB1 | 21.9 | 20.7 | 18.8 |
| SNRPB.HBA2 | 40.4 | 38.1 | 35.9 |
| SNRPB.HBB | 44.7 | 41.7 | 40.9 |

**Table 6. Indel% by UTR combination for the HAO1-2 ON ddPCR assay**

| **UTR Combination** | **Day2 Indel %** | **Day6 Indel %** | **Day9 Indel %** |
|---|---|---|---|
| HBA2.WPRE | 3.4 | 5.6 | 5.0 |
| HBA2.HBA2 | 1.0 | 4.0 | 4.7 |
| XBG.XBG | 14.2 | 21.0 | 20.0 |
| SNRPB.SNRPB1 | 13.0 | 20.3 | 19.1 |
| ALB.XBG | 13.8 | 20.1 | 18.9 |
| FGA.XBG | 12.6 | 17.4 | 17.2 |
| FTH1.XBG | 8.5 | 12.7 | 12.3 |
| GAPDH.XBG | 12.7 | 20.8 | 19.5 |
| HBA2.XBG | 1.6 | 1.9 | 2.0 |
| SNRPB.XBG | 13.8 | 19.9 | 18.8 |
| XBG.HBA2 | 14.1 | 21.2 | 27.2 |
| XBG.HBB | 15.0 | 23.7 | 23.0 |
| XBG.SNRPB1 | 14.0 | 22.9 | 20.9 |
| ALB.SNRPB1 | 16.4 | 28.6 | 26.7 |
| FGA.SNRPB1 | 16.1 | 23.4 | 22.0 |
| FTH1.SNRPB1 | 13.3 | 19.4 | 18.3 |
| GAPDH.SNRPB1 | 15.6 | 22.7 | 22.4 |
| HBA2.SNRPB1 | 8.1 | 8.4 | 9.0 |
| SNRPB.HBA2 | 13.1 | 16.8 | 18.5 |
| SNRPB.HBB | 14.6 | 21.1 | 21.1 |

### 3. Conclusions

These studies demonstrate the ability of the HAO 1-2 meganucleases to generate indels at the HAO 1-2 recognition sequence in a human cell line in vitro. MRNA encoding the HAO 1-2 meganuclease containing variations of the 5' and 3' UTR sequences were compared directly to a control mRNA having the 5'HBA2 UTR and 3' WPRE UTR. In most cases the control mRNA resulted in a considerably lower editing efficiency than mRNA having the unique combinations of UTRs tested. Notably, the 5' HBA2 UTR and 3' XBG UTR performed significantly worse than the control mRNA. It was observed that the SNRPB V1 3' UTR led to increased indel % for each of the paired 5' UTRs tested when compared to using a 3' XBG UTR (Figure 4C). Further, in the case of the 5' UTR, XBG tends to generate higher indels over the 5' SNRPB V1 UTR. In addition, the use of the 5'HBA2 UTR typically led to a significant decrease in activity.

### EXAMPLE 5

### The effect of different UTRs on the editing of an engineered meganuclease recognition sequence HAO 1-2 in human cell lines across differing dosages

### 1. Methods and Materials

These studies were conducted using in vitro cell-based systems to evaluate whether the improved mRNA designs increased the in vitro editing efficiencies of an engineered meganuclease designed to target a recognition sequence within the human HAO gene by digital PCR using an indel detection assay. The engineered meganuclease used in this experiment was HAO 1-2L.30S19 that has previously been described in PCT International Publication No. WO 2020/132659.

In these experiments, mRNAs encoding the HAO1-2L.30 S19 meganuclease with additional variable 5' and 3' UTRs according to Table 7 were electroporated into human cells (HEP3B at 2ng, 1ng, 9.5ng, and 0.25ng) using the Lonza Amaxa 4D system. Digital droplet PCR to determine the frequency of target insertions and deletions (indel%) for both the "ON" and "OFF" assay was conducted as described in Example 3. All coding sequences for the meganucleases were further modified using alternative codon sequences to reduce uridine content, while leaving the amino acid sequence identical. Each mRNA contained N1-methylpseudouridine and a 7-methylguanosine cap.

**Table 7. Example 5 mRNA**

| Name | Promoter | 5' UTR | N-term NLS | Meganuclease | 3' UTR |
|---|---|---|---|---|---|
| HBA2.WPRE (SEQ ID NO: 27) | T7AG | HBA2 | SV40 | HAO1-2L.30S19 | WPRE |
| XBG.XBG (SEQ ID NO: 24) | T7AG | XBG | SV40 | HAO1-2L.30S19 | XBG |
| XBG.HBB (SEQ ID NO: 37) | T7AG | XBG | SV40 | HAO1-2L.30S19 | HBB |
| ALB.SNRPB (SEQ ID NO: 39) | T7AG | ALB | SV40 | HAO1-2L.30S19 | SNRPBV1 |
| FGA.SNRPB (SEQ ID NO: 40) | T7AG | FGA | SV40 | HAO1-2L.30S19 | SNRPBV1 |
| GAPDH.SNRPB (SEQ ID NO: 42) | T7AG | GAPDH | SV40 | HAO1-2L.30S19 | SNRPBV1 |

### 2. Results

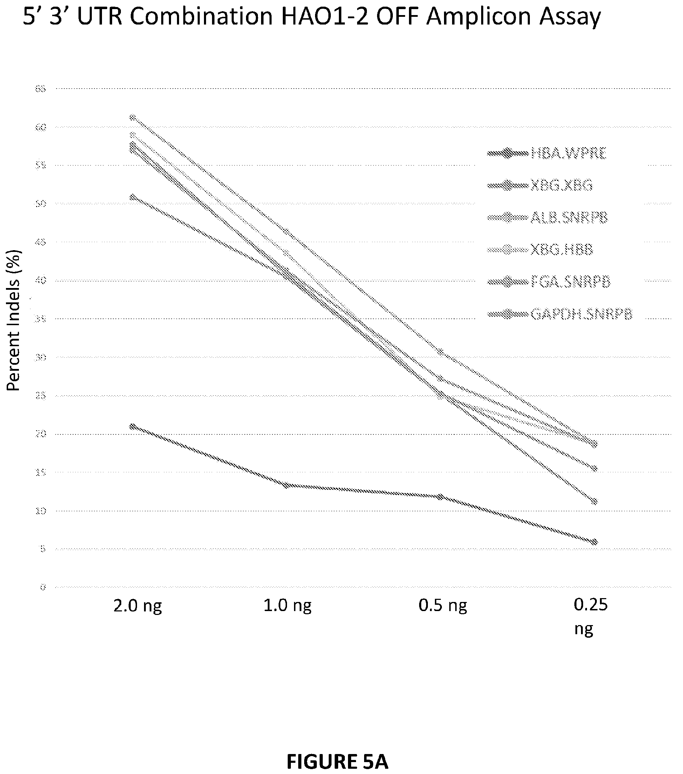

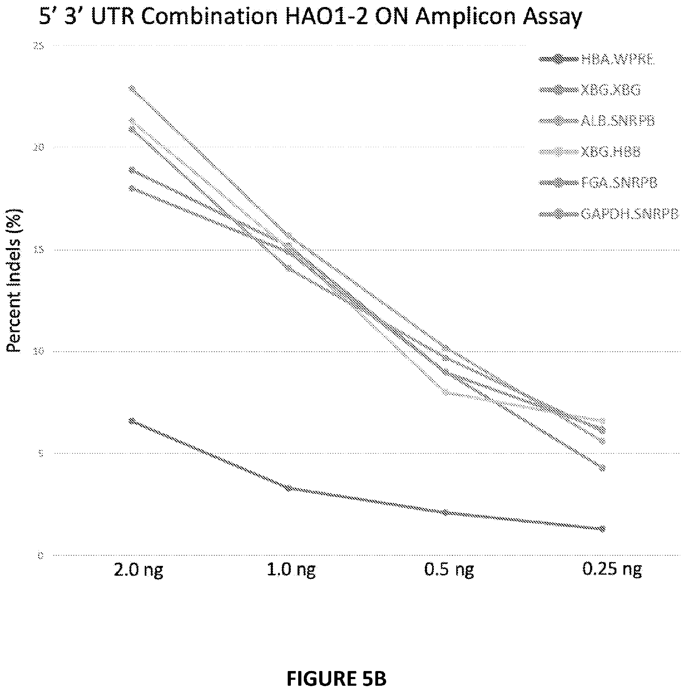

In these studies, indels (insertions and deletions) were measured by ddPCR in HEP3B cells at multiple low doses of mRNA using two biological replicates. Experimental data for HAO1-2 OFF amplicon assay is provided in Table 8 and shown in Figure 5A. Experimental data for HAO1-2 ON amplicon assay is provided in Table 9 and shown in Figure 5B.

**Table 8. Indel% by UTR combination for the HAO1-2 OFF ddPCR assay**

| **mRNA Dose** | **HBA.WPRE** | **XBG.XBG** | **ALB.SNRPB** | **XBG.HBB** | **FGA.SNRPB** | **GAPDH.SNRPB** |
|---|---|---|---|---|---|---|
| **2 ng** | 20.0 | 47.2 | 55.0 | 51.9 | 53.1 | 50.1 |
| **1 ng** | 10.5 | 37.1 | 43.6 | 41.8 | 38.7 | 35.1 |
| **0.5 ng** | 6.9 | 21.0 | 26.1 | 25.3 | 24.4 | 23.9 |
| **0.25 ng** | 3.5 | 9.9 | 17.4 | 16.5 | 12.7 | 16.5 |

**Table 9. Indel% by UTR combination for the HAO1-2 ON ddPCR assay**

| **mRNA Dose** | **HBA.WPRE** | **XBG.XBG** | **ALB.SNRPB** | **XBG.HBB** | **FGA.SNRPB** | **GAPDH.SNRPB** |
|---|---|---|---|---|---|---|
| **2 ng** | 5.4 | 15.9 | 19.1 | 18.2 | 15.7 | 17.1 |
| **1 ng** | 2.8 | 13.0 | 15.1 | 13.3 | 13.4 | 12.2 |
| **0.5 ng** | 1.7 | 7.2 | 8.9 | 7.4 | 7.6 | 7.5 |
| **0.25 ng** | 1.0 | 4.0 | 5.3 | 5.0 | 5.1 | 4.9 |

### 3. Conclusions

These studies demonstrate the ability of the mRNA containing the unique combinations of UTRs of the experiment encoding an HAO 1-2 meganuclease to generate indels at a greater percentage than the control (HBA.WPRE) across all dosages. This effect, is maintained down to 0.25 ng at low RNA doses with the ALB.SNRPB mRNA generating approximately 4 fold higher indels than the control mRNA.

### EXAMPLE 6

### Editing of HAO 25-26 recognition sequence in human cell lines using improved mRNA encoding the engineered HAO 25-26 meganucleases

### 1. Methods and Materials

These studies were conducted using in vitro cell-based systems to evaluate whether the improved mRNA designs increased the in vitro editing efficiencies of an engineered meganucleases designed to bind and cleave a target sequence within exon 2 of the HAO1 gene (i.e., the HAO 25-26 recognition sequence) by digital PCR using an indel detection assay. The engineered meganucleases used in this experiment were the HAO 25-26L.1128 and HAO 25-26L.1434 meganucleases that are encoded by the mRNA of SEQ ID NOs: 46-49.

These studies were conducted using in vitro cell-based systems to evaluate editing efficiencies of different HAO 25-26 meganucleases by digital PCR using an indel detection assay.

In these experiments, mRNA utilizing the combination of the 5' ALB UTR and 3' SNRPB V1 UTR with an additional C terminal NLS as a part of the engineered meganuclease were tested against standard mRNA that utilizes the 5' HBA2 UTR and 3' WPRE UTR. The nucleic acid coding sequence of the meganucleases in the improved mRNA were further modified using alternative codon sequences to reduce uridine content, while leaving the amino acid sequence identical. Each mRNA in the unmodified mRNA and improved mRNA contained N1-methylpseudouridine and a 7-methylguanosine cap. Each mRNA encoding the meganucleases were electroporated into HepG2 at a dosage of 0.1ng, 0.5ng, 2ng, 10ng, 50ng, and 100ng using the Lonza Amaxa 4D system.

The tested mRNA in this experiment are provided in Table 10.

**Table 10.**

| mRNA Name | Promoter | 5' UTR | N-term NLS | Meganuclease | C-term NLS | 3' UTR |
|---|---|---|---|---|---|---|
| HAO 25-26L.1434 (SEQ ID NO: 47) | T7AG | HBA2 | SV40 | HAO 25-26L.1434 | - | WPRE |
| HAO 25-26L.1434 MAX (SEQ ID NO: 49) | T7AG | XBG | NLS5 | HAO 25-26L.1434 | SV40 | XBG |
| HAO 25-26L.1128 (SEQ ID NO: 46) | T7AG | XBG | SV40 | HAO 25-26L.1128 | - | XBG |
| HAO 25-26L.1128 MAX (SEQ ID NO: 48) | T7AG | SNRPBV1 | SV40 | HAO 25-26L.1128 | SV40 | SNRPBV1 |

Cells were collected at seven days post electroporation for gDNA preparation and evaluated for transfection efficiency using a Beckman Coulter CytoFlex S cytometer. Transfection efficiency exceeded 90%. gDNA was prepared using the Macherey Nagel NucleoSpin Blood QuickPure kit.

Digital droplet PCR was utilized to determine the frequency of target insertions and deletions (indel%) using primers P1, F1, and R1 at the HAO 25-26 recognition sequence, as well as primers P2, F2, R2 to generate a reference amplicon. Amplifications were multiplexed in a 20uL reaction containing 1x ddPCR Supermix for Probes (no dUTP, BioRad), 250nM of each probe, 900nM of each primer, 5U of HindIII-HF, and about 50ng cellular gDNA. Droplets were generated using a QX100 droplet generator (BioRad). Cycling conditions for HAO 25-26 were as follows: 1 cycle of 95°C (2°C/s ramp) for 10 minutes, 44 cycles of 94°C (1°C/s ramp) for 30 seconds, 62°C (1°C/s ramp) for 30 seconds, 72C (0.2°C/s ramp) for 2 minutes, 1 cycle of 98°C for 10 minutes, 4°C hold. Cycling conditions for HAO 3-4 were as follows: 1 cycle of 95°C (2°C/s ramp) for 10 minutes, 44 cycles of 94°C (1°C/s ramp) for 30 seconds, 55°C (1°C/s ramp) for 30 seconds, 72C (0.2°C/s ramp) for 2 minutes, 1 cycle of 98°C for 10 minutes, 4°C hold.

Droplets were analyzed using a QX200 droplet reader (BioRad) and QuantaSoft analysis software (BioRad) was used to acquire and analyze data. Indel frequencies were calculated by dividing the number of positive copies for the binding site probe by the number of positive copies for the reference probe and comparing loss of FAM+ copies in nuclease-treated cells to mock-transfected cells.

### Primer Sets

P1: 34 HAO 25/26 P1 BS PROBE: TTGGATACAGCTTCCATCTA FAM (SEQ ID NO: 161)
F1: 21-HAO 25-25-15-16 F2: ACCAAACAAACAGTAAAATTGCC (SEQ ID NO: 162)
R1: 14-HAO15-16 25-26 R: GAGGTCGATAAACGTTAGCCTC (SEQ ID NO: 163)
P2: 44 12 REF PROBE1: TGTGGTCACCCTCTGCACAGTGT HEX (SEQ ID NO: 164)
F2: 28-HAO21-22 F2: CCACATAAGATTTGGCAAGCC (SEQ ID NO: 165)
R2: 27-HAO21-22 R2: TGTGGTCACCCTCTGCACAGTGT (SEQ ID NO: 166)

### 2. Results

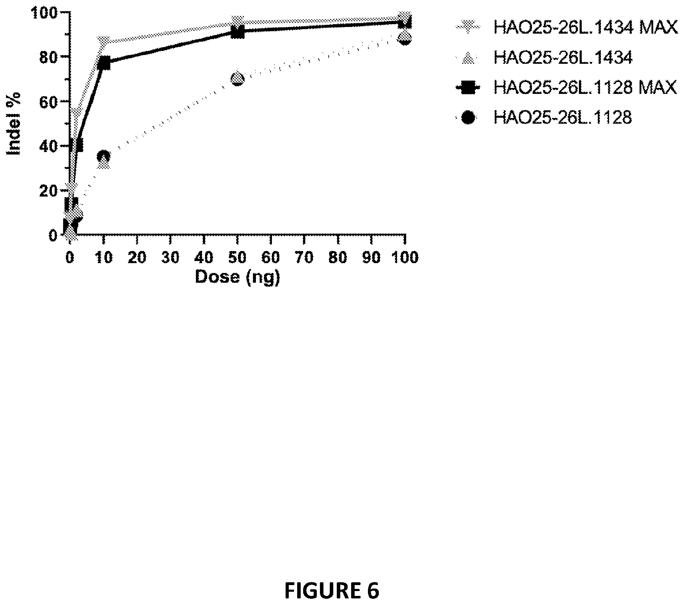

In these studies, indels (insertions and deletions) were measured by ddPCR across multiple dosages. The percentage of indels were greatly enhanced using the improved mRNA construct with alternative UTRs and uridine depletion. At a 10 ng dose, the HAO25-26L.1128 meganuclease generated about 35% indel formation, whereas the modified construct denoted as "MAX" generated about 77% indel formation (FIG. 6). Similarly, the HAO 25-26L.1434 meganuclease at a 10ng dose generated about 33% indel formation whereas the modified construct encoding the HAO 25-26L.1434 meganuclease denoted as "MAX" generated about 86% indels (FIG. 6). The trend of increased indel formation held across all dosages, but the difference between the two types of mRNA was decreased as the dose increased.

### 3. Conclusions

These studies demonstrate the ability of the HAO 25-26 meganucleases to generate indels at the HAO 25-26 recognition sequence in HepG2 cells. This experiment further shows that modification to mRNA encoding the meganucleases can have a profound effect on indel formation resulting in much greater indel formation at a lower mRNA dosage. This has the advantage of lowering the amount of mRNA needing to be delivered to a target cell as well as lowering potential immunogenicity to the mRNA.

### EXAMPLE 7

### The effect of different UTRs combinations on expression of an engineered meganuclease delivered by LNP in a mouse

### 1. Methods and Materials

In these studies, protein of an engineered meganuclease (referred to as TTR 15-16x.81) targeting a recognition sequence in the mouse TTR gene (referred to as the TTR 15-16 recognition sequence) was measured in mouse livers using antibodies specific for engineered meganucleases and a recombinant meganuclease protein standard in a sandwich ELISA on the MSD platform. The TTR 15-16x.81 meganuclease is described in the PCT international patent application WO2022/040528.

Mice were injected in the tail vein at a dose of 2mg mRNA/kg bodyweight with either PBS alone or PBS with LNPs containing TTR 15-16x.81 Max mRNA (which includes a 5' XBG UTR of SEQ ID NO: 7, a 3' XBG UTR of SEQ ID NO: 12, a c-myc NLS at the N-terminus and C-terminus, and the TTR 15-16x.81 coding sequence is codon optimized for uridine depletion) (SEQ ID NO: 188) or TTR 15-16x.81 Std mRNA that utilizes a standard control combination of an 5'HBA2 UTR, N-terminal SV40 sequence, and a 3' HBA2 UTR (SEQ ID NO: 189). At 3 hours post-injection, the mice were euthanized, and the median lobe of the liver was collected, and flash frozen on dry ice. ~40-90mg of each liver was weighed and homogenized in MSD Tris Lysis buffer containing complete Mini protease inhibitor using a SPEX MiniG 1600 Tissue homogenizer. Total protein concentration of each lysate was determined by BCA and lysates were diluted to 1mg/mL in MSD Diluent 100. One MULTI-ARRAY Standard 96-well plate from MSD was coated overnight at 4C with anti-meganuclease V34 antibody in PBS at a concentration of 4ug/mL. Standards were prepared using recombinant meganuclease protein diluted to concentrations from 0 - 10ug/mL in the 1mg/mL lysate from PBS alone-treated mice. The plate was blocked using 5% MSD Blocker A for 1h with shaking, washed 3 times using MSD Tris Wash Buffer, and then incubated with the lysates and standards for 90 minutes. The plate was washed 3 times again and incubated with sulfo-tagged anti-meganuclease M1 diluted to 1ug/mL in PBS for 1h with shaking. The plate was then washed, and MSD GOLD Read Buffer A was added to the wells. An MSD Quickplex SQ 120 instrument was used to read the plates and the data was analyzed using MSD Discovery Workbench software.

### EXAMPLE 8

### The effect of optimized mRNA on meganuclease activity in targeting the T Cell Receptor in vitro

### 1. Methods and Materials

This experiment was conducted to compare the efficiency of optimized mRNA formulations vs standard mRNA formulations encoding TRC1-2-specific meganucleases in primary human T cells delivered by electroporation. Meganucleases targeting the TRC1-2 recognition sequence in the T Cell Receptor Alpha Constant region (TRAC) are described in PCT international patent application WO2019/200122.In this pair of studies, an apheresis sample was drawn from a healthy, informed, and compensated donor, and the T cells were enriched using the CD3 positive selection kit II in accordance with the manufacturer's instructions (Stem Cell Technologies). T cells were activated using ImmunoCult T cell stimulator (anti-CD2/CD3/CD28 - Stem Cell Technologies) in Xuri medium (Cytiva) supplemented with 5% fetal bovine serum and 10ng/ml IL-2 (Gibco). After 3 days of stimulation, cells were collected and electroporated with standard mRNA formulation of the TRC1-2 L.2307 meganuclease that recognizes and cleave the TRC 1-2 site or a novel optimized formulation (MAX formulation). The standard formulation was delivered in 2-fold titrations from 3540ng per 1e6 cells down to 13.8ng per 1e6 cells. The MAX formulation was delivered in 2-fold titrations from 4000ng per 1e6 cells down to 62.5ng per 1e6 cells.

Following electroporation, cells were cultured in complete Xuri supplemented with 30ng/ml recombinant human IL-2 for 3-5 days with medium exchanges occurring every 2-3 days. Cells were counted after at least 3 days of culture, and stained for CD3 either by APC-conjugated anti-CD3 antibody (Biolegend) or FITC-conjugated anti-CD3 antibody (BioLegend). Data were acquired on a Beckman-Coulter CytoFLEX flow cytometer.

The DNA sequence of the constructs utilized in these experiments are provided in Table 11 below.

**Table 11 Description of mRNA used in Experiment of Example 8**

| mRNA Name | Promoter | 5' UTR | N-term NLS | Meganuclease | C-term NLS | 3' UTR |
|---|---|---|---|---|---|---|
| TRC1-2L.2307 Standard (SEQ ID NO: 171) | T7AG | HBA2 | SV40 | TRC1-2 L.2307 | - | WPRE |
| TRC1-2L.2307 MAX (SEQ ID NO: 172) | T7AG | ALB | SV40 | TRC1-2 L.2307 | SV40 | SNRPBV1 |

### 2. Results

Successful targeting of the TRAC gene at the TCR1-2 recognition site results in a loss of CD3 expression resulting in CD3 knock out (KO) cells. A table providing the knockout frequencies for the various experimental conditions is provided below.

**Table 12. Knockout of CD3 in primary human T Cells following administration of a standard or optimized mRNA formulations encoding a TRC1-2 targeting TRC1-2L.2307 meganuclease.**

| **L2307 Electroporations** | | | | |
|---|---|---|---|---|
| Sample | KO % | # of KO cells | Input | Fold Yield |
| L2307 3540ng | 82.49% | | | |
| L2307 1760ng | 82.2% | | | |
| L2307 884ng | 82.15% | | | |
| L2307 442ng | 78.455% | | | |
| L2307 221ng | 57.46% | | | |
| L2307 111ng | 27.165% | | | |
| L2307 55ng | 7.835% | | | |
| L2307 27.6ng | 8.09% | | | |
| L2307 13.8ng | 4.37% | | | |
| L2307 Mock | 4.19% | | | |
| L2307 Max 4000ng | 82.29% | 1.21e6 | 1e6 | 1.21 |
| L2307 Max 2000ng | 89.32% | 2.33e6 | 1e6 | 2.33 |
| L2307 Max 1000ng | 88.76% | 7.39r6 | 1e6 | 7.39 |
| L2307 Max 500ng | 88.52% | 1.12e7 | 1e6 | 11.2 |
| L2307 Max 250ng | 85.87% | 1.24e7 | 1e6 | 12.43 |
| L2307 Max 125ng | 78.61% | 1.11e7 | 1e6 | 11.07 |
| L2307 Max 62.5ng | 57.67% | 8.91e6 | 1e6 | 8.91 |
| L2307 Max Mock | 1.04% | NA | 1e6 | NA |

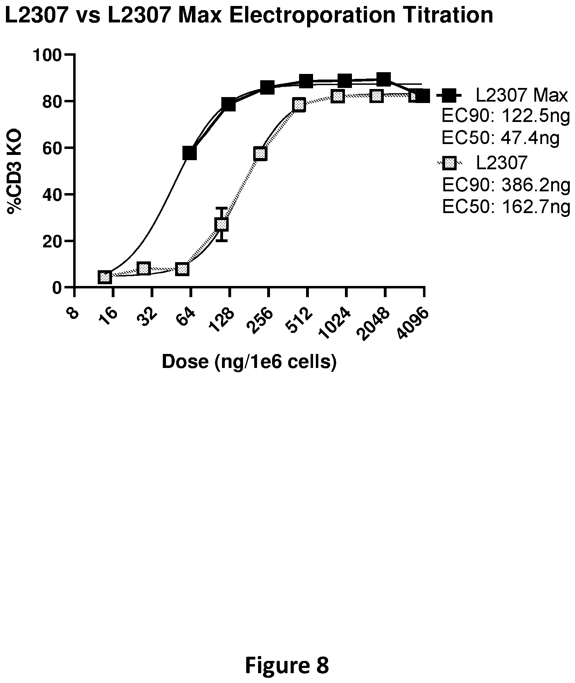

A dose response curve of CD3 knock out at various doses of the TRC1-2L.2307 meganuclease is provided in Figure 8 with EC90 and EC50 doses for each titration curve. In this dose response curve, the standard mRNA and the Max mRNA encoding the TRC 1-2L.2307 meganuclease was compared. These mRNAs were delivered in 2-fold doses by electroporation. As shown, the Max mRNA reduced the EC90 and EC50 dose of the TRC1-2 L.2307 meganuclease by at least half. For example, electroporation of 125ng of the Max mRNA encoding the TRC1-2 L.2307 meganuclease knocked out CD3 in 78.61% of T cells compared to the 78.45% generated by 442ng of standard mRNA encoding the TRC1-2 L.2307 meganuclease.

### 3. Conclusions

The results of this experiment demonstrate that the optimized Max mRNA encoding the TRC1-2L.2307 meganuclease outperformed a standard mRNA in a study where TRAC-edited T cell knockout-frequency was measured via CD3 knock out. These results demonstrate that the optimized Max mRNA encoding an engineered meganuclease performs in a superior fashion in a direct comparison to standard mRNA formulations consistent with the other examples described herein that utilized different targeting meganucleases.

### EXAMPLE 9

### Editing of HAO1-2 recognition sequence in human cell lines using improved mRNA encoding the engineered HOA1-2L.30 meganucleases

### 1. Methods and Materials

These studies were conducted using in vitro cell-based systems to evaluate whether the improved mRNA designs increased the in vitro editing efficiencies of an engineered meganuclease designed to bind and cleave a target sequence within exon 8 of the HAO1 gene (i.e., the HAO 1-2 recognition sequence) by digital PCR using an indel detection assay. The engineered meganucleases used in this experiment were the HAO 1-2L.30 S19 meganucleases that are encoded by the mRNA of SEQ ID NOs: 173-178. The HAO1-2L.30 meganucleases are described in PCT international patent application WO 2020/132659.

These studies were conducted using in vitro cell-based systems to evaluate editing efficiencies of different HAO 1-2 meganucleases by digital PCR using an indel detection assay.

In these experiments, mRNA utilizing combinations of 5' and 3' UTR's along with additional combinations of N and C terminal NLS as a part of the engineered meganuclease were tested against mRNA that utilizes the 5' HBA2 UTR and 3' WPRE UTR with a N terminal NLS. Each mRNA in the experiment contained N1-methylpseudouridine and a 7-methylguanosine cap. Each mRNA encoding the meganucleases were electroporated into Hep3B at a dosage of 2ng using the Lonza Amaxa 4D system.

The tested mRNA In this experiment are provided In Table 13.

**Table 13. mRNA Configurations for Experimental Treatments of Example 9**

| mRNA Name | Promoter | 5' UTR | N-term NLS | Meganuclease | C-term NLS | 3' UTR | Linear |
|---|---|---|---|---|---|---|---|
| 3503 HAO 1-2L.30S19 (SEQ ID NO: 173) | T7AG | XBG | SV40 | HAO 1-2L.30 S19 | SV40 | XBG | SspI |
| 3559 HAO 1-2L.30S19 (SEQ ID NO: 174) | T7AG | HBA2 | SV40 | HAO 1-2L.30 S19 | - | WPRE | BspQI |
| 35109 HAO 1-2L.30S19 (SEQ ID NO: 175) | T7AG | XBG | SV40 | HAO 1-2L.30 S19 | SV40 | XBG | BspQI |
| 35114 HAO 1-2L.30S19 (SEQ ID NO: 176) | T7AG | XBG | CMYC | HAO 1-2L.30 S19 | CMYC | XBG | BspQI |
| 35137 HAO 1-2L.30S19 (SEQ ID NO: 177) | T7AG | ALB | SV40 | HAO 1-2L.30 S19 | SV40 | SNRPBV1 | BspQI |
| 35138 HAO 1-2L.30S19 (SEQ ID NO: 178) | T7AG | REC-ALB | SV40 | HAO 1-2L.30 S19 | SV40 | SNRPBV1 | BspQI |

Cells were collected at 2, 6, and 9 days post electroporation for gDNA preparation and evaluated for transfection efficiency using a Beckman Coulter CytoFlex S cytometer. Transfection efficiency exceeded 90%. gDNA was prepared using the Macherey Nagel NucleoSpin Blood QuickPure kit.

Digital droplet PCR was utilized to determine the frequency of target insertions and deletions (indel%) using primers P1, F1, and R1 at the HAO 1-2 recognition sequence, as well as primers P2, F2, R2 to generate a reference amplicon. Amplifications were multiplexed in a 20uL reaction containing 1x ddPCR Supermix for Probes (no dUTP, BioRad), 250nM of each probe, 900nM of each primer, 5U of HindIII-HF, and about 50ng cellular gDNA. Droplets were generated using a QX100 droplet generator (BioRad). Cycling conditions for HAO 1-2 were as follows: 1 cycle of 95°C (2°C/s ramp) for 10 minutes, 44 cycles of 95°C (1°C/s ramp) for 30 seconds, 62°C (1°C/s ramp) for 30 seconds, 72C (0.2°C/s ramp) for 2 minutes, 1 cycle of 98°C for 10 minutes, 4°C hold. Cycling conditions for HAO 23-24 were as follows: 1 cycle of 95°C (2°C/s ramp) for 10 minutes, 44 cycles of 95°C (1°C/s ramp) for 30 seconds, 62°C (1°C/s ramp) for 30 seconds, 72C (0.2°C/s ramp) for 2 minutes, 1 cycle of 98°C for 10 minutes, 4°C hold.

Droplets were analyzed using a QX200 droplet reader (BioRad) and QuantaSoft analysis software (BioRad) was used to acquire and analyze data. Indel frequencies were calculated by dividing the number of positive copies for the binding site probe by the number of positive copies for the reference probe and comparing loss of FAM+ copies in nuclease-treated cells to mock-transfected cells.

### Primer Sets

P1: 42 HAO1 2 BHQ 1 PROBE: TTCCTCACCAATGTCTTGT FAM (SEQ ID NO: 150)
F1: 28-HAO21-22 F2: CCACATAAGATTTGGCAAGCC (SEQ ID NO: 151)
R1: 27-HAO21-22 R2: GGAAAAGAACGACACCCTTTG (SEQ ID NO: 152)
P2: 33 HAO23/24 P1: CCCGGCTAATTTGTATCA VIC (SEQ ID NO: 153)
F2: 29-HAO23-24 f1: GCTCACTTGATGTAAGCAACAG (SEQ ID NO: 154)
R2: 32-HAO23-24 R2: ACACACCACCAACGTAAAAC (SEQ ID NO: 155)

### 2. Results

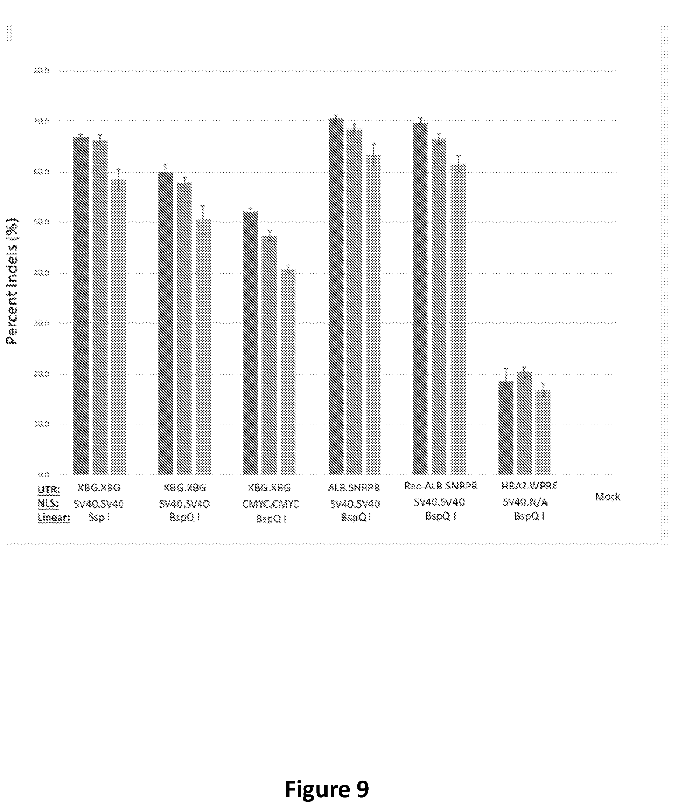

In these studies, indels (insertions and deletions) were measured by ddPCR at 2ng per 0.5e6 Hep3B cells. The percentage of indels were greatly enhanced using the improved mRNA construct with alternative UTRs and dual SV40 NLS. At a 2 ng dose, the HAO1-2L.30 control meganuclease generated about 17% indel formation on day 9, whereas the best performing modified construct denoted as 35137 HAO 1-2L.30 generated about 63% indel formation on day 9 (FIG. 9). Similarly, the 35138 HAO 1-2L.30 meganuclease at a 2 ng dose generated about 62% indel formation on day 9, whereas the modified construct encoding the 35114 HAO 1-2L.30 meganuclease generated about 41% indels (FIG. 9).

### 3. Conclusions

These studies demonstrate the ability of the HAO 1-2 meganucleases to generate indels at the HAO 1-2 recognition sequence in Hep3B cells. This experiment further shows that modification to mRNA encoding the meganucleases can have a profound effect on indel formation resulting in much greater indel formation at a lower mRNA dosage (2ng). This has the advantage of lowering the amount of mRNA needing to be delivered to a target cell as well as lowering potential immunogenicity to the mRNA. Additionally, these studies demonstrate a hierarchy of UTR and NLS combinations for indel generation. The 35137 HAO 1-2L.30 meganuclease modifications generate 63% indels and result in an increase in meganuclease expression over the 35114 construct encoding the same HAO 1-2L.30 meganuclease, which demonstrated 41% indels. Thus, the trend of increased indel formation over control held across all other modifications, but differences between the two types of UTR's and NLS tested was noted. These differences allow for a tunability of meganuclease expression based on mRNA.

### EXAMPLE 10

### The effect of different UTRs combinations on expression of an engineered meganuclease delivered by LNP in a mouse

### 1. Methods and Materials

In these studies, protein levels of engineered meganucleases described in Table 14 were measured in mouse livers using antibodies specific for engineered meganucleases and an engineered meganuclease protein standard in a sandwich ELISA on the MSD platform.

Mice were injected in the tail vein at a dose of 2mg mRNA/kg bodyweight with either PBS alone or PBS with LNPs containing an optimized (Max) or standard mRNA. A complete description of the constructs coding the respective meganucleases is provided in Table 14. The HBV 11-12 L.1090 meganucleases are described in PCT international patent application WO2021/113765. The coding sequences of the Max mRNAs were codon optimized for uridine depletion. These Max constructs include a 5'XBG UTR of SEQ ID NO. 7, a 3'XBG UTR of SEQID NO: 12, and a cMYC NLS at the N and C terminus. At 3 hours post-injection, the mice were euthanized, and the median lobe of the liver was collected, and flash frozen on dry ice. ~40-90mg of each liver was weighed and homogenized in MSD Tris Lysis buffer containing complete Mini protease inhibitor using a SPEX MiniG 1600 Tissue homogenizer. Total protein concentration of each lysate was determined by BCA and lysates were diluted to 1mg/mL in MSD Diluent 100. One MULTI-ARRAY Standard 96-well plate from MSD was coated overnight at 4C with anti-meganuclease V34 antibody in PBS at a concentration of 4ug/mL. Standards were prepared using standard engineered meganuclease protein diluted to concentrations from 0 - 10ug/mL in the 1mg/mL lysate from PBS alone-treated mice. The plate was blocked using 5% MSD Blocker A for 1h with shaking, washed 3 times using MSD Tris Wash Buffer, and then incubated with the lysates and standards for 90 minutes. The plate was washed 3 times again and incubated with sulfo-tagged anti-meganuclease M1 diluted to 1ug/mL in PBS for 1h with shaking. The plate was then washed, and MSD GOLD Read Buffer A was added to the wells. An MSD Quickplex SQ 120 instrument was used to read the plates and the data was analyzed using MSD Discovery Workbench software.

**Table 14. mRNA Construct Design for the Studies of Example 10**

| mRNA Name | Promoter | 5' UTR | N-term NLS | Meganuclease | C-term NLS | 3' UTR |
|---|---|---|---|---|---|---|
| HAO 1-2 L.30S19 MAX (SEQ ID NO: 179) | T7AG | XBG | Cmyc | HAO 1-2L.30S 19 | Cmyc | XBG |
| HBV11-12 L.1090 MAX (SEQ ID NO: 180) | T7AG | XBG | Cmyc | HBV11-12 L.1090 | Cmyc | XBG |
| HAO 1-2 L.30S19 STD (SEQ ID NO: 181) | T7AG | HBA2 | SV40 | HAO 1-2L.30S 19 | | WPRE |
| HBV11-12 L.1090 STD (SEQ ID NO: 182) | T7AG | HBA2 | SV40 | HBV11-12 L.1090 | | WPRE |

### 2. Results

Livers from mice injected with a standard mRNA encoding the HAO1-2 L.30S19 meganuclease showed protein expression ranging from 0.64-0.99µg/g tissue after collection 3h post-injection, while livers from mice injected with an optimized Max mRNA showed protein expression ranging from 0.99-1.61µg/g tissue. Similarly, livers from mice injected with the HBV11-12 1090 Std mRNA showed protein expression ranging from 0.15-0.48 µg/g tissue after collection 3h post-injection, while livers from mice injected with Max mRNA showed protein expression ranging from 0.5-1.3 µg/g tissue.

### 3. Conclusions

This experiment demonstrated the ability of LNP delivered mRNA encoding various engineered meganucleases to produce meganuclease protein in-vivo. Furthermore for the HAO1-2 L.30S19 and HBV11-12 L.1090 nucleases, mRNA containing XBG/XBG UTRs, a Cmyc NLS, and a uridine depleted sequence produced more protein than a standard control mRNA containing HBA2/WPRE UTRs, an SV40 NLS, and a non-uridine depleted sequence.

### EXAMPLE 11

### The effect of different UTRs combinations on expression of an engineered meganuclease delivered by LNP in a mouse

### 1. Methods and Materials

In these studies, protein of engineered meganucleases were measured in mouse livers using antibodies specific for engineered meganucleases and an engineered meganuclease protein standard in a sandwich ELISA on the MSD platform.

Mice were injected in the tail vein at a dose of 0.3 mg mRNA/kg bodyweight with either PBS alone or PBS with LNPs containing optimized Max or Std mRNAs encoding the respective meganucleases. A complete description of the constructs is displayed in Table 15. The HAO 25-26 meganucleases are described in PCT international patent application WO2022/150616 and the TTR 15-16x.81 meganuclease is described in PCT international patent application WO2022/040582. Each of the coding sequences of Max mRNAs were codon optimized for uridine depletion. At 3 hours post-injection, the mice were euthanized, and the median lobe of the liver was collected, and flash frozen on dry ice. ~40-90mg of each liver was weighed and homogenized in MSD Tris Lysis buffer containing complete Mini protease inhibitor using a SPEX MiniG 1600 Tissue homogenizer. Total protein concentration of each lysate was determined by BCA and lysates were diluted to 1mg/mL in MSD Diluent 100. One MULTI-ARRAY Standard 96-well plate from MSD was coated overnight at 4C with anti-meganuclease V34 antibody in PBS at a concentration of 4ug/mL. Standards were prepared using standard engineered meganuclease protein diluted to concentrations from 0 - 10ug/mL in the 1mg/mL lysate from PBS alone-treated mice. The plate was blocked using 5% MSD Blocker A for 1h with shaking, washed 3 times using MSD Tris Wash Buffer, and then incubated with the lysates and standards for 90 minutes. The plate was washed 3 times again and incubated with sulfo-tagged anti-meganuclease M1 diluted to 1ug/mL in PBS for 1h with shaking. The plate was then washed, and MSD GOLD Read Buffer A was added to the wells. An MSD Quickplex SQ 120 instrument was used to read the plates and the data was analyzed using MSD Discovery Workbench software.

**Table 15. mRNA Construct Design for the Studies of Example 11**

| mRNA Name | Promoter | 5' UTR | N-term NLS | Meganuclease | C-term NLS | 3' UTR |
|---|---|---|---|---|---|---|
| HAO25-26L.1128 STD (SEQ ID NO: 183) | T7AG | HBA2 | SV40 | HAO25-26L.1128 | | WPRE |
| HAO25-26L.1128 Max (SEQ ID NO: 184) | T7AG | ALB | SV40 | HAO25-26L.1128 | SV40 | SNRPB |
| HAO25-26L.1434 STD (SEQ ID NO: 185) | T7AG | HBA2 | SV40 | HAO25-26L.1434 | | WPRE |
| HAO25-26L.1434 Max (SEQ ID NO: 186) | T7AG | ALB | SV40 | HAO25-26L.1434 | SV40 | SNRPB |
| HAO25-26x.227 (SEQ ID NO: 187) | T7AG | HBA2 | SV40 | HAO25-26X.227 | | WPRE |
| HAO1-2 L.30S19 Max (SEQ ID NO: 188) | T7AG | XBG | Cmyc | HAO1-2 | Cmyc | XBG |
| TTR15-16X.81 Max (SEQ ID NO: 189) | T7AG | XBG | Cmyc | TTR15-16X.81 | Cmyc | XBG |

### 2. Results

Livers from mice injected with HAO 25-26L.1128 STD mRNA showed meganuclease protein expression ranging from 0.31-0.37 ng/mg total protein after collection 3h post-injection, while livers from mice injected with HAO 25-26L.1128 Max mRNA showed meganuclease protein expression ranging from 0.94-1.5 ng/mg of total protein. Similarly, livers from mice injected with HAO 25-26L.1434 STD mRNA showed meganuclease protein expression ranging between 0.5-0.6 ng/mg total protein while livers from mice injected with HAO 25-26L.1434 Max mRNA showed meganuclease protein expression between 0.7-1.2 ng/mg of total protein.

### 3. Conclusions

This experiment demonstrated the ability of LNP delivered mRNA encoding engineered meganucleases to produce meganuclease protein in-vivo. Furthermore for HAO25-26L.1128 and HAO25-26L.1434 meganucleases, mRNA containing ALB/SNRPB UTRs, SV40 NLS, and a uridine depleted sequence produced more protein than a standard control mRNA containing HBA2/WPRE UTRs, an SV40 NLS, and a non-uridine depleted sequence.

### Sequence Listing

SEQ ID NO: 1 
AATTATTGGTTAAAGAAGTATATTAGTGCTAATTTCCCTCCGTTTGTCCTAGCTTT
TCTCTTCTGTCAACCCCACACGCCTTTGCCACC
SEQ ID NO: 2 
AGGTTGGGAACTAGGAGTGGCAGCAATCCTTTCTTTCAGCTGGAGTGCTCCTCAG
GAGCCAGCCCCACCCTTAGCCACC
SEQ ID NO: 3 
ATAAGAGACCACAAGCGACCCGCAGGGCCAGACGTTCTTCGCCGAGAGTCGTCG
GGGTTTCCTGCTTCAACAGTGCTTGGACGGAACCCGGCGCTCGTTCCCCACCCCG
GCCGGCCGCCCATAGCCAGCCCTCCGTCACCTCTTCACCGCACCCTCGGACTGCC
CCAAGGCCCCCGCCGCCGCTCCAGCGCCGCGCAGCCACCGCCGCCGCCGCCGCC
TGCCACC
SEQ ID NO: 4 
GCTCTCTGCTCCTCCTGTTCGACAGTCAGCCGCATCTTCTTTGCGTCGCCAGCCG
AGCCACATCGCTCAGCCACC
SEQ ID NO: 5 
CATAAACCCTGGCGCGCTCGCGGCCGGCACTCTTCTGGTCCCACAGACTCAGA
GAGAAGCCA
SEQ ID NO: 6 
GCATTTCCGGTAGCGGCGGCGGAAATCGGCTGTGGAGAGAGGCTAGGCCTCT
GAGGAGGCGAATCCGGCGGGTATCAGAGCCATCAGAACCGCCAC
SEQ ID NO: 7 AAGCTCAGAATAAACGCTCAACTTTGGCC
SEQ ID NO: 8 
GCTGGAGCCTCGGTGGCCATGCTTCTTGCCCCTTGGGCCTCCCCCCAGCCCCTCCT
CCCCTTCCTGCACCCGTACCCCCGTGGTCTTTGAATAAAGTCTGAGTGGGCGGCA
G
SEQ ID NO: 9 
GCTCGCTTTCTTGCTGTCCAATTTCTATTAAAGGTTCCTTTGTTCCCTAAGTCCAA
CTACTAAACTGGGGGATATTATGAAGGGCCTTGAGCATCTGGATTCTGCCTAATA
AAAAACATTTATTTTCATTGC
SEQ ID NO: 10 
ACTCATCTTGGCCCTCCTCAGCTCCCTGCCTGTTTCCCGTAAGGCTGTACATAGTC
CTTTTATCTCCTTGTGGCCTATGAAACTGGTTTATAATAAACTCTTAAGAGAACAT
TA
SEQ ID NO: 11 
CCCTTGGCCACAGAGTATGGAAGTAGCTCCGCAGAGGCGTGGCTCGATTCCTC
AGGGCCACGTTACCACAGACCTGTTTGTTTCTTATGCTGTTGTTCGTGGAGTCTCA
TGGGATTGTCTGGTTTCCCTTACAGGGCCCCCTCCCCCGGGAATGCGCCCACCAA
GGCCCTAGACTCATCTTGGCCCTCCTCAGCTCCCTGCCTGTTTCCCGTAAGGCTGT
ACATAGTCCTTTTATCTCCTTGTGGCCTATGAAACTGGTTTATAATAAACTCTTAA
GAGAACATTA
SEQ ID NO: 12 
ACCAGCCTCAAGAACACCCGAATGGAGTCTCTAAGCTACATAATACCAACTTAC
ACTTTACAAAATGTTGTCCCCCAAAATGTAGCCATTCGTATCTGCTCCTAATAAA
AAGAAAGTTTCTTCACATTCT
SEQ ID NO: 13 
ATCAACCTCTGGATTACAAAATTTGTGAAAGATTGACTGATATTCTTAACTATGT
TGCTCCTTTTACGCTGTGTGGATATGCTGCTTTAATGCCTCTGTATCATGCTATTG
CTTCCCGTACGGCTTTCGTTTTCTCCTCCTTGTATAAATCCTGGTTGCTGTCTCTTT
ATGAGGAGTTGTGGCCCGTTGTCCGTCAACGTGGCGTGGTGTGCTCTGTGTTTGC
TGACGCAACCCCCACTGGCTGGGGCATTGCCACCACCTGTCAACTCCTTTCTGGG
ACTTTCGCTTTCCCCCTCCCGATCGCCACGGCAGAACTCATCGCCGCCTGCCTTGC
CCGCTGCTGGACAGGGGCTAGGTTGCTGGGCACTGATAATTCCGTGGTGTTGTCG
GGGAAGCTGACGTCCTTTCCAGGGCTGCTCGCCTGTGTTGCCAACTGGATCCTGC
GCGGGACGTCCTTCTGCTACGTCCCTTCGGCTCTCAATCCAGCGGACCTCCCTTCC
CGAGGCCTTCTGCCGGTTCTGCGGCCTCTCCCGCGTCTTCGCTTTCGGCCTCCGAC
GAGTCGGATCTCCCTTTGGGCCGCCTCCCCGCCTG
SEQ ID NO: 14 
GACTCACTATTTGTTTTCGCGCCCAGTTGCAAAAGTGTCGCCGCATCTAGAGGG
CC
SEQ ID NO: 15
   PKKKRKV
SEQ ID NO: 16
   RAAKRPRTT
SEQ ID NO: 17
   PAAKRVKLD
SEQ ID NO: 18
   HHPKKKRKV
SEQ ID NO: 19
   CCCAAGAAGAAGCGCAAGGTG
SEQ ID NO: 20
   CGGGCCGCCAAGCGGCCACGGACCACC
SEQ ID NO: 21 
TAATACGACTCACTATAAGGGACTCACTATTTGTTTTCGCGCCCAGTTGCAAAA
AGTGTCGCCGCATCTAGAGGGCCCATAAACCCTGGCGCGCTCGCGGCCGGCAC
TCTTCTGGTCCCCACAGACTCAGAGAGAACCCACCATGGCCCCCAAGAAGAAGC
GCAAGGTGCACATGAACACCAAGTACAACAAGGAGTTCCTGCTGTACCTGGCCG
GCTTCGTGGACAGCGACGGCAGCATCTACGCCGCCATCCGCCCCAGCCAGACCG
CCAAGTTCAAGCACCGCCTGCAGCTGTTCTTCGCCGTGTACCAGAAGACCCAGCG
CCGCTGGTTCCTGGACAAGCTGGTGGACGAGATCGGCGTGGGCTACGTGACCGA
CGCCGGCAGCGTGAGCAGCTACTTCCTGAGCGAGATCAAGCCCCTGCACAACTT
CCTGACCCAGCTGCAGCCCTTCCTGAAGCTGAAGCAGAAGCAGGCCAACCTGGT
GCTGAAGATCATCGAGCAGCTGCCCAGCGCCAAGGAGAGCCCCGACAAGTTCCT
GGAGGTGTGCACCTGGGTGGACCAGATCGCCGCCCTGAACGACAGCAAGACCCG
CAAGACCACCAGCGAGACCGTGCGCGCCGTGCTGGACAGCCTGCCCGGCAGCGT
GGGCGGCCTGAGCCCCAGCCAGGCCAGCAGCGCCGCCAGCAGCGCCAGCAGCA
GCCCCGGCAGCGGCATCAGCGAGGCCCTGCGCGCCGGCGCCGGCAGCGGCACCG
GCTACAACAAGGAGTTCCTGCTGTACCTGGCCGGCTTCGTGGACGGCGACGGCA
GCATCTTCGCCAGCATCCACCCCCAGCAGCGCAACAAGTTCAAGCACCAGCTGA
GCCTGCACTTCACCGTGCGCCAGAAGACCCAGCGCCGCTGGTTCCTGGACAAGCT
GGTGGACGAGATCGGCGTGGGCTACGTGATCGACGAGGGCAGCGTGAGCAGCTA
CCGCCTGAGCAAGATCAAGCCCCTGCACAACTTCCTGACCCAGCTGCAGCCCTTC
CTGAAGCTGAAGCAGAAGCAGGCCAACCTGGTGCTGAAGATCATCGAGCAGCTG
CCCAGCGCCAAGGAGAGCCCCGACAAGTTCCTGGAGGTGTGCACCTGGGTGGAC
CAGATCGCCGCCCTGAACGACAGCAAGACCCGCAAGACCACCAGCGAGACCGTG
CGCGCCGTGCTGGACAGCCTGAGCGAGAAGAAGAAGTCCAGCCCCTAATGAGGT
ACCAGCGGCCGCATCAACCTCTGGATTACAAAATTTGTGAAAGATTGACTGATAT
TCTTAACTATGTTGCTCCTTTTACGCTGTGTGGATATGCTGCTTTAATGCCTCTGT
ATCATGCTATTGCTTCCCGTACGGCTTTCGTTTTCTCCTCCTTGTATAAATCCTGG
TTGCTGTCTCTTTATGAGGAGTTGTGGCCCGTTGTCCGTCAACGTGGCGTGGTGTG
CTCTGTGTTTGCTGACGCAACCCCCACTGGCTGGGGCATTGCCACCACCTGTCAA
CTCCTTTCTGGGACTTTCGCTTTCCCCCTCCCGATCGCCACGGCAGAACTCATCGC
CGCCTGCCTTGCCCGCTGCTGGACAGGGGCTAGGTTGCTGGGCACTGATAATTCC
GTGGTGTTGTCGGGGAAGCTGACGTCCTTTCCAGGGCTGCTCGCCTGTGTTGCCA
ACTGGATCCTGCGCGGGACGTCCTTCTGCTACGTCCCTTCGGCTCTCAATCCAGC
GGACCTCCCTTCCCGAGGCCTTCTGCCGGTTCTGCGGCCTCTCCCGCGTCTTCGCT
TTCGGCCTCCGACGAGTCGGATCTCCCTTTGGGCCGCCTCCCCGCCTGGGCGCGC
CAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAA
SEQ ID NO: 22 
TAATACGACTCACTATAAGGGGACTCACTATTTGTTTTCGCGCCCAGTTGCAAAA
AGTGTCGCCGCATCTAGAGGGCCCCATGGCCCCCAAGAAGAAGCGCAAGGTGCA
CATGAACACCAAGTACAACAAGGAGTTCCTGCTGTACCTGGCCGGCTTCGTGGA
CAGCGACGGCAGCATCTACGCCGCCATCCGCCCCAGCCAGACCGCCAAGTTCAA
GCACCGCCTGCAGCTGTTCTTCGCCGTGTACCAGAAGACCCAGCGCCGCTGGTTC
CTGGACAAGCTGGTGGACGAGATCGGCGTGGGCTACGTGACCGACGCCGGCAGC
GTGAGCAGCTACTTCCTGAGCGAGATCAAGCCCCTGCACAACTTCCTGACCCAGC
TGCAGCCCTTCCTGAAGCTGAAGCAGAAGCAGGCCAACCTGGTGCTGAAGATCA
TCGAGCAGCTGCCCAGCGCCAAGGAGAGCCCCGACAAGTTCCTGGAGGTGTGCA
CCTGGGTGGACCAGATCGCCGCCCTGAACGACAGCAAGACCCGCAAGACCACCA
GCGAGACCGTGCGCGCCGTGCTGGACAGCCTGCCCGGCAGCGTGGGCGGCCTGA
 
GCCCCAGCCAGGCCAGCAGCGCCGCCAGCAGCGCCAGCAGCAGCCCCGGCAGCG
GCATCAGCGAGGCCCTGCGCGCCGGCGCCGGCAGCGGCACCGGCTACAACAAGG
AGTTCCTGCTGTACCTGGCCGGCTTCGTGGACGGCGACGGCAGCATCTTCGCCAG
CATCCACCCCCAGCAGCGCAACAAGTTCAAGCACCAGCTGAGCCTGCACTTCAC
CGTGCGCCAGAAGACCCAGCGCCGCTGGTTCCTGGACAAGCTGGTGGACGAGAT
CGGCGTGGGCTACGTGATCGACGAGGGCAGCGTGAGCAGCTACCGCCTGAGCAA
GATCAAGCCCCTGCACAACTTCCTGACCCAGCTGCAGCCCTTCCTGAAGCTGAAG
CAGAAGCAGGCCAACCTGGTGCTGAAGATCATCGAGCAGCTGCCCAGCGCCAAG
GAGAGCCCCGACAAGTTCCTGGAGGTGTGCACCTGGGTGGACCAGATCGCCGCC
CTGAACGACAGCAAGACCCGCAAGACCACCAGCGAGACCGTGCGCGCCGTGCTG
GACAGCCTGAGCGAGAAGAAGAAGTCCAGCCCCTAATGAGGTACCAGCGGCCGC
ATCAACCTCTGGATTACAAAATTTGTGAAAGATTGACTGATATTCTTAACTATGT
TGCTCCTTTTACGCTGTGTGGATATGCTGCTTTAATGCCTCTGTATCATGCTATTG
CTTCCCGTACGGCTTTCGTTTTCTCCTCCTTGTATAAATCCTGGTTGCTGTCTCTTT
ATGAGGAGTTGTGGCCCGTTGTCCGTCAACGTGGCGTGGTGTGCTCTGTGTTTGC
TGACGCAACCCCCACTGGCTGGGGCATTGCCACCACCTGTCAACTCCTTTCTGGG
ACTTTCGCTTTCCCCCTCCCGATCGCCACGGCAGAACTCATCGCCGCCTGCCTTGC
CCGCTGCTGGACAGGGGCTAGGTTGCTGGGCACTGATAATTCCGTGGTGTTGTCG
GGGAAGCTGACGTCCTTTCCAGGGCTGCTCGCCTGTGTTGCCAACTGGATCCTGC
GCGGGACGTCCTTCTGCTACGTCCCTTCGGCTCTCAATCCAGCGGACCTCCCTTCC
CGAGGCCTTCTGCCGGTTCTGCGGCCTCTCCCGCGTCTTCGCTTTCGGCCTCCGAC
GAGTCGGATCTCCCTTTGGGCCGCCTCCCCGCCTGGGCGCGCCAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAA
SEQ ID NO: 23 
TAATACGACTCACTATAAGGGAAGCTCAGAATAAACGCTCAACTTTGGCCACCA
TGGCACGGGCCGCCAAGCGGCCACGGACCACCCATATGAACACCAAGTACAACA
AGGAGTTCCTGCTCTACCTGGCGGGCTTCGTCGACTCCGACGGCTCCATCTACGC
CGCCATCCGCCCGAGCCAGACCGCCAAGTTCAAGCATCGGCTGCAGCTGTTCTTC
GCCGTCTACCAGAAGACACAGCGCCGTTGGTTCCTCGACAAGCTGGTGGACGAG
ATCGGGGTGGGCTACGTGACCGACGCCGGCAGCGTCAGCAGCTACTTCCTGTCC
GAGATCAAGCCTCTGCACAACTTCCTGACCCAGCTCCAGCCCTTCCTGAAGCTCA
AGCAGAAGCAGGCCAACCTCGTGCTGAAGATCATCGAGCAGCTGCCCTCCGCCA
AGGAATCCCCGGACAAGTTCCTGGAGGTGTGCACGTGGGTCGACCAGATCGCGG
CCCTCAACGACAGCAAGACCCGCAAGACGACCTCGGAAACGGTGCGGGCGGTCC
TGGACTCCCTCCCAGGATCCGTGGGAGGTCTATCGCCATCTCAGGCATCCAGCGC
CGCATCCTCGGCTTCCTCAAGCCCGGGTTCAGGGATCTCCGAAGCACTCAGAGCT
GGAGCAGGTTCCGGCACTGGATACAACAAGGAATTCCTGCTCTACCTGGCGGGC
TTCGTCGACGGGGACGGCTCCATCTTCGCCTCCATCCACCCGCAGCAGCGCAACA
AGTTCAAGCATCAGCTGAGCCTCCACTTCACCGTCAGGCAGAAGACACAGCGCC
GTTGGTTCCTCGACAAGCTGGTGGACGAGATCGGGGTGGGCTACGTGATCGACG
AGGGCAGCGTCAGCAGCTACCGCCTGTCCAAGATCAAGCCTCTGCACAACTTCCT
GACCCAGCTCCAGCCCTTCCTGAAGCTCAAGCAGAAGCAGGCCAACCTCGTGCT
GAAGATCATCGAGCAGCTGCCCTCCGCCAAGGAATCCCCGGACAAGTTCCTGGA
GGTGTGCACCTGGGTGGACCAGATCGCCGCTCTGAACGACTCCAAGACCCGCAA
GACCACTTCCGAAACCGTCCGCGCCGTTCTAGACAGTCTCTCCGAGAAGAAGAA
GTCGTCCCCCGACTACAAGGACGACGACGACAAGTAATGAGGTACCAGCGGCCG
CACCAGCCTCAAGAACACCCGAATGGAGTCTCTAAGCTACATAATACCAACTTA
 
CACTTTACAAAATGTTGTCCCCCAAAATGTAGCCATTCGTATCTGCTCCTAATAA
AAAGAAAGTTTCTTCACATTCTGGCGCGCCAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAA
SEQ ID NO: 24 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCACCAG
CCTCAAGAACACCCGAATGGAGTCTCTAAGCTACATAATACCAACTTACACTTTA
CAAAATGTTGTCCCCCAAAATGTAGCCATTCGTATCTGCTCCTAATAAAAAGAAA
GTTTCTTCACATTCTGGCGCGCCAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAA
SEQ ID NO: 25 
TAATACGACTCACTATAAGGGGCATTTCCGGTAGCGGCGGGAAATCGGCTG
TGGGAGAGAGGCTAGGCCTCTGAGGAGGCGAATCCGGCGGGTATCAGAGCCATC
AGAACCGCCACCATGGCACCGAAGAAGAAGCGCAAGGTGCATATGAACACCAA
GTACAACAAGGAGTTCCTGCTCTACCTGGCGGGCTTCGTCGACTCCGACGGCTCC
ATCTACGCCGCCATCCGCCCGAGCCAGACCGCCAAGTTCAAGCATCGGCTGCAG
CTGTTCTTCGCCGTCTACCAGAAGACACAGCGCCGTTGGTTCCTCGACAAGCTGG
TGGACGAGATCGGGGTGGGCTACGTGACCGACGCCGGCAGCGTCAGCAGCTACT
TCCTGTCCGAGATCAAGCCTCTGCACAACTTCCTGACCCAGCTCCAGCCCTTCCT
GAAGCTCAAGCAGAAGCAGGCCAACCTCGTGCTGAAGATCATCGAGCAGCTGCC
CTCCGCCAAGGAATCCCCGGACAAGTTCCTGGAGGTGTGCACGTGGGTCGACCA
GATCGCGGCCCTCAACGACAGCAAGACCCGCAAGACGACCTCGGAAACGGTGCG
GGCGGTCCTGGACTCCCTCCCAGGATCCGTGGGAGGTCTATCGCCATCTCAGGCA
TCCAGCGCCGCATCCTCGGCTTCCTCAAGCCCGGGTTCAGGGATCTCCGAAGCAC
 
TCAGAGCTGGAGCAGGTTCCGGCACTGGATACAACAAGGAATTCCTGCTCTACCT
GGCGGGCTTCGTCGACGGGGACGGCTCCATCTTCGCCTCCATCCACCCGCAGCAG
CGCAACAAGTTCAAGCATCAGCTGAGCCTCCACTTCACCGTCAGGCAGAAGACA
CAGCGCCGTTGGTTCCTCGACAAGCTGGTGGACGAGATCGGGGTGGGCTACGTG
ATCGACGAGGGCAGCGTCAGCAGCTACCGCCTGTCCAAGATCAAGCCTCTGCAC
AACTTCCTGACCCAGCTCCAGCCCTTCCTGAAGCTCAAGCAGAAGCAGGCCAAC
CTCGTGCTGAAGATCATCGAGCAGCTGCCCTCCGCCAAGGAATCCCCGGACAAG
TTCCTGGAGGTGTGCACCTGGGTGGACCAGATCGCCGCTCTGAACGACTCCAAG
ACCCGCAAGACCACTTCCGAAACCGTCCGCGCCGTTCTAGACAGTCTCTCCGAGA
AGAAGAAGTCGTCCCCCGACTACAAGGACGACGACGACAAGTAATGAGGTACCA
GCGGCCGCACTCATCTTGGCCCTCCTCAGCTCCCTGCCTGTTTCCCGTAAGGCTGT
ACATAGTCCTTTTATCTCCTTGTGGCCTATGAAACTGGTTTATAATAAACTCTTAA
GAGAACATTAGGCGCGCCAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AA
SEQ ID NO: 26 
TAATACGACTCACTATAAGGGGCATTTCCGGTAGCGGCGGGAAATCGGCTG
TGGGAGAGAGGCTAGGCCTCTGAGGAGGCGAATCCGGCGGTATCAGAGCCATC
AGAACCGCCACCATGGCACCGAAGAAGAAGCGCAAGGTGCATATGAACACCAA
GTACAACAAGGAGTTCCTGCTCTACCTGGCGGGCTTCGTCGACTCCGACGGCTCC
ATCTACGCCGCCATCCGCCCGAGCCAGACCGCCAAGTTCAAGCATCGGCTGCAG
CTGTTCTTCGCCGTCTACCAGAAGACACAGCGCCGTTGGTTCCTCGACAAGCTGG
TGGACGAGATCGGGGTGGGCTACGTGACCGACGCCGGCAGCGTCAGCAGCTACT
TCCTGTCCGAGATCAAGCCTCTGCACAACTTCCTGACCCAGCTCCAGCCCTTCCT
GAAGCTCAAGCAGAAGCAGGCCAACCTCGTGCTGAAGATCATCGAGCAGCTGCC
CTCCGCCAAGGAATCCCCGGACAAGTTCCTGGAGGTGTGCACGTGGGTCGACCA
GATCGCGGCCCTCAACGACAGCAAGACCCGCAAGACGACCTCGGAAACGGTGCG
GGCGGTCCTGGACTCCCTCCCAGGATCCGTGGGAGGTCTATCGCCATCTCAGGCA
TCCAGCGCCGCATCCTCGGCTTCCTCAAGCCCGGGTTCAGGGATCTCCGAAGCAC
TCAGAGCTGGAGCAGGTTCCGGCACTGGATACAACAAGGAATTCCTGCTCTACCT
GGCGGGCTTCGTCGACGGGGACGGCTCCATCTTCGCCTCCATCCACCCGCAGCAG
CGCAACAAGTTCAAGCATCAGCTGAGCCTCCACTTCACCGTCAGGCAGAAGACA
CAGCGCCGTTGGTTCCTCGACAAGCTGGTGGACGAGATCGGGGTGGGCTACGTG
ATCGACGAGGGCAGCGTCAGCAGCTACCGCCTGTCCAAGATCAAGCCTCTGCAC
AACTTCCTGACCCAGCTCCAGCCCTTCCTGAAGCTCAAGCAGAAGCAGGCCAAC
CTCGTGCTGAAGATCATCGAGCAGCTGCCCTCCGCCAAGGAATCCCCGGACAAG
TTCCTGGAGGTGTGCACCTGGGTGGACCAGATCGCCGCTCTGAACGACTCCAAG
ACCCGCAAGACCACTTCCGAAACCGTCCGCGCCGTTCTAGACAGTCTCTCCGAGA
AGAAGAAGTCGTCCCCCGACTACAAGGACGACGACGACAAGTAATGAGGTACCA
GCGGCCGCCCCTTGGCCACAGAGTATGGAAGTAGCTCCGCAGAGGCGTGGGCTC
GATTCCTCAGGGCCACGTTACCACAGACCTGTTTGTTTCTTATGCTGTTGTTCGTG
GAGTCTCATGGGATTGTCTGGTTTCCCTTACAGGGCCCCCTCCCCCGGGAATGCG
CCCACCAAGGCCCTAGACTCATCTTGGCCCTCCTCAGCTCCCTGCCTGTTTCCCGT
AAGGCTGTACATAGTCCTTTTATCTCCTTGTGGCCTATGAAACTGGTTTATAATAA
ACTCTTAAGAGAACATTAGGCGCGCCAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAA
SEQ ID NO: 27 
TAATACGACTCACTATAAGGGCATAAACCCTGGCGCGCTCGCGGGCCGGCACTC
TTCTGGTCCCCACAGACTCAGAGAGAACCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCATCAACCTCTGGATTACAAAATTT
GTGAAAGATTGACTGATATTCTTAACTATGTTGCTCCTTTTACGCTGTGTGGATAT
GCTGCTTTAATGCCTCTGTATCATGCTATTGCTTCCCGTACGGCTTTCGTTTTCTCC
TCCTTGTATAAATCCTGGTTGCTGTCTCTTTATGAGGAGTTGTGGCCCGTTGTCCG
TCAACGTGGCGTGGTGTGCTCTGTGTTTGCTGACGCAACCCCCACTGGCTGGGGC
ATTGCCACCACCTGTCAACTCCTTTCTGGGACTTTCGCTTTCCCCCTCCCGATCGC
CACGGCAGAACTCATCGCCGCCTGCCTTGCCCGCTGCTGGACAGGGGCTAGGTTG
CTGGGCACTGATAATTCCGTGGTGTTGTCGGGGAAGCTGACGTCCTTTCCAGGGC
TGCTCGCCTGTGTTGCCAACTGGATCCTGCGCGGGACGTCCTTCTGCTACGTCCCT
TCGGCTCTCAATCCAGCGGACCTCCCTTCCCGAGGCCTTCTGCCGGTTCTGCGGC
CTCTCCCGCGTCTTCGCTTTCGGCCTCCGACGAGTCGGATCTCCCTTTGGGCCGCC
TCCCCGCCTGGCGCGCCAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
A
SEQ ID NO: 28 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
 
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCGCTGGAGCCTCGGTGGCCATGCTT
CTTGCCCCTTGGGCCTCCCCCCAGCCCCTCCTCCCCTTCCTGCACCCGTACCCCCG
TGGTCTTTGAATAAAGTCTGAGTGGGCGGCAGGGCGCGCCAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAA
SEQ ID NO: 29 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCGCTCGCTTTCTTGCTGTCCAATTTC
TATTAAAGGTTCCTTTGTTCCCTAAGTCCAACTACTAAACTGGGGGATATTATGA
AGGGCCTTGAGCATCTGGATTCTGCCTAATAAAAAACATTTATTTTCATTGCGGC
GCGCCAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAA
SEQ ID NO: 30 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCACCAG
CCTCAAGAACACCCGAATGGAGTCTCTAAGCTACATAATACCAACTTACACTTTA
CAAAATGTTGTCCCCCAAAATGTAGCCATTCGTATCTGCTCCTAATAAAAAGAAA
GTTTCTTCACATTCTGGCGCGCCAAAAAAAAAAAAAAAAAAAAAAAAAAAAAA
AAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA
AAAAAA
SEQ ID NO: 31 
TAATACGACTCACTATAAGGGAGGTTGGGAACTAGGAGTGGCAGCAATCCTTTC
TTTCAGCTGGAGTGCTCCTCAGGAGCCAGCCCCACCCTTAGCCACCATGGCACCG
AAGAAGAAGCGCAAGGTGCATATGAACACCAAGTACAACAAGGAGTTCCTGCTC
TACCTGGCGGGCTTCGTCGACTCCGACGGCTCCATCTACGCCGCCATCCGCCCGA
GCCAGACCGCCAAGTTCAAGCATCGGCTGCAGCTGTTCTTCGCCGTCTACCAGAA
GACACAGCGCCGTTGGTTCCTCGACAAGCTGGTGGACGAGATCGGGGTGGGCTA
CGTGACCGACGCCGGCAGCGTCAGCAGCTACTTCCTGTCCGAGATCAAGCCTCTG
CACAACTTCCTGACCCAGCTCCAGCCCTTCCTGAAGCTCAAGCAGAAGCAGGCC
AACCTCGTGCTGAAGATCATCGAGCAGCTGCCCTCCGCCAAGGAATCCCCGGAC
AAGTTCCTGGAGGTGTGCACGTGGGTCGACCAGATCGCGGCCCTCAACGACAGC
AAGACCCGCAAGACGACCTCGGAAACGGTGCGGGCGGTCCTGGACTCCCTCCCA
GGATCCGTGGGAGGTCTATCGCCATCTCAGGCATCCAGCGCCGCATCCTCGGCTT
CCTCAAGCCCGGGTTCAGGGATCTCCGAAGCACTCAGAGCTGGAGCAGGTTCCG
GCACTGGATACAACAAGGAATTCCTGCTCTACCTGGCGGGCTTCGTCGACGGGG
ACGGCTCCATCTTCGCCTCCATCCACCCGCAGCAGCGCAACAAGTTCAAGCATCA
GCTGAGCCTCCACTTCACCGTCAGGCAGAAGACACAGCGCCGTTGGTTCCTCGAC
AAGCTGGTGGACGAGATCGGGGTGGGCTACGTGATCGACGAGGGCAGCGTCAGC
AGCTACCGCCTGTCCAAGATCAAGCCTCTGCACAACTTCCTGACCCAGCTCCAGC
CCTTCCTGAAGCTCAAGCAGAAGCAGGCCAACCTCGTGCTGAAGATCATCGAGC
AGCTGCCCTCCGCCAAGGAATCCCCGGACAAGTTCCTGGAGGTGTGCACCTGGG
TGGACCAGATCGCCGCTCTGAACGACTCCAAGACCCGCAAGACCACTTCCGAAA
CCGTCCGCGCCGTTCTAGACAGTCTCTCCGAGAAGAAGAAGTCGTCCCCCGACTA
CAAGGACGACGACGACAAGTAATGAGGTACCAGCGGCCGCACCAGCCTCAAGA
ACACCCGAATGGAGTCTCTAAGCTACATAATACCAACTTACACTTTACAAAATGT
TGTCCCCCAAAATGTAGCCATTCGTATCTGCTCCTAATAAAAAGAAAGTTTCTTC
ACATTCTGGCGCGCCAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
SEQ ID NO: 32 
TAATACGACTCACTATAAGGGATAAGAGACCACAAGCGACCCGCAGGGCCAGAC
GTTCTTCGCCGAGAGTCGTCGGGGTTTCCTGCTTCAACAGTGCTTGGACGGAACC
CGGCGCTCGTTCCCCACCCCGGCCGGCCGCCCATAGCCAGCCCTCCGTCACCTCT
TCACCGCACCCTCGGACTGCCCCAAGGCCCCCGCCGCCGCTCCAGCGCCGCGCA
GCCACCGCCGCCGCCGCCGCCTGCCACCATGGCACCGAAGAAGAAGCGCAAGGT
GCATATGAACACCAAGTACAACAAGGAGTTCCTGCTCTACCTGGCGGGCTTCGTC
GACTCCGACGGCTCCATCTACGCCGCCATCCGCCCGAGCCAGACCGCCAAGTTCA
AGCATCGGCTGCAGCTGTTCTTCGCCGTCTACCAGAAGACACAGCGCCGTTGGTT
CCTCGACAAGCTGGTGGACGAGATCGGGGTGGGCTACGTGACCGACGCCGGCAG
CGTCAGCAGCTACTTCCTGTCCGAGATCAAGCCTCTGCACAACTTCCTGACCCAG
CTCCAGCCCTTCCTGAAGCTCAAGCAGAAGCAGGCCAACCTCGTGCTGAAGATC
ATCGAGCAGCTGCCCTCCGCCAAGGAATCCCCGGACAAGTTCCTGGAGGTGTGC
ACGTGGGTCGACCAGATCGCGGCCCTCAACGACAGCAAGACCCGCAAGACGACC
TCGGAAACGGTGCGGGCGGTCCTGGACTCCCTCCCAGGATCCGTGGGAGGTCTA
TCGCCATCTCAGGCATCCAGCGCCGCATCCTCGGCTTCCTCAAGCCCGGGTTCAG
GGATCTCCGAAGCACTCAGAGCTGGAGCAGGTTCCGGCACTGGATACAACAAGG
AATTCCTGCTCTACCTGGCGGGCTTCGTCGACGGGGACGGCTCCATCTTCGCCTC
CATCCACCCGCAGCAGCGCAACAAGTTCAAGCATCAGCTGAGCCTCCACTTCACC
GTCAGGCAGAAGACACAGCGCCGTTGGTTCCTCGACAAGCTGGTGGACGAGATC
 
GGGGTGGGCTACGTGATCGACGAGGGCAGCGTCAGCAGCTACCGCCTGTCCAAG
ATCAAGCCTCTGCACAACTTCCTGACCCAGCTCCAGCCCTTCCTGAAGCTCAAGC
AGAAGCAGGCCAACCTCGTGCTGAAGATCATCGAGCAGCTGCCCTCCGCCAAGG
AATCCCCGGACAAGTTCCTGGAGGTGTGCACCTGGGTGGACCAGATCGCCGCTCT
GAACGACTCCAAGACCCGCAAGACCACTTCCGAAACCGTCCGCGCCGTTCTAGA
CAGTCTCTCCGAGAAGAAGAAGTCGTCCCCCGACTACAAGGACGACGACGACAA
GTAATGAGGTACCAGCGGCCGCACCAGCCTCAAGAACACCCGAATGGAGTCTCT
AAGCTACATAATACCAACTTACACTTTACAAAATGTTGTCCCCCAAAATGTAGCC
ATTCGTATCTGCTCCTAATAAAAAGAAAGTTTCTTCACATTCTGGCGCGCCAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAA
SEQ ID NO: 33 
TAATACGACTCACTATAAGGGCTCTCTGCTCCTCCTGTTCGACAGTCAGCCGCA
TCTTCTTTTGCGTCGCCAGCCGAGCCACATCGCTCAGCCACCATGGCACCGAAGA
AGAAGCGCAAGGTGCATATGAACACCAAGTACAACAAGGAGTTCCTGCTCTACC
TGGCGGGCTTCGTCGACTCCGACGGCTCCATCTACGCCGCCATCCGCCCGAGCCA
GACCGCCAAGTTCAAGCATCGGCTGCAGCTGTTCTTCGCCGTCTACCAGAAGACA
CAGCGCCGTTGGTTCCTCGACAAGCTGGTGGACGAGATCGGGGTGGGCTACGTG
ACCGACGCCGGCAGCGTCAGCAGCTACTTCCTGTCCGAGATCAAGCCTCTGCACA
ACTTCCTGACCCAGCTCCAGCCCTTCCTGAAGCTCAAGCAGAAGCAGGCCAACCT
CGTGCTGAAGATCATCGAGCAGCTGCCCTCCGCCAAGGAATCCCCGGACAAGTT
CCTGGAGGTGTGCACGTGGGTCGACCAGATCGCGGCCCTCAACGACAGCAAGAC
CCGCAAGACGACCTCGGAAACGGTGCGGGCGGTCCTGGACTCCCTCCCAGGATC
CGTGGGAGGTCTATCGCCATCTCAGGCATCCAGCGCCGCATCCTCGGCTTCCTCA
AGCCCGGGTTCAGGGATCTCCGAAGCACTCAGAGCTGGAGCAGGTTCCGGCACT
GGATACAACAAGGAATTCCTGCTCTACCTGGCGGGCTTCGTCGACGGGGACGGC
TCCATCTTCGCCTCCATCCACCCGCAGCAGCGCAACAAGTTCAAGCATCAGCTGA
GCCTCCACTTCACCGTCAGGCAGAAGACACAGCGCCGTTGGTTCCTCGACAAGCT
GGTGGACGAGATCGGGGTGGGCTACGTGATCGACGAGGGCAGCGTCAGCAGCTA
CCGCCTGTCCAAGATCAAGCCTCTGCACAACTTCCTGACCCAGCTCCAGCCCTTC
CTGAAGCTCAAGCAGAAGCAGGCCAACCTCGTGCTGAAGATCATCGAGCAGCTG
CCCTCCGCCAAGGAATCCCCGGACAAGTTCCTGGAGGTGTGCACCTGGGTGGAC
CAGATCGCCGCTCTGAACGACTCCAAGACCCGCAAGACCACTTCCGAAACCGTC
CGCGCCGTTCTAGACAGTCTCTCCGAGAAGAAGAAGTCGTCCCCCGACTACAAG
GACGACGACGACAAGTAATGAGGTACCAGCGGCCGCACCAGCCTCAAGAACACC
CGAATGGAGTCTCTAAGCTACATAATACCAACTTACACTTTACAAAATGTTGTCC
CCCAAAATGTAGCCATTCGTATCTGCTCCTAATAAAAAGAAAGTTTCTTCACATT
CTGGCGCGCCAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA
SEQ ID NO: 34 
TAATACGACTCACTATAAGGGCATAAACCCTGGCGCGCTCGCGGCCGGCACTC
TTCTGGTCCCCACAGACTCAGAGAGAAGCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCACCAGCCTCAAGAACACCCGAATG
GAGTCTCTAAGCTACATAATACCAACTTACACTTACAAAATGTTGTCCCCCAAA
ATGTAGCCATTCGTATCTGCTCCTAATAAAAAGAAAGTTTCTTCACATTCTGGCG
CGCCAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAA
SEQ ID NO: 35 
TAATACGACTCACTATAAGGGGCATTTCCGGTAGCGGCGGGAAATCGGCTG
TGGGAGAGAGGCTAGGCCTCTGAGGAGGCGAATCCGGCGGGTATCAGAGCCATC
AGAACCGCCACCATGGCACCGAAGAAGAAGCGCAAGGTGCATATGAACACCAA
GTACAACAAGGAGTTCCTGCTCTACCTGGCGGGCTTCGTCGACTCCGACGGCTCC
ATCTACGCCGCCATCCGCCCGAGCCAGACCGCCAAGTTCAAGCATCGGCTGCAG
CTGTTCTTCGCCGTCTACCAGAAGACACAGCGCCGTTGGTTCCTCGACAAGCTGG
TGGACGAGATCGGGGTGGGCTACGTGACCGACGCCGGCAGCGTCAGCAGCTACT
TCCTGTCCGAGATCAAGCCTCTGCACAACTTCCTGACCCAGCTCCAGCCCTTCCT
GAAGCTCAAGCAGAAGCAGGCCAACCTCGTGCTGAAGATCATCGAGCAGCTGCC
CTCCGCCAAGGAATCCCCGGACAAGTTCCTGGAGGTGTGCACGTGGGTCGACCA
GATCGCGGCCCTCAACGACAGCAAGACCCGCAAGACGACCTCGGAAACGGTGCG
GGCGGTCCTGGACTCCCTCCCAGGATCCGTGGGAGGTCTATCGCCATCTCAGGCA
TCCAGCGCCGCATCCTCGGCTTCCTCAAGCCCGGGTTCAGGGATCTCCGAAGCAC
TCAGAGCTGGAGCAGGTTCCGGCACTGGATACAACAAGGAATTCCTGCTCTACCT
GGCGGGCTTCGTCGACGGGGACGGCTCCATCTTCGCCTCCATCCACCCGCAGCAG
CGCAACAAGTTCAAGCATCAGCTGAGCCTCCACTTCACCGTCAGGCAGAAGACA
CAGCGCCGTTGGTTCCTCGACAAGCTGGTGGACGAGATCGGGGTGGGCTACGTG
ATCGACGAGGGCAGCGTCAGCAGCTACCGCCTGTCCAAGATCAAGCCTCTGCAC
AACTTCCTGACCCAGCTCCAGCCCTTCCTGAAGCTCAAGCAGAAGCAGGCCAAC
 
CTCGTGCTGAAGATCATCGAGCAGCTGCCCTCCGCCAAGGAATCCCCGGACAAG
TTCCTGGAGGTGTGCACCTGGGTGGACCAGATCGCCGCTCTGAACGACTCCAAG
ACCCGCAAGACCACTTCCGAAACCGTCCGCGCCGTTCTAGACAGTCTCTCCGAGA
AGAAGAAGTCGTCCCCCGACTACAAGGACGACGACGACAAGTAATGAGGTACCA
GCGGCCGCACCAGCCTCAAGAACACCCGAATGGAGTCTCTAAGCTACATAATAC
CAACTTACACTTTACAAAATGTTGTCCCCCAAAATGTAGCCATTCGTATCTGCTC
CTAATAAAAAGAAAGTTTCTTCACATTCTGGCGCGCCAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAA
SEQ ID NO: 36 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCGCTGG
AGCCTCGGTGGCCATGCTTCTTGCCCCTTGGGCCTCCCCCCAGCCCCTCCTCCCCT
TCCTGCACCCGTACCCCCGTGGTCTTTGAATAAAGTCTGAGTGGGCGGCAGGGCG
CGCCAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAA
SEQ ID NO: 37 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
 
AACGACAGCAAGACCCGCAAGACGACCTCGGAAACGGTGCGGGCGGTCCTGGA
CTCCCTCCCAGGATCCGTGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCGCTCG
CTTTCTTGCTGTCCAATTTCTATTAAAGGTTCCTTTGTTCCCTAAGTCCAACTACT
AAACTGGGGGATATTATGAAGGGCCTTGAGCATCTGGATTCTGCCTAATAAAAA
ACATTTATTTTCATTGCGGCGCGCCAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAA
SEQ ID NO: 38 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCACTCA
TCTTGGCCCTCCTCAGCTCCCTGCCTGTTTCCCGTAAGGCTGTACATAGTCCTTTT
ATCTCCTTGTGGCCTATGAAACTGGTTTATAATAAACTCTTAAGAGAACATTAGG
CGCGCCAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAA
SEQ ID NO: 39 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCGACTACAAGGACGACGACGACAAGTAATGAGGTACCAGCGGCCGCACTCA
TCTTGGCCCTCCTCAGCTCCCTGCCTGTTTCCCGTAAGGCTGTACATAGTCCTTTT
ATCTCCTTGTGGCCTATGAAACTGGTTTATAATAAACTCTTAAGAGAACATTA
SEQ ID NO: 40 
TAATACGACTCACTATAAGGGAGGTTGGGAACTAGGAGTGGCAGCAATCCTTTC
TTTCAGCTGGAGTGCTCCTCAGGAGCCAGCCCACCCTTAGCCACCATGGCACCG
AAGAAGAAGCGCAAGGTGCATATGAACACCAAGTACAACAAGGAGTTCCTGCTC
TACCTGGCGGGCTTCGTCGACTCCGACGGCTCCATCTACGCCGCCATCCGCCCGA
GCCAGACCGCCAAGTTCAAGCATCGGCTGCAGCTGTTCTTCGCCGTCTACCAGAA
GACACAGCGCCGTTGGTTCCTCGACAAGCTGGTGGACGAGATCGGGGTGGGCTA
CGTGACCGACGCCGGCAGCGTCAGCAGCTACTTCCTGTCCGAGATCAAGCCTCTG
CACAACTTCCTGACCCAGCTCCAGCCCTTCCTGAAGCTCAAGCAGAAGCAGGCC
AACCTCGTGCTGAAGATCATCGAGCAGCTGCCCTCCGCCAAGGAATCCCCGGAC
AAGTTCCTGGAGGTGTGCACGTGGGTCGACCAGATCGCGGCCCTCAACGACAGC
AAGACCCGCAAGACGACCTCGGAAACGGTGCGGGCGGTCCTGGACTCCCTCCCA
GGATCCGTGGGAGGTCTATCGCCATCTCAGGCATCCAGCGCCGCATCCTCGGCTT
CCTCAAGCCCGGGTTCAGGGATCTCCGAAGCACTCAGAGCTGGAGCAGGTTCCG
GCACTGGATACAACAAGGAATTCCTGCTCTACCTGGCGGGCTTCGTCGACGGGG
ACGGCTCCATCTTCGCCTCCATCCACCCGCAGCAGCGCAACAAGTTCAAGCATCA
GCTGAGCCTCCACTTCACCGTCAGGCAGAAGACACAGCGCCGTTGGTTCCTCGAC
AAGCTGGTGGACGAGATCGGGGTGGGCTACGTGATCGACGAGGGCAGCGTCAGC
AGCTACCGCCTGTCCAAGATCAAGCCTCTGCACAACTTCCTGACCCAGCTCCAGC
CCTTCCTGAAGCTCAAGCAGAAGCAGGCCAACCTCGTGCTGAAGATCATCGAGC
AGCTGCCCTCCGCCAAGGAATCCCCGGACAAGTTCCTGGAGGTGTGCACCTGGG
TGGACCAGATCGCCGCTCTGAACGACTCCAAGACCCGCAAGACCACTTCCGAAA
CCGTCCGCGCCGTTCTAGACAGTCTCTCCGAGAAGAAGAAGTCGTCCCCCGACTA
CAAGGACGACGACGACAAGTAATGAGGTACCAGCGGCCGCACTCATCTTGGCCC
 
TCCTCAGCTCCCTGCCTGTTTCCCGTAAGGCTGTACATAGTCCTTTTATCTCCTTG
TGGCCTATGAAACTGGTTTATAATAAACTCTTAAGAGAACATTAGGCGCGCCAA
AAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAA
SEQ ID NO: 41 
TAATACGACTCACTATAAGGGATAAGAGACCACAAGCGACCCGCAGGGCCAGAC
GTTCTTCGCCGAGAGTCGTCGGGGTTTCCTGCTTCAACAGTGCTTGGACGGAACC
CGGCGCTCGTTCCCCACCCCGGCCGGCCGCCCATAGCCAGCCCTCCGTCACCTCT
TCACCGCACCCTCGGACTGCCCCAAGGCCCCCGCCGCCGCTCCAGCGCCGCGCA
GCCACCGCCGCCGCCGCCGCCTGCCACCATGGCACCGAAGAAGAAGCGCAAGGT
GCATATGAACACCAAGTACAACAAGGAGTTCCTGCTCTACCTGGCGGGCTTCGTC
GACTCCGACGGCTCCATCTACGCCGCCATCCGCCCGAGCCAGACCGCCAAGTTCA
AGCATCGGCTGCAGCTGTTCTTCGCCGTCTACCAGAAGACACAGCGCCGTTGGTT
CCTCGACAAGCTGGTGGACGAGATCGGGGTGGGCTACGTGACCGACGCCGGCAG
CGTCAGCAGCTACTTCCTGTCCGAGATCAAGCCTCTGCACAACTTCCTGACCCAG
CTCCAGCCCTTCCTGAAGCTCAAGCAGAAGCAGGCCAACCTCGTGCTGAAGATC
ATCGAGCAGCTGCCCTCCGCCAAGGAATCCCCGGACAAGTTCCTGGAGGTGTGC
ACGTGGGTCGACCAGATCGCGGCCCTCAACGACAGCAAGACCCGCAAGACGACC
TCGGAAACGGTGCGGGCGGTCCTGGACTCCCTCCCAGGATCCGTGGGAGGTCTA
TCGCCATCTCAGGCATCCAGCGCCGCATCCTCGGCTTCCTCAAGCCCGGGTTCAG
GGATCTCCGAAGCACTCAGAGCTGGAGCAGGTTCCGGCACTGGATACAACAAGG
AATTCCTGCTCTACCTGGCGGGCTTCGTCGACGGGGACGGCTCCATCTTCGCCTC
CATCCACCCGCAGCAGCGCAACAAGTTCAAGCATCAGCTGAGCCTCCACTTCACC
GTCAGGCAGAAGACACAGCGCCGTTGGTTCCTCGACAAGCTGGTGGACGAGATC
GGGGTGGGCTACGTGATCGACGAGGGCAGCGTCAGCAGCTACCGCCTGTCCAAG
ATCAAGCCTCTGCACAACTTCCTGACCCAGCTCCAGCCCTTCCTGAAGCTCAAGC
AGAAGCAGGCCAACCTCGTGCTGAAGATCATCGAGCAGCTGCCCTCCGCCAAGG
AATCCCCGGACAAGTTCCTGGAGGTGTGCACCTGGGTGGACCAGATCGCCGCTCT
GAACGACTCCAAGACCCGCAAGACCACTTCCGAAACCGTCCGCGCCGTTCTAGA
CAGTCTCTCCGAGAAGAAGAAGTCGTCCCCCGACTACAAGGACGACGACGACAA
GTAATGAGGTACCAGCGGCCGCACTCATCTTGGCCCTCCTCAGCTCCCTGCCTGT
TTCCCGTAAGGCTGTACATAGTCCTTTTATCTCCTTGTGGCCTATGAAACTGGTTT
ATAATAAACTCTTAAGAGAACATTAGGCGCGCCAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAA
SEQ ID NO: 42 
TAATACGACTCACTATAAGGGGCTCTCTGCTCCTCCTGTTCGACAGTCAGCCGCA
TCTTCTTTTGCGTCGCCAGCCGAGCCACATCGCTCAGCCACCATGGCACCGAAGA
AGAAGCGCAAGGTGCATATGAACACCAAGTACAACAAGGAGTTCCTGCTCTACC
TGGCGGGCTTCGTCGACTCCGACGGCTCCATCTACGCCGCCATCCGCCCGAGCCA
GACCGCCAAGTTCAAGCATCGGCTGCAGCTGTTCTTCGCCGTCTACCAGAAGACA
CAGCGCCGTTGGTTCCTCGACAAGCTGGTGGACGAGATCGGGGTGGGCTACGTG
ACCGACGCCGGCAGCGTCAGCAGCTACTTCCTGTCCGAGATCAAGCCTCTGCACA
ACTTCCTGACCCAGCTCCAGCCCTTCCTGAAGCTCAAGCAGAAGCAGGCCAACCT
CGTGCTGAAGATCATCGAGCAGCTGCCCTCCGCCAAGGAATCCCCGGACAAGTT
CCTGGAGGTGTGCACGTGGGTCGACCAGATCGCGGCCCTCAACGACAGCAAGAC
 
CCGCAAGACGACCTCGGAAACGGTGCGGGCGGTCCTGGACTCCCTCCCAGGATC
CGTGGGAGGTCTATCGCCATCTCAGGCATCCAGCGCCGCATCCTCGGCTTCCTCA
AGCCCGGGTTCAGGGATCTCCGAAGCACTCAGAGCTGGAGCAGGTTCCGGCACT
GGATACAACAAGGAATTCCTGCTCTACCTGGCGGGCTTCGTCGACGGGGACGGC
TCCATCTTCGCCTCCATCCACCCGCAGCAGCGCAACAAGTTCAAGCATCAGCTGA
GCCTCCACTTCACCGTCAGGCAGAAGACACAGCGCCGTTGGTTCCTCGACAAGCT
GGTGGACGAGATCGGGGTGGGCTACGTGATCGACGAGGGCAGCGTCAGCAGCTA
CCGCCTGTCCAAGATCAAGCCTCTGCACAACTTCCTGACCCAGCTCCAGCCCTTC
CTGAAGCTCAAGCAGAAGCAGGCCAACCTCGTGCTGAAGATCATCGAGCAGCTG
CCCTCCGCCAAGGAATCCCCGGACAAGTTCCTGGAGGTGTGCACCTGGGTGGAC
CAGATCGCCGCTCTGAACGACTCCAAGACCCGCAAGACCACTTCCGAAACCGTC
CGCGCCGTTCTAGACAGTCTCTCCGAGAAGAAGAAGTCGTCCCCCGACTACAAG
GACGACGACGACAAGTAATGAGGTACCAGCGGCCGCACTCATCTTGGCCCTCCT
CAGCTCCCTGCCTGTTTCCCGTAAGGCTGTACATAGTCCTTTTATCTCCTTGTGGC
CTATGAAACTGGTTTATAATAAACTCTTAAGAGAACATTAGGCGCGCCAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAA
SEQ ID NO: 43 
TAATACGACTCACTATAAGGGCATAAACCCTGGCGCGCTCGCGGCCGGCACTC
TTCTGGTCCCCACAGACTCAGAGAGAAGCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCACTCATCTTGGCCCTCCTCAGCTCC
CTGCCTGTTTCCCGTAAGGCTGTACATAGTCCTTTTATCTCCTTGTGGCCTATGAA
ACTGGTTTATAATAAACTCTTAAGAGAACATTAGGCGCGCCAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAA
SEQ ID NO: 44 
TAATACGACTCACTATAAGGGGCATTTCCGGTAGCGGCGGCGGGAAATCGGCTG
TGGGAGAGAGGCTAGGCCTCTGAGGAGGCGAATCCGGCGGGTATCAGAGCCATC
AGAACCGCCACCATGGCACCGAAGAAGAAGCGCAAGGTGCATATGAACACCAA
GTACAACAAGGAGTTCCTGCTCTACCTGGCGGGCTTCGTCGACTCCGACGGCTCC
ATCTACGCCGCCATCCGCCCGAGCCAGACCGCCAAGTTCAAGCATCGGCTGCAG
CTGTTCTTCGCCGTCTACCAGAAGACACAGCGCCGTTGGTTCCTCGACAAGCTGG
TGGACGAGATCGGGGTGGGCTACGTGACCGACGCCGGCAGCGTCAGCAGCTACT
TCCTGTCCGAGATCAAGCCTCTGCACAACTTCCTGACCCAGCTCCAGCCCTTCCT
GAAGCTCAAGCAGAAGCAGGCCAACCTCGTGCTGAAGATCATCGAGCAGCTGCC
CTCCGCCAAGGAATCCCCGGACAAGTTCCTGGAGGTGTGCACGTGGGTCGACCA
GATCGCGGCCCTCAACGACAGCAAGACCCGCAAGACGACCTCGGAAACGGTGCG
GGCGGTCCTGGACTCCCTCCCAGGATCCGTGGGAGGTCTATCGCCATCTCAGGCA
TCCAGCGCCGCATCCTCGGCTTCCTCAAGCCCGGGTTCAGGGATCTCCGAAGCAC
TCAGAGCTGGAGCAGGTTCCGGCACTGGATACAACAAGGAATTCCTGCTCTACCT
GGCGGGCTTCGTCGACGGGGACGGCTCCATCTTCGCCTCCATCCACCCGCAGCAG
CGCAACAAGTTCAAGCATCAGCTGAGCCTCCACTTCACCGTCAGGCAGAAGACA
CAGCGCCGTTGGTTCCTCGACAAGCTGGTGGACGAGATCGGGGTGGGCTACGTG
ATCGACGAGGGCAGCGTCAGCAGCTACCGCCTGTCCAAGATCAAGCCTCTGCAC
AACTTCCTGACCCAGCTCCAGCCCTTCCTGAAGCTCAAGCAGAAGCAGGCCAAC
CTCGTGCTGAAGATCATCGAGCAGCTGCCCTCCGCCAAGGAATCCCCGGACAAG
TTCCTGGAGGTGTGCACCTGGGTGGACCAGATCGCCGCTCTGAACGACTCCAAG
ACCCGCAAGACCACTTCCGAAACCGTCCGCGCCGTTCTAGACAGTCTCTCCGAGA
AGAAGAAGTCGTCCCCCGACTACAAGGACGACGACGACAAGTAATGAGGTACCA
GCGGCCGCGCTGGAGCCTCGGTGGCCATGCTTCTTGCCCCTTGGGCCTCCCCCCA
GCCCCTCCTCCCCTTCCTGCACCCGTACCCCCGTGGTCTTTGAATAAAGTCTGAGT
GGGCGGCAGGGCGCGCCAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAA
A
SEQ ID NO: 45 
TAATACGACTCACTATAAGGGCATTTCCGGTAGCGGCGGGAAATCGGCTG
TGGGAGAGAGGCTAGGCCTCTGAGGAGGCGAATCCGGCGGGTATCAGAGCCATC
AGAACCGCCACCATGGCACCGAAGAAGCGCAAGGTGCATATGAACACCAA
GTACAACAAGGAGTTCCTGCTCTACCTGGCGGGCTTCGTCGACTCCGACGGCTCC
ATCTACGCCGCCATCCGCCCGAGCCAGACCGCCAAGTTCAAGCATCGGCTGCAG
CTGTTCTTCGCCGTCTACCAGAAGACACAGCGCCGTTGGTTCCTCGACAAGCTGG
TGGACGAGATCGGGGTGGGCTACGTGACCGACGCCGGCAGCGTCAGCAGCTACT
TCCTGTCCGAGATCAAGCCTCTGCACAACTTCCTGACCCAGCTCCAGCCCTTCCT
GAAGCTCAAGCAGAAGCAGGCCAACCTCGTGCTGAAGATCATCGAGCAGCTGCC
CTCCGCCAAGGAATCCCCGGACAAGTTCCTGGAGGTGTGCACGTGGGTCGACCA
GATCGCGGCCCTCAACGACAGCAAGACCCGCAAGACGACCTCGGAAACGGTGCG
GGCGGTCCTGGACTCCCTCCCAGGATCCGTGGGAGGTCTATCGCCATCTCAGGCA
TCCAGCGCCGCATCCTCGGCTTCCTCAAGCCCGGGTTCAGGGATCTCCGAAGCAC
TCAGAGCTGGAGCAGGTTCCGGCACTGGATACAACAAGGAATTCCTGCTCTACCT
GGCGGGCTTCGTCGACGGGGACGGCTCCATCTTCGCCTCCATCCACCCGCAGCAG
CGCAACAAGTTCAAGCATCAGCTGAGCCTCCACTTCACCGTCAGGCAGAAGACA
CAGCGCCGTTGGTTCCTCGACAAGCTGGTGGACGAGATCGGGGTGGGCTACGTG
ATCGACGAGGGCAGCGTCAGCAGCTACCGCCTGTCCAAGATCAAGCCTCTGCAC
AACTTCCTGACCCAGCTCCAGCCCTTCCTGAAGCTCAAGCAGAAGCAGGCCAAC
CTCGTGCTGAAGATCATCGAGCAGCTGCCCTCCGCCAAGGAATCCCCGGACAAG
TTCCTGGAGGTGTGCACCTGGGTGGACCAGATCGCCGCTCTGAACGACTCCAAG
ACCCGCAAGACCACTTCCGAAACCGTCCGCGCCGTTCTAGACAGTCTCTCCGAGA
AGAAGAAGTCGTCCCCCGACTACAAGGACGACGACGACAAGTAATGAGGTACCA
GCGGCCGCGCTCGCTTTCTTGCTGTCCAATTTCTATTAAAGGTTCCTTTGTTCCCT
AAGTCCAACTACTAAACTGGGGGATATTATGAAGGGCCTTGAGCATCTGGATTCT
GCCTAATAAAAAACATTTATTTTCATTGCGGCGCGCCAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAA
SEQ ID NO: 46 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTGGGCCCATATCGAGCCTTGCCAGTGGGTGAA
GTTCAAGCACAGGCTGAGGCTCTCTCTCAATGTCACTCAGAAGACACAGCGCCGT
TGGTTCCTCGACAAGCTGGTGGACGAGATCGGTGTGGGTTACGTGCGTGACACG
GGCAGCGTCTCCCAGTACCATCTGTCCGAGATCAAGCCTTTGCATAATTTTTTAA
CACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
 
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAA
SEQ ID NO: 47 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTGGGCCTATATCGAGCCTTGCCAGTGGGTGAA
GTTCAAGCACAGGCTGAAGCTCCAGCTCAATGTCACTCAGAAGACACAGCGCCG
TTGGTTCCTCGACAAGCTGGTGGACGAGATCGGTGTGGGTTACGTGCGTGACACG
GGCAGCGTCTCCCAGTACATGCTGTCCGAGATCAAGCCTTTGCATAATTTTTTAA
CACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
 
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA
AAAAAAAAAAAAAAAAAAAAAA
SEQ ID NO: 48 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTGGGCCCATATCGAGCCTTGCCAGTGGGTGAA
GTTCAAGCACAGGCTGAGGCTCTCTCTCAATGTCACTCAGAAGACACAGCGCCGT
TGGTTCCTCGACAAGCTGGTGGACGAGATCGGTGTGGGTTACGTGCGTGACACG
GGCAGCGTCTCCCAGTACCATCTGTCCGAGATCAAGCCTTTGCATAATTTTTAA
CACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAA
SEQ ID NO: 49 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGCGC
AAGGTGCATATGAATACAAAATATAATAAAGAGTTCTTACTCTACTTAGCAGGGT
TTGTAGACGCTGACGGTTCCATCTGGGCCTATATCGAGCCTTGCCAGTGGGTGAA
 
GTTCAAGCACAGGCTGAAGCTCCAGCTCAATGTCACTCAGAAGACACAGCGCCG
TTGGTTCCTCGACAAGCTGGTGGACGAGATCGGTGTGGGTTACGTGCGTGACACG
GGCAGCGTCTCCCAGTACATGCTGTCCGAGATCAAGCCTTTGCATAATTTTTTAA
CACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAA
SEQ ID NO: 50
   ACAAAA
SEQ ID NO: 51
   ACAAAC
SEQ ID NO: 52
   ACAAAG
SEQ ID NO: 53
   ACAACA
SEQ ID NO: 54
   ACAACC
SEQ ID NO: 55
   ACAACG
SEQ ID NO: 56
   ACAGAA
SEQ ID NO: 57
   ACAGAC
SEQ ID NO: 58
   ACAGAG
SEQ ID NO: 59
   ACAGCA
SEQ ID NO: 60
   ACAGCC
SEQ ID NO: 61
   ACAGCG
SEQ ID NO: 62
   ACCAAA
SEQ ID NO: 63
   ACCAAC
SEQ ID NO: 64
   ACCAAG
SEQ ID NO: 65
   ACCACA
SEQ ID NO: 66
   ACCACC
SEQ ID NO: 67
   ACCACG
SEQ ID NO: 68
   ACCGAA
SEQ ID NO: 69
   ACCGAC
SEQ ID NO: 70
   ACCGAG
SEQ ID NO: 71
   ACCGCA
SEQ ID NO: 72
   ACCGCC
SEQ ID NO: 73
   ACCGCG
SEQ ID NO: 74
   ATAAAA
SEQ ID NO: 75
   ATAAAC
SEQ ID NO: 76
   ATAAAG
SEQ ID NO: 77
   ATAACA
SEQ ID NO: 78
   ATAACC
SEQ ID NO: 79
   ATAACG
SEQ ID NO: 80
   ATAGAA
SEQ ID NO: 81
   ATAGAC
SEQ ID NO: 82
   ATAGAG
SEQ ID NO: 83
   ATAGCA
SEQ ID NO: 84
   ATAGCC
SEQ ID NO: 85
   ATAGCG
SEQ ID NO: 86
   ATCAAA
SEQ ID NO: 87
   ATCAAC
SEQ ID NO: 88
   ATCAAG
SEQ ID NO: 89
   ATCACA
SEQ ID NO: 90
   ATCACC
SEQ ID NO: 91
   ATCACG
SEQ ID NO: 92
   ATCGAA
SEQ ID NO: 93
   ATCGAC
SEQ ID NO: 94
   ATCGAG
SEQ ID NO: 95
   ATCGCA
SEQ ID NO: 96
   ATCGCC
SEQ ID NO: 97
   ATCGCG
SEQ ID NO: 98
   GCAAAA
SEQ ID NO: 99
   GCAAAC
SEQ ID NO: 100
   GCAAAG
SEQ ID NO: 101
   GCAACA
SEQ ID NO: 102
   GCAACC
SEQ ID NO: 103
   GCAACG
SEQ ID NO: 104
   GCAGAA
SEQ ID NO: 105
   GCAGAC
SEQ ID NO: 106
   GCAGAG
SEQ ID NO: 107
   GCAGCA
SEQ ID NO: 108
   GCAGCC
SEQ ID NO: 109
   GCAGCG
SEQ ID NO: 110
   GCCAAA
SEQ ID NO: 111
   GCCAAC
SEQ ID NO: 112
   GCCAAG
SEQ ID NO: 113
   GCCACA
SEQ ID NO: 114
   GCCACC
SEQ ID NO: 115
   GCCACG
SEQ ID NO: 116
   GCCGAA
SEQ ID NO: 117
   GCCGAC
SEQ ID NO: 118
   GCCGAG
SEQ ID NO: 119
   GCCGCA
SEQ ID NO: 120
   GCCGCC
SEQ ID NO: 121
   GCCGCG
SEQ ID NO: 122
   GTAAAA
SEQ ID NO: 123
   GTAAAC
SEQ ID NO: 124
   GTAAAG
SEQ ID NO: 125
   GTAACA
SEQ ID NO: 126
   GTAACC
SEQ ID NO: 127
   GTAACG
SEQ ID NO: 128
   GTAGAA
SEQ ID NO: 129
   GTAGAC
SEQ ID NO: 130
   GTAGAG
SEQ ID NO: 131
   GTAGCA
SEQ ID NO: 132
   GTAGCC
SEQ ID NO: 133
   GTAGCG
SEQ ID NO: 134
   GTCAAA
SEQ ID NO: 135
   GTCAAC
SEQ ID NO: 136
   GTCAAG
SEQ ID NO: 137
   GTCACA
SEQ ID NO: 138
   GTCACC
SEQ ID NO: 139
   GTCACG
SEQ ID NO: 140
   GTCGAA
SEQ ID NO: 141
   GTCGAC
SEQ ID NO: 142
   GTCGAG
SEQ ID NO: 143
   GTCGCA
SEQ ID NO: 144
   GTCGCC
SEQ ID NO: 145
   GTCGCG
SEQ ID NO: 146
   GGCACC
SEQ ID NO: 147
   GACACC
SEQ ID NO: 148
   CCCACC
SEQ ID NO: 149
   GGCCCC
SEQ ID NO: 150
   TTCCTCACCAATGTCTTGT
SEQ ID NO: 151
   CCACATAAGATTTGGCAAGCC
SEQ ID NO: 152
   GGAAAAGAACGACACCCTTTG
SEQ ID NO: 153
   CCCGGCTAATTTGTATCA
SEQ ID NO: 154
   GCTCACTTGATGTAAGCAACAG
SEQ ID NO: 155
   ACACACCACCAACGTAAAAC
SEQ ID NO: 156
   CCTCCCAGGAGTACTTCTCCAGG
SEQ ID NO: 157
   GATGCCTTCAGTGTCCTT
SEQ ID NO: 158
   CTTTGCTGACGTCCTAGT
SEQ ID NO: 159
   TACACGGGACACCTCACACCTG
SEQ ID NO: 160
   TGTGGTCACCCTCTGCACAGTGT
SEQ ID NO: 161
   TTGGATACAGCTTCCATCTA
SEQ ID NO: 162
   ACCAAACAAACAGTAAAATTGCC
SEQ ID NO: 163
   GAGGTCGATAAACGTTAGCCTC
SEQ ID NO: 164
   TGTGGTCACCCTCTGCACAGTGT
SEQ ID NO: 165
   CCACATAAGATTTGGCAAGCC
SEQ ID NO: 166
   TGTGGTCACCCTCTGCACAGTGT
SEQ ID NO: 167
   MAPKKKRKVH
SEQ ID NO: 168
   ATGGCCCCCAAGAAGAAGCGCAAGGTGCAT
SEQ ID NO: 169 
MNTKYNKEFLLYLAGFVDGDGSIIAQIKPNQSYKFKHQLSLAFQVTQKTQRRWFLD
KLVDEIGVGYVRDRGSVSDYILSEIKPLHNFLTQLQPFLKLKQKQANLVLKIIWRLPS
AKESPDKFLEVCTWVDQIAALNDSKTRKTTSETVRAVLDSLSEKKKSSP
SEQ ID NO: 170 
MNTKYNKEFLLYLAGFVDGDGSIIAQIKPNQSYKFKHQLSLAFQVTQKTQRRWFLD
KLVDEIGVGYVRDRGSVSDYILSEIKPLHNFLTQLQPFLKLKQKQANLVLKIIEQLPSA
KESPDKFLEVCTWVDQIAALNDSKTRKTTSETVRAVLDSLPGSVGGLSPSQASSAAS
SASSSPGSGISEALRAGAGSGTGYNKEFLLYLAGFVDGDGSIIAQIKPNQSYKFKHQL
 
SLAFQVTQKTQRRWFLDKLVDEIGVGYVRDRGSVSDYILSEIKPLHNFLTQLQPFLKL
KQKQANLVLKIIEQLPSAKESPDKFLEVCTWVDQIAALNDSKTRKTTSETVRAVLDS
LSEKKKSSP
SEQ ID NO: 171 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTATGCTGTTATCTATCCTCATCAACGTGCTAAG
TTCAAGTACTTCCTGAAGCTGCTTTTCACGGTCAATCAGAGTACAAAGCGCCGTT
GGTTCCTCGACAAGCTGGTGGACGAGATCGGTGTGGGTTACGTGTATGACGGGC
CGCGTACGTCCGAGTACCATCTGTCCGAGATCAAGCCTTTGCATAATTTTTTAAC
ACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAGGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
TGTATCCGGCCGAGGCAGTGTAGTAAGTTCAAGCACAGGCTGACTCTGGGGTTC
GCGGTCGGGCAGAAGACACAGCGCCGTTGGTTCCTCGACAAGCTGGTGGACGAG
ATCGGTGTGGGTTACGTGTATGACAGAGGCAGCGTCTCCGAGTACGTGCTGTCCC
AGATCAAGCCTCTGCACAACTTCCTGACCCAGCTCCAGCCCTTCCTGAAGCTCAA
GCAGAAGCAGGCCAACCTCGTGCTGAAGATCATCGAGCAGCTGCCCTCCGCCAA
GGAATCCCCGGACAAGTTCCTGGAGGTGTGCACCTGGGTGGACCAGATCGCCGC
TCTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGCGCGCCAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAA
SEQ ID NO: 172 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCCCCCAAGAAGAAGCGCAAGGTGCATATGAACACCAAGTACAACAAGGAG
TTCCTGCTGTACCTGGCCGGCTTCGTGGACGCCGACGGCAGCATCTACGCCGTGA
TCTACCCCCACCAGCGCGCCAAGTTCAAGTACTTCCTGAAGCTGCTGTTCACCGT
GAACCAGAGCACCAAGCGCCGCTGGTTCCTGGACAAGCTGGTGGACGAGATCGG
CGTGGGCTACGTGTACGACGGCCCCCGCACCAGCGAGTACCACCTGAGCGAGAT
 
CAAGCCCCTGCACAACTTCCTGACCCAGCTGCAGCCCTTCCTGAAGCTGAAGCAG
AAGCAGGCCAACCTGGTGCTGAAGATCATCGAGCAGCTGCCCAGCGCCAAGGAG
AGCCCCGACAAGTTCCTGGAGGTGTGCACCTGGGTGGACCAGATCGCCGCCCTG
AACGACAGCCGCACCCGCAAGACCACCAGCGAGACCGTGCGCGCCGTGCTGGAC
AGCCTGCCCGGCAGCGTGGGCGGCCTGAGCCCCAGCCAGGCCAGCAGCGCCGCC
AGCAGCGCCAGCAGCAGCCCCGGCAGCGGCATCAGCGAGGCCCTGCGCGCCGGC
GCCGGCAGCGGCACCGGCTACAACAAGGAGTTCCTGCTGTACCTGGCCGGCTTC
GTGGACGGCGACGGCAGCATCTACGCCTGCATCCGCCCCCGCCAGTGCAGCAAG
TTCAAGCACCGCCTGACCCTGGGCTTCGCCGTGGGCCAGAAGACCCAGCGCCGC
TGGTTCCTGGACAAGCTGGTGGACGAGATCGGCGTGGGCTACGTGTACGACCGC
GGCAGCGTGAGCGAGTACGTGCTGAGCCAGATCAAGCCCCTGCACAACTTCCTG
ACCCAGCTGCAGCCCTTCCTGAAGCTGAAGCAGAAGCAGGCCAACCTGGTGCTG
AAGATCATCGAGCAGCTGCCCAGCGCCAAGGAGAGCCCCGACAAGTTCCTGGAG
GTGTGCACCTGGGTGGACCAGATCGCCGCCCTGAACGACAGCAAGACCCGCAAG
ACCACCAGCGAGACCGTGCGCGCCGTTCTAGACAGCCTGAGCGAGAAGAAGAAA
AGCAGCCCCCCCAAGAAGAAGCGCAAGGTGTGATGAGGTACCAGCGGCCGCACT
CATCTTGGCCCTCCTCAGCTCCCTGCCTGTTTCCCGTAAGGCTGTACATAGTCCTT
TTATCTCCTTGTGGCCTATGAAACTGGTTTATAATAAACTCTTAAGAGAACATTA
GGCGCGCCAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAA
SEQ ID NO: 173 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCCCGAAGAAGAAGCGCAAGGTGTAATGAGGTACCAGCGGCCGCACCAGCCT
CAAGAACACCCGAATGGAGTCTCTAAGCTACATAATACCAACTTACACTTTACAA
AATGTTGTCCCCCAAAATGTAGCCATTCGTATCTGCTCCTAATAAAAAGAAAGTT
TCTTCACATTCTGGCGCGCCAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAA
SEQ ID NO: 174 
TAATACGACTCACTATAAGGGCATAAACCCTGGCGCGCTCGCGGGCCGGCACTC
TTCTGGTCCCCACAGACTCAGAGAGAACCCACCATGGCACCGAAGAAGAAGCGC
AAGGTGCATATGAACACCAAGTACAACAAGGAGTTCCTGCTCTACCTGGCGGGC
TTCGTCGACTCCGACGGCTCCATCTACGCCGCCATCCGCCCGAGCCAGACCGCCA
AGTTCAAGCATCGGCTGCAGCTGTTCTTCGCCGTCTACCAGAAGACACAGCGCCG
TTGGTTCCTCGACAAGCTGGTGGACGAGATCGGGGTGGGCTACGTGACCGACGC
CGGCAGCGTCAGCAGCTACTTCCTGTCCGAGATCAAGCCTCTGCACAACTTCCTG
ACCCAGCTCCAGCCCTTCCTGAAGCTCAAGCAGAAGCAGGCCAACCTCGTGCTG
AAGATCATCGAGCAGCTGCCCTCCGCCAAGGAATCCCCGGACAAGTTCCTGGAG
GTGTGCACGTGGGTCGACCAGATCGCGGCCCTCAACGACAGCAAGACCCGCAAG
ACGACCTCGGAAACGGTGCGGGCGGTCCTGGACTCCCTCCCAGGATCCGTGGGA
GGTCTATCGCCATCTCAGGCATCCAGCGCCGCATCCTCGGCTTCCTCAAGCCCGG
GTTCAGGGATCTCCGAAGCACTCAGAGCTGGAGCAGGTTCCGGCACTGGATACA
ACAAGGAATTCCTGCTCTACCTGGCGGGCTTCGTCGACGGGGACGGCTCCATCTT
CGCCTCCATCCACCCGCAGCAGCGCAACAAGTTCAAGCATCAGCTGAGCCTCCA
CTTCACCGTCAGGCAGAAGACACAGCGCCGTTGGTTCCTCGACAAGCTGGTGGA
CGAGATCGGGGTGGGCTACGTGATCGACGAGGGCAGCGTCAGCAGCTACCGCCT
GTCCAAGATCAAGCCTCTGCACAACTTCCTGACCCAGCTCCAGCCCTTCCTGAAG
CTCAAGCAGAAGCAGGCCAACCTCGTGCTGAAGATCATCGAGCAGCTGCCCTCC
GCCAAGGAATCCCCGGACAAGTTCCTGGAGGTGTGCACCTGGGTGGACCAGATC
GCCGCTCTGAACGACTCCAAGACCCGCAAGACCACTTCCGAAACCGTCCGCGCC
GTTCTAGACAGTCTCTCCGAGAAGAAGAAGTCGTCCCCCGACTACAAGGACGAC
GACGACAAGTAATGAGGTACCAGCGGCCGCATCAACCTCTGGATTACAAAATTT
GTGAAAGATTGACTGATATTCTTAACTATGTTGCTCCTTTTACGCTGTGTGGATAT
GCTGCTTTAATGCCTCTGTATCATGCTATTGCTTCCCGTACGGCTTTCGTTTTCTCC
TCCTTGTATAAATCCTGGTTGCTGTCTCTTTATGAGGAGTTGTGGCCCGTTGTCCG
TCAACGTGGCGTGGTGTGCTCTGTGTTTGCTGACGCAACCCCCACTGGCTGGGGC
ATTGCCACCACCTGTCAACTCCTTTCTGGGACTTTCGCTTTCCCCCTCCCGATCGC
CACGGCAGAACTCATCGCCGCCTGCCTTGCCCGCTGCTGGACAGGGGCTAGGTTG
CTGGGCACTGATAATTCCGTGGTGTTGTCGGGGAAGCTGACGTCCTTTCCAGGGC
TGCTCGCCTGTGTTGCCAACTGGATCCTGCGCGGGACGTCCTTCTGCTACGTCCCT
TCGGCTCTCAATCCAGCGGACCTCCCTTCCCGAGGCCTTCTGCCGGTTCTGCGGC
CTCTCCCGCGTCTTCGCTTTCGGCCTCCGACGAGTCGGATCTCCCTTTGGGCCGCC
TCCCCGCCTGGCGCGCCAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
A
SEQ ID NO: 175 
TAATACGACTCACTATAAGGGAAGCTCAGAATAAACGCTCAACTTTGGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
 
AACGACAGCAAGACCCGCAAGACGACCTCGGAAACGGTGCGGGCGGTCCTGGA
CTCCCTCCCAGGATCCGTGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCCCGAAGAAGAAGCGCAAGGTGTAATGAGGTACCAGCGGCCGCACCAGCCT
CAAGAACACCCGAATGGAGTCTCTAAGCTACATAATACCAACTTACACTTTACAA
AATGTTGTCCCCCAAAATGTAGCCATTCGTATCTGCTCCTAATAAAAAGAAAGTT
TCTTCACATTCTGGCGCGCCAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAA
SEQ ID NO: 176 
TAATACGACTCACTATAAGGGAAGCTCAGAATAAACGCTCAACTTTGGCCACCA
TGGCACCGGCCGCTAAGCGCGTGAAGCTGGACCATATGAACACCAAGTACAACA
AGGAGTTCCTGCTCTACCTGGCGGGCTTCGTCGACTCCGACGGCTCCATCTACGC
CGCCATCCGCCCGAGCCAGACCGCCAAGTTCAAGCATCGGCTGCAGCTGTTCTTC
GCCGTCTACCAGAAGACACAGCGCCGTTGGTTCCTCGACAAGCTGGTGGACGAG
ATCGGGGTGGGCTACGTGACCGACGCCGGCAGCGTCAGCAGCTACTTCCTGTCC
GAGATCAAGCCTCTGCACAACTTCCTGACCCAGCTCCAGCCCTTCCTGAAGCTCA
AGCAGAAGCAGGCCAACCTCGTGCTGAAGATCATCGAGCAGCTGCCCTCCGCCA
AGGAATCCCCGGACAAGTTCCTGGAGGTGTGCACGTGGGTCGACCAGATCGCGG
CCCTCAACGACAGCAAGACCCGCAAGACGACCTCGGAAACGGTGCGGGCGGTCC
TGGACTCCCTCCCAGGATCCGTGGGAGGTCTATCGCCATCTCAGGCATCCAGCGC
CGCATCCTCGGCTTCCTCAAGCCCGGGTTCAGGGATCTCCGAAGCACTCAGAGCT
GGAGCAGGTTCCGGCACTGGATACAACAAGGAATTCCTGCTCTACCTGGCGGGC
TTCGTCGACGGGGACGGCTCCATCTTCGCCTCCATCCACCCGCAGCAGCGCAACA
AGTTCAAGCATCAGCTGAGCCTCCACTTCACCGTCAGGCAGAAGACACAGCGCC
GTTGGTTCCTCGACAAGCTGGTGGACGAGATCGGGGTGGGCTACGTGATCGACG
AGGGCAGCGTCAGCAGCTACCGCCTGTCCAAGATCAAGCCTCTGCACAACTTCCT
GACCCAGCTCCAGCCCTTCCTGAAGCTCAAGCAGAAGCAGGCCAACCTCGTGCT
GAAGATCATCGAGCAGCTGCCCTCCGCCAAGGAATCCCCGGACAAGTTCCTGGA
GGTGTGCACCTGGGTGGACCAGATCGCCGCTCTGAACGACTCCAAGACCCGCAA
GACCACTTCCGAAACCGTCCGCGCCGTTCTAGACAGTCTCTCCGAGAAGAAGAA
GTCGTCCCCCCCGGCCGCTAAGCGCGTGAAGCTGGACTAATGAGGTACCAGCGG
CCGCACCAGCCTCAAGAACACCCGAATGGAGTCTCTAAGCTACATAATACCAAC
TTACACTTTACAAAATGTTGTCCCCCAAAATGTAGCCATTCGTATCTGCTCCTAAT
AAAAAGAAAGTTTCTTCACATTCTGGCGCGCCAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAA
SEQ ID NO: 177 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCACCGAAGAAGAAGCGCAAGGTGCATATGAACACCAAGTACAACAAGGAG
TTCCTGCTCTACCTGGCGGGCTTCGTCGACTCCGACGGCTCCATCTACGCCGCCA
TCCGCCCGAGCCAGACCGCCAAGTTCAAGCATCGGCTGCAGCTGTTCTTCGCCGT
CTACCAGAAGACACAGCGCCGTTGGTTCCTCGACAAGCTGGTGGACGAGATCGG
GGTGGGCTACGTGACCGACGCCGGCAGCGTCAGCAGCTACTTCCTGTCCGAGAT
CAAGCCTCTGCACAACTTCCTGACCCAGCTCCAGCCCTTCCTGAAGCTCAAGCAG
AAGCAGGCCAACCTCGTGCTGAAGATCATCGAGCAGCTGCCCTCCGCCAAGGAA
TCCCCGGACAAGTTCCTGGAGGTGTGCACGTGGGTCGACCAGATCGCGGCCCTC
AACGACAGCAAGACCCGCAAGACGACCTCGGAAACGGTGCGGGCGGTCCTGGA
CTCCCTCCCAGGATCCGTGGGAGGTCTATCGCCATCTCAGGCATCCAGCGCCGCA
TCCTCGGCTTCCTCAAGCCCGGGTTCAGGGATCTCCGAAGCACTCAGAGCTGGAG
CAGGTTCCGGCACTGGATACAACAAGGAATTCCTGCTCTACCTGGCGGGCTTCGT
CGACGGGGACGGCTCCATCTTCGCCTCCATCCACCCGCAGCAGCGCAACAAGTTC
AAGCATCAGCTGAGCCTCCACTTCACCGTCAGGCAGAAGACACAGCGCCGTTGG
TTCCTCGACAAGCTGGTGGACGAGATCGGGGTGGGCTACGTGATCGACGAGGGC
AGCGTCAGCAGCTACCGCCTGTCCAAGATCAAGCCTCTGCACAACTTCCTGACCC
AGCTCCAGCCCTTCCTGAAGCTCAAGCAGAAGCAGGCCAACCTCGTGCTGAAGA
TCATCGAGCAGCTGCCCTCCGCCAAGGAATCCCCGGACAAGTTCCTGGAGGTGT
GCACCTGGGTGGACCAGATCGCCGCTCTGAACGACTCCAAGACCCGCAAGACCA
CTTCCGAAACCGTCCGCGCCGTTCTAGACAGTCTCTCCGAGAAGAAGAAGTCGTC
CCCCCCGAAGAAGAAGCGCAAGGTGTAATGAGGTACCAGCGGCCGCACTCATCT
TGGCCCTCCTCAGCTCCCTGCCTGTTTCCCGTAAGGCTGTACATAGTCCTTTTATC
TCCTTGTGGCCTATGAAACTGGTTTATAATAAACTCTTAAGAGAACATTAGGCGC
GCCAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAA
SEQ ID NO: 178 
TAATACGACTCACTATAAGGGGACTCACTATTTGTTTTCGCGCCCAGTTGCAAAA
AGTGTCGCCGCATCTAGAGGGCCAATTATTGGTTAAAGAAGTATATTAGTGCTAA
TTTCCCTCCGTTTGTCCTAGCTTTTCTCTTCTGTCAACCCCACACGCCTTTGCCACC
ATGGCACCGAAGAAGAAGCGCAAGGTGCATATGAACACCAAGTACAACAAGGA
GTTCCTGCTCTACCTGGCGGGCTTCGTCGACTCCGACGGCTCCATCTACGCCGCC
ATCCGCCCGAGCCAGACCGCCAAGTTCAAGCATCGGCTGCAGCTGTTCTTCGCCG
TCTACCAGAAGACACAGCGCCGTTGGTTCCTCGACAAGCTGGTGGACGAGATCG
GGGTGGGCTACGTGACCGACGCCGGCAGCGTCAGCAGCTACTTCCTGTCCGAGA
TCAAGCCTCTGCACAACTTCCTGACCCAGCTCCAGCCCTTCCTGAAGCTCAAGCA
GAAGCAGGCCAACCTCGTGCTGAAGATCATCGAGCAGCTGCCCTCCGCCAAGGA
ATCCCCGGACAAGTTCCTGGAGGTGTGCACGTGGGTCGACCAGATCGCGGCCCT
CAACGACAGCAAGACCCGCAAGACGACCTCGGAAACGGTGCGGGCGGTCCTGG
ACTCCCTCCCAGGATCCGTGGGAGGTCTATCGCCATCTCAGGCATCCAGCGCCGC
ATCCTCGGCTTCCTCAAGCCCGGGTTCAGGGATCTCCGAAGCACTCAGAGCTGGA
GCAGGTTCCGGCACTGGATACAACAAGGAATTCCTGCTCTACCTGGCGGGCTTCG
TCGACGGGGACGGCTCCATCTTCGCCTCCATCCACCCGCAGCAGCGCAACAAGTT
CAAGCATCAGCTGAGCCTCCACTTCACCGTCAGGCAGAAGACACAGCGCCGTTG
GTTCCTCGACAAGCTGGTGGACGAGATCGGGGTGGGCTACGTGATCGACGAGGG
CAGCGTCAGCAGCTACCGCCTGTCCAAGATCAAGCCTCTGCACAACTTCCTGACC
 
CAGCTCCAGCCCTTCCTGAAGCTCAAGCAGAAGCAGGCCAACCTCGTGCTGAAG
ATCATCGAGCAGCTGCCCTCCGCCAAGGAATCCCCGGACAAGTTCCTGGAGGTG
TGCACCTGGGTGGACCAGATCGCCGCTCTGAACGACTCCAAGACCCGCAAGACC
ACTTCCGAAACCGTCCGCGCCGTTCTAGACAGTCTCTCCGAGAAGAAGAAGTCGT
CCCCCCCGAAGAAGAAGCGCAAGGTGTAATGAGGTACCAGCGGCCGCACTCATC
TTGGCCCTCCTCAGCTCCCTGCCTGTTTCCCGTAAGGCTGTACATAGTCCTTTTAT
CTCCTTGTGGCCTATGAAACTGGTTTATAATAAACTCTTAAGAGAACATTAGGCG
CGCCAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAA
SEQ ID NO: 179 
TAATACGACTCACTATAAGGGAAGCTCAGAATAAACGCTCAACTTTGGCCACCA
TGGCCCCCGCCGCCAAGCGCGTGAAGCTGGACCACATGAACACCAAGTACAACA
AGGAGTTCCTGCTGTACCTGGCCGGCTTCGTGGACAGCGACGGCAGCATCTACGC
CGCCATCCGCCCCAGCCAGACCGCCAAGTTCAAGCACCGCCTGCAGCTGTTCTTC
GCCGTGTACCAGAAGACCCAGCGCCGCTGGTTCCTGGACAAGCTGGTGGACGAG
ATCGGCGTGGGCTACGTGACCGACGCCGGCAGCGTGAGCAGCTACTTCCTGAGC
GAGATCAAGCCCCTGCACAACTTCCTGACCCAGCTGCAGCCCTTCCTGAAGCTGA
AGCAGAAGCAGGCCAACCTGGTGCTGAAGATCATCGAGCAGCTGCCCAGCGCCA
AGGAGAGCCCCGACAAGTTCCTGGAGGTGTGCACCTGGGTGGACCAGATCGCCG
CCCTGAACGACAGCAAGACCCGCAAGACCACCAGCGAGACCGTGCGCGCCGTGC
TGGACAGCCTGCCCGGCAGCGTGGGCGGCCTGAGCCCCAGCCAGGCCAGCAGCG
CCGCCAGCAGCGCCAGCAGCAGCCCCGGCAGCGGCATCAGCGAGGCCCTGCGCG
CCGGCGCCGGCAGCGGCACCGGCTACAACAAGGAGTTCCTGCTGTACCTGGCCG
GCTTCGTGGACGGCGACGGCAGCATCTTCGCCAGCATCACCCCCAGCAGCGCA
ACAAGTTCAAGCACCAGCTGAGCCTGCACTTCACCGTGCGCCAGAAGACCCAGC
GCCGCTGGTTCCTGGACAAGCTGGTGGACGAGATCGGCGTGGGCTACGTGATCG
ACGAGGGCAGCGTGAGCAGCTACCGCCTGAGCAAGATCAAGCCCCTGCACAACT
TCCTGACCCAGCTGCAGCCCTTCCTGAAGCTGAAGCAGAAGCAGGCCAACCTGG
TGCTGAAGATCATCGAGCAGCTGCCCAGCGCCAAGGAGAGCCCCGACAAGTTCC
TGGAGGTGTGCACCTGGGTGGACCAGATCGCCGCCCTGAACGACAGCAAGACCC
GCAAGACCACCAGCGAGACCGTGCGCGCCGTGCTGGACAGCCTGAGCGAGAAG
AAGAAGTCCAGCCCCCCGCCGCCAAGCGCGTGAAGCTGGACTGATGAGGTACC
AGCGGCCGCACCAGCCTCAAGAACACCCGAATGGAGTCTCTAAGCTACATAATA
CCAACTTACACTTTACAAAATGTTGTCCCCCAAAATGTAGCCATTCGTATCTGCT
CCTAATAAAAAGAAAGTTTCTTCACATTCTGGCGCGCCAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAA
SEQ ID NO: 180 
TAATACGACTCACTATAAGGGAAGCTCAGAATAAACGCTCAACTTTGGCCACCA
TGGCCCCCGCCGCCAAGCGCGTGAAGCTGGACCACATGAACACCAAGTACAACA
AGGAGTTCCTGCTGTACCTGGCCGGCTTCGTGGACAGCGACGGCAGCATCAACG
CCAGCATCAGCCCCCGCCAGAGCTTCAAGTTCAAGCACGGCCTGAAGCTGCGCTT
CGAGGTGGGCCAGAAGACCCAGCACCGCTGGTTCCTGGACAAGCTGGTGGACGA
GATCGGCGTGGGCTACGTGTACGACAACGGCAGCGTGAGCGTGTACAGCCTGAG
CCAGATCAAGCCCCTGCACAACTTCCTGACCCAGCTGCAGCCCTTCCTGAAGCTG
AAGCAGAAGCAGGCCAACCTGGTGCTGAAGATCATCGAGCAGCTGCCCAGCGCC
 
AAGGAGAGCCCCGACAAGTTCCTGGAGGTGTGCACCTGGGTGGACCAGATCGCC
GCCCTGAACGACAGCAAGACCCGCAAGACCACCAGCGAGACCGTGCGCGCCGTG
CTGGACAGCCTGCCCGGCAGCGTGGGCGGCCTGAGCCCCAGCCAGGCCAGCAGC
GCCGCCAGCAGCGCCAGCAGCAGCCCCGGCAGCGGCATCAGCGAGGCCCTGCGC
GCCGGCGCCGGCAGCGGCACCGGCTACAACAAGGAGTTCCTGCTGTACCTGGCC
GGCTTCGTGGACGGCGACGGCAGCATCTTCGCCAGCATCCGCCCCCGCCAGCAC
GCCAAGTTCAAGCACGACCTGGAGCTGTGCTTCAACGTGCGCCAGAAGACCCAG
CGCCGCTGGTTCCTGGACAAGCTGGTGGACGAGATCGGCGTGGGCTACGTGATC
GACTGGCGCGGCGCCAGCACCTACAAGCTGAGCCAGATCAAGCCCCTGCACAAC
TTCCTGACCCAGCTGCAGCCCTTCCTGAAGCTGAAGCAGAAGCAGGCCAACCTG
GTGCTGAAGATCATCGAGCAGCTGCCCAGCGCCAAGGAGAGCCCCGACAAGTTC
CTGGAGGTGTGCACCTGGGTGGACCAGATCGCCGCCCTGAACGACAGCAAGACC
CGCAAGACCACCAGCGAGACCGTGCGCGCCGTGCTGGACAGCCTGAGCGAGAAG
AAGAAGTCCAGCCCCCCCGCCGCCAAGCGCGTGAAGCTGGACTAATGAGGTACC
AGCGGCCGCACCAGCCTCAAGAACACCCGAATGGAGTCTCTAAGCTACATAATA
CCAACTTACACTTTACAAAATGTTGTCCCCCAAAATGTAGCCATTCGTATCTGCT
CCTAATAAAAAGAAAGTTTCTTCACATTCTGGCGCGCCAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAA
SEQ ID NO: 181 
TAATACGACTCACTATAAGGGCATAAACCCTGGCGCTCGCGGCCGGCACTC
TTCTGGTCCCCACAGACTCAGAGAGAACCCACCATGGCACCGAAGAAGCGC
AAGGTGCATATGAATACAAAATATAATAAAGAGTTCTTACTCTACTTAGCAGGGT
TTGTAGACTCCGACGGTTCCATCTATGCAGCGATCAGGCCCAGTCAGACAGCTAA
GTTCAAGCACCGGCTGCAGCTCTTTTTGCGGTCTATCAAAAGACGCAGCGCCGT
TGGTTCCTCGACAAGCTGGTGGACGAGATCGGTGTGGGTTACGTGACTGACGCTG
GCAGCGTCTCCAGTTACTTTCTGTCCGAGATCAAGCCTTTGCATAATTTTTAACA
CAACTACAACCTTTTCTAAAACTAAAACAAAAACAAGCAAATTTAGTTTTAAAA
ATTATTGAACAACTTCCGTCAGCAAAAGAATCCCCGGACAAATTCTTAGAAGTTT
GTACATGGGTGGATCAAATTGCAGCTCTGAATGATTCGAAGACGCGTAAAACAA
CTTCTGAAACCGTTCGTGCTGTGCTAGACAGTTTACCAGGATCCGTGGGAGGTCT
ATCGCCATCTCAGGCATCCAGCGCCGCATCCTCGGCTTCCTCAAGCCCGGGTTCA
GGGATCTCCGAAGCACTCAGAGCTGGAGCAGGTTCCGGCACTGGATACAACAAG
GAATTCCTGCTCTACCTGGCGGGCTTCGTCGACGGGGACGGCTCCATCTTTGCCA
GTATCCATCCTCAGCAACGTAATAAGTTCAAGCACCAGCTGTCTCTCCATTTCAC
GGTCCGTCAGAAGACACAGCGCCGTTGGTTCCTTGACAAGCTGGTGGACGAGAT
CGGTGTGGGTTACGTGATTGACGAGGGCAGCGTCTCCAGTTATCGGTTAAGCAA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
 
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAA
SEQ ID NO: 182 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACTCTGACGGTTCCATCAACGCCAGCATCTCGCCGCGGCAGTCGTTCAA
GTTCAAGCACGGGCTGAAGCTCCGGTTCGAGGTCGGTCAGAAGACACAGCACCG
TTGGTTCCTCGACAAGCTGGTGGACGAGATCGGTGTGGGTTACGTGTATGACAAT
GGCAGCGTCTCCGTTTACTCTCTGTCCCAGATCAAGCCTTTGCATAATTTTTAAC
ACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAGGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTTTGCA
TCGATCCGGCCTCGTCAACATGCTAAGTTCAAGCACGATCTGGAGCTCTGTTTCA
ATGTCAGGCAGAAGACACAGCGCCGTTGGTTCCTCGACAAGCTGGTGGACGAGA
TCGGTGTGGGTTACGTGATTGACTGGCGTGGCGCCTCCACTTACAAGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAA
SEQ ID NO: 183 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
 
AAGGTGCATATGAATACAAAATATAATAAAGAGTTCTTACTCTACTTAGCAGGGT
TTGTAGACGCTGACGGTTCCATCTGGGCCCATATCGAGCCTTGCCAGTGGGTGAA
GTTCAAGCACAGGCTGAGGCTCTCTCTCAATGTCACTCAGAAGACACAGCGCCGT
TGGTTCCTCGACAAGCTGGTGGACGAGATCGGTGTGGGTTACGTGCGTGACACG
GGCAGCGTCTCCCAGTACCATCTGTCCGAGATCAAGCCTTTGCATAATTTTTTAA
CACAACTACAACCTTTTCTAAAACTAAAACAAAAACAAGCAAATTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAA
SEQ ID NO: 184 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCCCCCAAGAAGAAGCGCAAGGTGCATATGAACACCAAGTACAACAAGGAG
TTCCTGCTGTACCTGGCCGGCTTCGTGGACGCCGACGGCAGCATCTGGGCCCACA
TCGAGCCCTGCCAGTGGGTGAAGTTCAAGCACCGCCTGCGCCTGAGCCTGAACG
TGACCCAGAAGACCCAGCGCCGCTGGTTCCTGGACAAGCTGGTGGACGAGATCG
GCGTGGGCTACGTGCGCGACACCGGCAGCGTGAGCCAGTACCACCTGAGCGAGA
TCAAGCCCCTGCACAACTTCCTGACCCAGCTGCAGCCCTTCCTGAAGCTGAAGCA
GAAGCAGGCCAACCTGGTGCTGAAGATCATCGAGCAGCTGCCCAGCGCCAAGGA
GAGCCCCGACAAGTTCCTGGAGGTGTGCACCTGGGTGGACCAGATCGCCGCCCT
GAACGACAGCAAGACCCGCAAGACCACCAGCGAGACCGTGCGCGCCGTGCTGG
ACAGCCTGCCCGGCAGCGTGGGCGGCCTGAGCCCCAGCCAGGCCAGCAGCGCCG
CCAGCAGCGCCAGCAGCAGCCCCGGCAGCGGCATCAGCGAGGCCCTGCGCGCCG
GCGCCGGCAGCGGCACCGGCTACAACAAGGAGTTCCTGCTGTACCTGGCCGGCT
 
TCGTGGACGGCGACGGCAGCATCTACGCCAAGATCCGCCCCCAGCAGGCCAGCA
AGTTCAAGCACGTGCTGGAGCTGGTGTTCGAGGTGACCCAGAGCACCCAGCGCC
GCTGGTTCCTGGACAAGCTGGTGGACGAGATCGGCGTGGGCTACGTGTACGACT
GGAAGCAGGCCAGCATGTACCGCCTGAGCCAGATCAAGCCCCTGCACAACTTCC
TGACCCAGCTGCAGCCCTTCCTGAAGCTGAAGCAGAAGCAGGCCAACCTGGTGC
TGAAGATCATCGAGCAGCTGCCCAGCGCCAAGGAGAGCCCCGACAAGTTCCTGG
AGGTGTGCACCTGGGTGGACCAGATCGCCGCCCTGAACGACAGCAAGACCCGCA
AGACCACCAGCGAGACCGTGCGCGCCGTTCTAGACAGCCTGAGCGAGAAGAAGA
AAAGCAGCCCCCCCAAGAAGAAGCGCAAGGTGTAATAAGGTACCAGCGGCCGC
ACTCATCTTGGCCCTCCTCAGCTCCCTGCCTGTTTCCCGTAAGGCTGTACATAGTC
CTTTTATCTCCTTGTGGCCTATGAAACTGGTTTATAATAAACTCTTAAGAGAACAT
TAGGCGCGCCAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA
SEQ ID NO: 185 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTGGGCCTATATCGAGCCTTGCCAGTGGGTGAA
GTTCAAGCACAGGCTGAAGCTCCAGCTCAATGTCACTCAGAAGACACAGCGCCG
TTGGTTCCTCGACAAGCTGGTGGACGAGATCGGTGTGGGTTACGTGCGTGACACG
GGCAGCGTCTCCCAGTACATGCTGTCCGAGATCAAGCCTTTGCATAATTTTTTAA
CACAACTACAACCTTTTCTAAAACTAAAACAAAAACAAGCAAATTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AAGATCCGTCCTCAGCAAGCTTCTAAGTTCAAGCACGTTCTGGAGCTCGTGTTCG
AGGTCACTCAGTCGACACAGCGCCGTTGGTTCCTCGACAAGCTGGTGGACGAGA
TCGGTGTGGGTTACGTGTATGACTGGAAGCAGGCCTCCATGTACCGGCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
 
AAAAAAAAAAAAAAAAAAAAAAAAAAAAAAAAAAAAAAAAAAAAAAAAAAAAAAAAAAA
AAAAAAAAAAAAAAAAAAAAAAAAAA
SEQ ID NO: 186 
TAATACGACTCACTATAAGGGAATTATTGGTTAAAGAAGTATATTAGTGCTAATT
TCCCTCCGTTTGTCCTAGCTTTTCTCTTCTGTCAACCCCACACGCCTTTGCCACCA
TGGCCCCCAAGAAGAAGCGCAAGGTGCATATGAACACCAAGTACAACAAGGAG
TTCCTGCTGTACCTGGCCGGCTTCGTGGACGCCGACGGCAGCATCTGGGCCTACA
TCGAGCCCTGCCAGTGGGTGAAGTTCAAGCACCGCCTGAAGCTGCAGCTGAACG
TGACCCAGAAGACCCAGCGCCGCTGGTTCCTGGACAAGCTGGTGGACGAGATCG
GCGTGGGCTACGTGCGCGACACCGGCAGCGTGAGCCAGTACATGCTGAGCGAGA
TCAAGCCCCTGCACAACTTCCTGACCCAGCTGCAGCCCTTCCTGAAGCTGAAGCA
GAAGCAGGCCAACCTGGTGCTGAAGATCATCGAGCAGCTGCCCAGCGCCAAGGA
GAGCCCCGACAAGTTCCTGGAGGTGTGCACCTGGGTGGACCAGATCGCCGCCCT
GAACGACAGCAAGACCCGCAAGACCACCAGCGAGACCGTGCGCGCCGTGCTGG
ACAGCCTGCCCGGCAGCGTGGGCGGCCTGAGCCCCAGCCAGGCCAGCAGCGCCG
CCAGCAGCGCCAGCAGCAGCCCCGGCAGCGGCATCAGCGAGGCCCTGCGCGCCG
GCGCCGGCAGCGGCACCGGCTACAACAAGGAGTTCCTGCTGTACCTGGCCGGCT
TCGTGGACGGCGACGGCAGCATCTACGCCAAGATCCGCCCCCAGCAGGCCAGCA
AGTTCAAGCACGTGCTGGAGCTGGTGTTCGAGGTGACCCAGAGCACCCAGCGCC
GCTGGTTCCTGGACAAGCTGGTGGACGAGATCGGCGTGGGCTACGTGTACGACT
GGAAGCAGGCCAGCATGTACCGCCTGAGCCAGATCAAGCCCCTGCACAACTTCC
TGACCCAGCTGCAGCCCTTCCTGAAGCTGAAGCAGAAGCAGGCCAACCTGGTGC
TGAAGATCATCGAGCAGCTGCCCAGCGCCAAGGAGAGCCCCGACAAGTTCCTGG
AGGTGTGCACCTGGGTGGACCAGATCGCCGCCCTGAACGACAGCAAGACCCGCA
AGACCACCAGCGAGACCGTGCGCGCCGTTCTAGACAGCCTGAGCGAGAAGAAGA
AAAGCAGCCCCCCCAAGAAGAAGCGCAAGGTGTGATAAGGTACCAGCGGCCGC
ACTCATCTTGGCCCTCCTCAGCTCCCTGCCTGTTTCCCGTAAGGCTGTACATAGTC
CTTTTATCTCCTTGTGGCCTATGAAACTGGTTTATAATAAACTCTTAAGAGAACAT
TAGGCGCGCCAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAA
SEQ ID NO: 187 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTATGCCACGATCCGGCCTGTTCAAAGGGCTAA
GTTCAAGCACTCGCTGCGTCTCTTTTTCAATGTCAGTCAGAAGACACAGCGCCGT
TGGTTCCTCGACAAGCTGGTGGACGAGATCGGTGTGGGTTACGTGCTGGACAAG
GGCAGCGTCTCCTATTACATTCTGTCCCAGATCAAGCCTTTGCATAATTTTTTAAC
ACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAAGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGATGGGGACGGCTCCATCTTTGCC
CAGATCCGGCCTAGGCAAGGGCATAAGTTCAAGCACGGCCTGGAGCTCTCGTTC
GAGGTCACTCAGCATACAAAGCGCCGTTGGTTCCTCGACAAGCTGGTGGACGAG
 
ATCGGTGTGGGTTACGTGTATGACTGCGGCCCGGCCTGCAGCTACCGGCTGTCCC
AGATCAAGCCTCTGCACAACTTCCTGACCCAGCTCCAGCCCTTCCTGAAGCTCAA
GCAGAAGCAGGCCAACCTCGTGCTGAAGATCATCGAGCAGCTGCCCTCCGCCAA
GGAATCCCCGGACAAGTTCCTGGAGGTGTGCACCTGGGTGGACCAGATCGCCGC
TCTGAACGACTCCAGGACCCGCAAGACCACTTCCGAAACCGTCCGCGCCGTTCTA
GACAGTCTCTCCGAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAA
AAAAAAAAAAAAAAAAAAAAAAAA
SEQ ID NO: 188 
TAATACGACTCACTATAAGGGCATAAACCCTGGCGCGCTCGCGGCCGGCACTC
TTCTGGTCCCCACAGACTCAGAGAGAACCCACCATGGCACCGAAGAAGAAGCGC
AAGGTGCATATGAATACAAAATATAATAAAGAGTTCTTACTCTACTTAGCAGGGT
TTGTAGACGCTGACGGTTCCATCTATGCCTGTATCACGCCTCGTCAAACTCATAA
GTTCAAGCACGTTCTGGCGCTCGGGTTCTCAGTCATTCAGCGTACACGTCGCCGT
TGGTTCCTCGACAAGCTGGTGGACGAGATCGGTGTGGGTTACGTGCGTGACAGG
GATACGACCAGCGAATACAGACTGTCCCAGATCAAGCCTTTGCATAATTTTTAA
CACAACTACAACCTTTTCTAAAACTAAAACAAAAACAAGCAAATTTAGTTTTAAA
AATTATTGAACAACTTCCGTCAGCAAAAGAATCCCCGGACAAATTCTTAGAAGTT
TGTACATGGGTGGATCAAATTGCAGCTCTGAATGATTCGAGGACGCGTAAAACA
ACTTCTGAAACCGTTCGTGCTGTGCTAGACAGTTTACCAGGATCCGTGGGAGGTC
TATCGCCATCTCAGGCATCCAGCGCCGCATCCTCGGCTTCCTCAAGCCCGGGTTC
AGGGATCTCCGAAGCACTCAGAGCTGGAGCAGGTTCCGGCACTGGATACAACAA
GGAATTCCTGCTCTACCTGGCGGGCTTCGTCGACGGGGACGGCTCCATCTATGCC
AGTATCGATCCTGATCAACGGAGTAAGTTCAAGCACGGGCTGAGGCTCAATTTCC
AGGTCTCTCAGAAGACACAGCGCCGTTGGTTCCTCGACAAGCTGGTGGACGAGA
TCGGTGTGGGTTACGTGCAGGACAAGGGCAGCGTCTCCCATTACATTCTGTCCCA
GATCAAGCCTCTGCACAACTTCCTGACCCAGCTCCAGCCCTTCCTGAAGCTCAAG
CAGAAGCAGGCCAACCTCGTGCTGAAGATCATCGAGCAGCTGCCCTCCGCCAAG
GAATCCCCGGACAAGTTCCTGGAGGTGTGCACCTGGGTGGACCAGATCGCCGCT
CTGAACGACTCCAAGACCCGCAAGACCACTTCCGAAACCGTCCGCGCCGTTCTA
GACAGTCTCTCCGAGAAGAAGAAGTCGTCCCCCTAAGGTACCAGCGGCCGCATC
AACCTCTGGATTACAAAATTTGTGAAAGATTGACTGATATTCTTAACTATGTTGC
TCCTTTTACGCTGTGTGGATATGCTGCTTTAATGCCTCTGTATCATGCTATTGCTT
CCCGTACGGCTTTCGTTTTCTCCTCCTTGTATAAATCCTGGTTGCTGTCTCTTTATG
AGGAGTTGTGGCCCGTTGTCCGTCAACGTGGCGTGGTGTGCTCTGTGTTTGCTGA
CGCAACCCCCACTGGCTGGGGCATTGCCACCACCTGTCAACTCCTTTCTGGGACT
TTCGCTTTCCCCCTCCCGATCGCCACGGCAGAACTCATCGCCGCCTGCCTTGCCC
 
GCTGCTGGACAGGGGCTAGGTTGCTGGGCACTGATAATTCCGTGGTGTTGTCGGG
GAAGCTGACGTCCTTTCCAGGGCTGCTCGCCTGTGTTGCCAACTGGATCCTGCGC
GGGACGTCCTTCTGCTACGTCCCTTCGGCTCTCAATCCAGCGGACCTCCCTTCCCG
AGGCCTTCTGCCGGTTCTGCGGCCTCTCCCGCGTCTTCGCTTTCGGCCTCCGACGA
GTCGGATCTCCCTTTGGGCCGCCTCCCCGCCTGGGCGCGCCAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAA
SEQ ID NO: 189

TAATACGACTCACTATAAGGGAAGCTCAGAATAAACGCTCAACTTTGGCCACCA
TGGCCCCGGCCGCTAAGCGCGTGAAGCTGGACCACATGAACACCAAGTACAACA
AGGAGTTCCTGCTGTACCTGGCCGGCTTCGTGGACGCCGACGGCAGCATCTACGC
CTGCATCACCCCCCGCCAGACCCACAAGTTCAAGCACGTGCTGGCCCTGGGCTTC
AGCGTGATCCAGCGCACCCGCCGCCGCTGGTTCCTGGACAAGCTGGTGGACGAG
ATCGGCGTGGGCTACGTGCGCGACCGCGACACCACCAGCGAGTACCGCCTGAGC
CAGATCAAGCCCCTGCACAACTTCCTGACCCAGCTGCAGCCCTTCCTGAAGCTGA
AGCAGAAGCAGGCCAACCTGGTGCTGAAGATCATCGAGCAGCTGCCCAGCGCCA
AGGAGAGCCCCGACAAGTTCCTGGAGGTGTGCACCTGGGTGGACCAGATCGCCG
CCCTGAACGACAGCCGCACCCGCAAGACCACCAGCGAGACCGTGCGCGCCGTGC
TGGACAGCCTGCCCGGCAGCGTGGGCGGCCTGAGCCCCAGCCAGGCCAGCAGCG
CCGCCAGCAGCGCCAGCAGCAGCCCCGGCAGCGGCATCAGCGAGGCCCTGCGCG
CCGGCGCCGGCAGCGGCACCGGCTACAACAAGGAGTTCCTGCTGTACCTGGCCG
GCTTCGTGGACGGCGACGGCAGCATCTACGCCAGCATCGACCCCGACCAGCGCA
GCAAGTTCAAGCACGGCCTGCGCCTGAACTTCCAGGTGAGCCAGAAGACCCAGC
GCCGCTGGTTCCTGGACAAGCTGGTGGACGAGATCGGCGTGGGCTACGTGCAGG
ACAAGGGCAGCGTGAGCCACTACATCCTGAGCCAGATCAAGCCCCTGCACAACT
TCCTGACCCAGCTGCAGCCCTTCCTGAAGCTGAAGCAGAAGCAGGCCAACCTGG
TGCTGAAGATCATCGAGCAGCTGCCCAGCGCCAAGGAGAGCCCCGACAAGTTCC
TGGAGGTGTGCACCTGGGTGGACCAGATCGCCGCCCTGAACGACAGCAAGACCC
GCAAGACCACCAGCGAGACCGTGCGCGCCGTGCTGGACAGCCTGAGCGAGAAG
AAGAAGTCCAGCCCCCGGCCGCTAAGCGCGTGAAGCTGGACTAATGAGGTACC
AGCGGCCGCACCAGCCTCAAGAACACCCGAATGGAGTCTCTAAGCTACATAATA
CCAACTTACACTTTACAAAATGTTGTCCCCCAAAATGTAGCCATTCGTATCTGCT
CCTAATAAAAAGAAAGTTTCTTCACATTCTGGCGCGCCAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAA

## Claims

1. A polynucleotide comprising a nucleic acid sequence encoding an engineered meganuclease, wherein said nucleic acid sequence comprises:
(a) a 5' untranslated region (UTR) comprising the nucleic acid sequence set forth in SEQ ID NO: 1;
(b) a coding sequence encoding said engineered meganuclease, wherein said engineered meganuclease comprises a first nuclear localization sequence (NLS) positioned at the N-terminus and a second NLS positioned at the C-terminus, wherein said first NLS and said second NLS each comprise the amino acid sequence set forth in SEQ ID NO: 15;
(c) a 3' UTR comprising the nucleic acid sequence set forth in SEQ ID NO: 10;
(d) a poly A sequence; and
(e) a promoter operably linked to said nucleic acid sequence encoding said engineered meganuclease.

2. The polynucleotide of claim 1, wherein said polynucleotide further comprises a modification to said coding sequence encoding said engineered meganuclease to reduce thymidine content of said coding sequence, wherein said modification does not alter the amino acid sequence of said engineered meganuclease.

3. The polynucleotide of claim 1 or claim 2, wherein said promoter is a T7AG promoter.

4. A messenger RNA (mRNA) encoded by said polynucleotide of any one of claims 1-3.

5. The mRNA of claim 4, wherein said mRNA comprises a 5' cap.

6. The mRNA of claim 5, wherein said 5' cap comprises a 5' methyl guanosine cap.

7. The mRNA of any one of claims 4-6, wherein a uridine present in said mRNA is pseudouridine or 2-thiouridine.

8. The mRNA of any one of claims 4-6, wherein a uridine present in said mRNA is methylated.

9. The mRNA of claim 8, wherein a uridine present in said mRNA is N1-methylpseudouridine, 5-methyluridine, or 2'-O-methyluridine.

10. A recombinant DNA construct comprising said polynucleotide of any one of claims 1-3.

11. A recombinant virus comprising said polynucleotide of any one of claims 1-3.

12. The recombinant virus of claim 11, wherein said recombinant virus is a recombinant adeno-associated virus (AAV).

13. A lipid nanoparticle composition comprising lipid nanoparticles comprising said mRNA of any one of claims 4-9.

14. A pharmaceutical composition comprising a pharmaceutically acceptable carrier and said lipid nanoparticle composition of claim 13.

## Patentansprüche

1. Ein Polynukleotid umfassend eine Nukleinsäuresequenz, die eine künstliche Meganuklease kodiert, wobei jene Nukleinsäuresequenz Folgendes umfasst:
(a) eine 5'-nichttranslatierte Region (UTR) umfassend die in SEQ ID NO: 1 dargestellte Nukleinsäuresequenz;
(b) eine kodierende Sequenz, die jene künstliche Meganuklease kodiert, wobei jene künstliche Meganuklease eine erste Kernlokalisierungssequenz (NLS), die am N-Terminus positioniert ist, und eine zweite NLS, die am C-Terminus positioniert ist, umfasst, wobei jene erste NLS und jene zweite NLS jeweils die in SEQ ID NO: 15 dargestellte Aminosäuresequenz umfassen;
(c) eine 3'-UTR umfassend die in SEQ ID NO: 10 dargestellte Nukleinsäuresequenz;
(d) eine Poly-A-Sequenz; und
(e) einen mit jener Nukleinsäuresequenz, die jene künstliche Meganuklease kodiert, operable verknüpften Promotor.

2. Das Polynukleotid aus Anspruch 1, wobei jenes Polynukleotid weiter eine Modifikation jener kodierenden Sequenz, die jene künstliche Meganuklease kodiert, umfasst, um den Thymidingehalt jener kodierenden Sequenz zu verringern, wobei jene Modifikation die Aminosäuresequenz jener künstlichen Meganuklease nicht verändert.

3. Das Polynukleotid aus Anspruch 1 oder Anspruch 2, wobei jener Promotor ein T7AG-Promotor ist.

4. Eine Messenger-RNA (mRNA), die von jenem Polynukleotid aus irgendeinem der Ansprüche 1-3 kodiert wird.

5. Die mRNA aus Anspruch 4, wobei jene mRNA eine 5'-Kappe umfasst.

6. Die mRNA aus Anspruch 5, wobei jene 5'-Kappe eine 5'-Methylguanosin-Kappe umfasst.

7. Die mRNA aus irgendeinem der Ansprüche 4-6, wobei ein Uridin, das in jener mRNA vorkommt, ein Pseudouridin oder 2-Thiouridin ist.

8. Die mRNA aus irgendeinem der Ansprüche 4-6, wobei ein Uridin, das in jener mRNA vorkommt, methyliert ist.

9. Die mRNA aus Anspruch 8, wobei ein Uridin, das in jener mRNA vorkommt, N1-Methylpseudouridin, 5-Methyluridin oder 2'-O-Methyluridin ist.

10. Ein rekombinantes DNA-Konstrukt umfassend jenes Polynukleotid aus irgendeinem der Ansprüche 1-3.

11. Ein rekombinantes Virus umfassend jenes Polynukleotid aus irgendeinem der Ansprüche 1-3.

12. Das rekombinante Virus aus Anspruch 11, wobei jenes rekombinante Virus ein rekombinantes Adeno-assoziiertes Virus (AAV) ist.

13. Eine Lipidnanopartikel-Zusammensetzung umfassend Lipidnanopartikel umfassend jene mRNA aus irgendeinem der Ansprüche 4-9.

14. Eine pharmazeutische Zusammensetzung umfassend einen pharmazeutisch annehmbaren Träger und jene Lipidnanopartikel-Zusammensetzung aus Anspruch 13.

## Revendications

1. Polynucléotide comprenant une séquence d'acides nucléiques codant une méganucléase modifiée, dans lequel ladite séquence d'acides nucléiques comprend :
(a) une région non traduite (UTR) 5' comprenant la séquence d'acide nucléique présentée dans la SEQ ID NO : 1 ;
(b) une séquence de codage codant ladite méganucléase modifiée, dans lequel ladite méganucléase modifiée comprend une première séquence de localisation nucléaire (NLS) positionnée au niveau de l'extrémité N-terminale et une deuxième NLS positionnée au niveau extrémité C-terminale, dans lequel ladite première NLS et ladite deuxième NLS comprennent chacune la séquence d'acides aminés énoncée dans la SEQ ID NO : 15 ;
(c) une UTR 3' comprenant la séquence d'acide nucléique présentée dans la SEQ ID NO : 10 ;
(d) une séquence poly A ; et
(e) un promoteur lié de manière opérationnelle à ladite séquence d'acide nucléique codant ladite méganucléase modifiée.

2. Polynucléotide selon la revendication 1, dans lequel ledit polynucléotide comprend en outre une modification de ladite séquence de codage codant ladite méganucléase modifiée pour réduire la teneur en thymidine de ladite séquence de codage, dans lequel ladite modification ne change pas la séquence d'acides aminés de ladite méganucléase modifiée.

3. Polynucléotide selon la revendication 1 ou la revendication 2, dans lequel ledit promoteur est un promoteur de T7AG.

4. ARN messager (ARNm) codé par ledit polynucléotide selon l'une quelconque des revendications 1 à 3.

5. ARNm selon la revendication 4, dans lequel ledit ARNm comprend une coiffe 5'.

6. ARNm selon la revendication 5, dans lequel ladite coiffe 5' comprend une coiffe 5' de méthylguanosine.

7. ARNm selon l'une quelconque des revendications 4 à 6, dans lequel une uridine présente dans ledit ARNm est la pseudouridine ou la 2-thiouridine.

8. ARNm selon l'une quelconque des revendications 4 à 6, dans lequel une uridine présente dans ledit ARNm est méthylée.

9. ARNm selon la revendication 8, dans lequel une uridine présente dans ledit ARNm est la N1-méthylpseudouridine, la 5-méthyluridine ou la 2'-O-méthyluridine.

10. Construction d'ADN recombinant comprenant ledit polynucléotide selon l'une quelconque des revendications 1 à 3.

11. Virus recombinant comprenant ledit polynucléotide selon l'une quelconque des revendications 1 à 3.

12. Virus recombinant selon la revendication 11, dans lequel ledit virus recombinant est un virus adéno-associé (AAV) recombinant.

13. Composition de nanoparticules lipidiques comprenant des nanoparticules lipidiques comprenant ledit ARNm selon l'une quelconque des revendications 4 à 9.

14. Composition pharmaceutique comprenant un support pharmaceutiquement acceptable et ladite composition de nanoparticules lipidiques selon la revendication 13.
